# EUROPEAN PATENT SPECIFICATION

(11) **EP 3 827 241 B1**
(45) Date of publication and mention of the grant of the patent: **13.03.2024**
(21) Application number: 19744682.6
(22) Date of filing: 23.07.2019
(51) Int. Cl.: G01N 1/28, B01L 3/00, C12M 3/02, C12M 1/06, C12M 1/33, C12M 1/00

(54) **DISSOCIATION OF BIOLOGICAL SAMPLES**
DISSOZIIERUNG VON BIOLOGISCHEN PROBEN
DISSOCIATION D'ECHANTILLON BIOLOGIQUE

(30) Priority: 24.07.2018 GB 201812073; 16.11.2018 GB 201818730; 06.03.2019 GB 201902984
(43) Date of publication of application: 02.06.2021
(73) Proprietor: Goldsborough, Andrew Simon, 33400 Talence (FR); Bates, Malcolm Robert, Cambridge CB1 1EE (GB)
(72) Inventor: Goldsborough, Andrew Simon, 33400 Talence (FR); Bates, Malcolm Robert, Cambridge CB1 1EE (GB)
(74) Representative: Page White Farrer
(86) International application number: PCT/EP2019/069846
(87) International publication number: WO 2020/020913

(56) References cited:
- EP-A1- 2 584 029
- WO-A1-2017/127921
- WO-A1-2018/091379
- TW-A- 201 715 100
- US-A1- 2009 221 075

## Description

### TECHNICAL FIELD

The present invention generally relates to methods of processing a biological sample to obtain single cells and/or groups of cells that may maintain some or all of their *in situ* morphology.

### BACKGROUND

The dissociation of biological samples into cells has been well known for decades and was the origin of many of the tissue culture cell lines that are used widely today. Such tissue culture cell lines were derived from diverse samples including cancers. Tissue culture cell lines have served as the foundation of biomedical research. In order to produce a cell line, the tissue to be dissociated must be metabolically active (Osborne N.N. Biochem. Soc. Trans. (1981) 9:583-585., Harrer D.S. et al., Nature (1964) 203:319-320., Scherer W.F. et al.; J. Exp. Med (1953) 97:695-710).

The classic method used for over a century to observe cells within a sample, such as a cancer biopsy, involves a complex procedure taking several days and requires tissue fixation with formaldehyde, dehydration, paraffin embedding, sectioning, mounting on a slide, staining and observation with a microscope (Forkner C.E. J. Exp. Med. (1930) 52:379-384). Because the paraffin embedded sample is cut into 4-8 µm sections, the majority of cells are cut open and therefore can only be observed in 2D so that the overall 3D shape and morphology of the cell is lost. Complicated methods have been developed to take consecutive parallel sections and using computer software, the partial 3D shape of individual cells can be partially ascertained, however this is not a simple or standardized method. As an alternative and as described here, one aim is to dissociate a tissue sample into groups of, or individual cells so that the 3D cell shape and morphology can be readily and rapidly determined by simple observation using a normal phase contrast microscope and a slide.

One aim is to reduce pre-analytical variables that occur as a result of a sample being removed from its natural environment, for example a cancer biopsy after surgical removal from the body. As has been described and set out in US patent no. 9,696,247 to Goldsborough and Bates metabolically active cells tend to respond with a stress response immediately when they are deprived of a blood supply or undergo a change in temperature, and one important response to such stress is an immediate change of transcription and degradation of the mRNA component (transcriptome). As a result of such changes, the abundance of specific mRNA molecules may radically change within minutes of surgical removal or blood draw (Malentacchi F. et al., PLoS One (2014) 9:11). As the population of mRNA in the cell is commonly used to ascertain the nature of the disease, for example the type or grade of cancer, it is imperative to arrest such changes as rapidly as possible. As way of example, biological samples are frequently plunged into liquid nitrogen to arrest such pre-analytical changes. It will be understood that profound alterations also occur to other types of biomolecules such as DNA, proteins, phosphoproteins, lipids and small molecules such as metabolites therefore the present disclosure relates to all types of biomolecules.

Whilst in the past it has been common to use RNA extracted from bulk samples, meaning many thousands or millions of cells weighing tens of milligrams or even grams, modern analytical techniques have made it possible to study biomolecules such as RNA in single cells or nuclei leading to fundamental discoveries about the diversity and heterogeneity of cells for example in a tissue or a cancer. However, despite the extraordinary advances in techniques for analyzing single-cells, methods for preserving the original representation of biomolecules in these cells has not advanced sufficiently, leading to sub-standard or an unfaithful representation of, for example, the transcriptome and proteome. The consequences of pre-analytical changes can be profound; the inaccurate measurement of biomarkers can lead to the incorrect therapeutic intervention of cancer for example.

The vast majority of techniques used for dissociating samples into single-cells rely either on enzymatic treatment such as with a protease, and/or a mechanical action to physically separate cells from one another. Whilst these methods may be appropriate for creating tissue culture cell lines, they are completely inappropriate for preserving either the cell morphology or the biomolecule composition of the single-cell.

Many tissue and cell fixatives are well known and some have been used for more than a century, most fall into either a cross-linking fixative such as formaldehyde or a protein precipitant or coagulant such as alcohols, in particular methanol. As described in US Patent 9,696,247, such fixatives are not appropriate for stabilizing biomolecules such as RNA. They are also very poorly suited to tissue dissociation into single-cells.

EP 2 584 029 A1 discloses a system for dissociating a sheet-shaped cell culture into individual cells.

WO 2017/127921 A1 discloses a method for dissociating cell aggregates in an agitated reactor.

US 2009/221075 A1 discloses a system to separate, enrich, and/or purify a cellular population from a biological tissue, such as a tissue sample.

TW 201 715 100 A discloses a method for processing fiber raw materials.

WO 2018/091379 A1 discloses a process for purifying a gaseous effluent containing at least one volatile organic compound, said process comprising bringing a defined deep eutectic solvent into contact with the gaseous effluent to be purified under conditions favourable to the absorption of the volatile organic compound by the deep eutectic solvent.

### SUMMARY

In one aspect, the present invention provides a method of processing a biological sample, according to claim 1. Certain more specific aspects of the invention are set out in the dependent claims.

It has been found that Deep Eutectic Solvents (DES) are not only excellent stabilizers of biomolecules as described in US Patent 9,696,247 but are also unusually well suited to fixing tissues that can subsequently be dissociated into single-cells for further detailed analytical procedures such as single-cell RNA sequencing (scRNAseq), single-cell genomic sequencing, single-cell proteome and/or phosphoproteome analysis, FACS and Flow Cytometry (Kortmann, H. et al., Adv. Biochem. Eng. Biotechnol. (2011) 124:99-122.; Proserpio V et al., Immunol Cell. Biol. (2016) 94:225-229.; Irish J.M. et al., Curr. Top. Microbiol. Immunol. (2014) 377:1-21.; Liang J. et al., J. Genet. Genomics. (2014) 41:513-528.; Dueck et al., Genome Biol. (2015) 16:122.; Grun, D. et al., Cell (2015) 163:799-810).

We describe here methods for the dissociation of DES fixed biological samples into single-cells using the surprisingly efficient mechanical method of sonication. Whilst sonication is a well-known and commonly used method in many types of laboratories from breaking down particles into their smallest possible components, including chemical, physical and engineering, it has not been successfully employed for producing morphologically distinct single-cells with an intact representative biomolecule repertoire. Importantly, in addition to stabilizing biomolecules, the cell morphology of the single-cells is retained providing an entirely new platform to analyse the structure and composition of the sample. It has therefore been discovered that not only are biomolecules faithfully preserved, but that the biomolecules are preserved in dissociated single cells that are morphologically comparable with living cells in the original sample. The application of sonication, also known as ultrasound energy, has been found to be particularly useful for producing very large numbers of such cells whilst preserving both extracellular and intracellular biomolecules and cell shape and morphology.

In a first aspect, the present invention provides a method of processing a biological sample to obtain single cells and/or groups of cells, as defined in claim 1. The method comprises: fixing cells of the biological sample using a fixative; contacting the biological sample with a dissociation medium; and dissociating the biological sample in the presence of the dissociation medium to obtain the single cells and/or groups of cells; wherein the fixative comprises a deep eutectic solvent. It has been found that deep eutectic solvents are not only excellent stabilizers of biomolecules as described in US Patent 9,696,247 but are also unusually well suited to fixing tissues that can subsequently be dissociated into single cells. Without wishing to be bound by theory, it is believed that in addition to fixing cells the deep eutectic solvent weakens interactions between adjacent cells. Fixation using a deep eutectic solvent is also effective for inhibiting degradation of biomolecules in the cells, allowing for improved analysis of the cells.

The fixing and the dissociating may be discrete steps or may be combined in a single step. The fixing and dissociating may partially or fully overlap in time. All or part of the biological sample may be fixed before the dissociating. Cells of the biological sample may be fixed during the dissociating.

A group of cells is a clump or cluster of cells which is smaller than the original biological sample. The size of a group of cells is not particularly limited, but typically comprises from 2 to 10,000 cells, for example 2 to 99 cells, 100 to 999 cells, or 1000 to 9,999 cells.

Various techniques can be used to perform the dissociating. Examples of such techniques include sonication, freeze-thawing of the sample, enzymatic digestion, and mechanical methods. Preferably, dissociating the biological sample comprises sonication of the biological sample using a sonicator. The application of sonication, also known as ultrasound energy, has been found to be particularly useful for producing very large numbers of such cells whilst preserving both extracellular and intracellular biomolecules and cell shape and morphology.

The sonication may be direct sonication, wherein the biological sample is in direct physical contact with a probe for applying ultrasound to the biological sample. Direct sonication may allow for particularly rapid dissociation of the sample.

Alternatively, the sonication may be indirect sonication using for example a block sonicator or bath sonicator. In indirect sonication, the biological sample is not in direct physical contact with the source of ultrasound. The ultrasound is instead transmitted through the dissociation medium. Indirect sonication may have a reduced risk of contamination of the sample compared to direct sonication. Indirect sonication may expose the sample to less heat compared to direct sonication. Excessive heat can accelerate degradation of cells or biomolecules.

In examples where indirect sonication is used, the method may further comprise constraining at least a part of the biological sample to a cavitation zone during the sonication. Constraining comprises controlling or restricting movement of the biological sample. A cavitation zone is an area where cavitation of the dissociation medium occurs. Some indirect sonicators, in particular bath sonicators, may produce an uneven distribution of ultrasound such that cavitation is focused in certain regions of space. These regions are referred to as cavitation zones. If an even distribution is achieved, the whole of the sonicated region can be regarded as a cavitation zone. Cavitation is useful for dissociating cells from one another and it may be advantageous to hold the biological sample in a region where cavitation occurs.

The constraining may comprise holding the biological sample using a biopsy cassette, cage, mesh, needle, pin, skewer or hook. Of these, the biopsy cassette or cage may be preferred. By using a biopsy cassette or cage having appropriately-sized apertures, separation of components of the biological sample may be achieved. For example, apertures having a size of about 50 µm may allow most mammalian cells to pass therethrough while retaining undissociated cells and fragments of extracellular matrix. In examples where it is desired to separate small cells from the sample, the apertures of the cage or cassette may be selected as appropriate. An 8 µm mesh for example has been found to be useful for erythrocytes. Illustrative mesh sizes are in the range 5 to 70 µm. The biopsy cassette, cage, mesh, needle, pin, skewer or hook may for example be arranged in a tube or other container.

The constraining may comprise feeding the biological sample into the cavitation zone as the biological sample is reduced in size. This may be useful if the biological sample is large. In an example, a biological sample is fed progressively into the dissociation medium during the dissociating. This may allow for a concentrated suspension of single cells to be collected in a small volume of dissociation medium.

The sonication may be pulse sonication. In other words, pulses of ultrasound may be applied with a rest period between each pulse. Pulse sonication may result in less heating of the biological sample and/or dissociation medium. The pulse sonication may have a pulse cycle wherein the sonicator is active for 50 to 95 %, 65 to 95 %, or 50 to 90 % of the pulse cycle and inactive for a remainder of the pulse cycle. For example, the sonicator may be active for 65 to 75 % of the pulse cycle. Each pulse cycle may have a duration of 0.5 to 5 seconds, or about 1 second. In examples where pulse sonication is used, the sonicator is typically a block sonicator.

The sonication may be at a frequency of 20 to 40 kHz, and in a more specific aspect, at a frequency of 20 to 30 kHz. Frequencies within these ranges have been found to be effective for dissociating biological samples, without generating excessive cell debris. Sonication frequency may be selected as appropriate depending on the nature of the biological sample.

The biological sample may be sonicated at a pressure in the range 81 kPa to 405 kPa (0.8 to 4 atm). Sonication in a reduced pressure environment increases the rate of dissociation. Sonication in an increased pressure environment decreases the rate of dissociation. Adjustment of the pressure is one useful technique for controlling the rate of dissociation.

The dissociation may be performed at a temperature in the range -20 to 37°C. For example, the sonication may be performed at a temperature of less than or equal to 24°C, or less than or equal to 12°C, or about 4°C. Controlling temperature, in particular avoiding excessive heating, may inhibit degradation of cells and/or biomolecules.

The method may include maintaining the temperature of the biological sample within the range 4°C to 24°C during at least the fixing, the contacting and the dissociating. Freezing of the biological sample between steps is contemplated.

The sonication may be applied for a time in the range 10 to 60 minutes, or for about 20 minutes. These are illustrative periods found to yield single cells for typical tissue samples. The sonication period may be adjusted as appropriate depending on the sample.

The method may further comprise adjusting power, frequency, amplitude and/or pulse cycle during the sonication. A biological sample may include two or more types of cells. Making adjustments during the dissociating may allow for efficient dissociating of the sample. In some examples, it may be desirable to reduce the intensity of sonication as the biological sample reduces in size during the dissociating.

The sonicator may be a block sonicator. Block sonicators may allow for greater control over certain parameters during the sonicating, including for example amplitude. Amplitude is the distance traveled in micrometres by the sonitrode of the sonicator, and therefore also the metal block or device holding the container with the sample. In examples where a block sonicator is used, the sonication may have an amplitude of less than or equal to 10 µm, and more particularly the sonication may have an amplitude in the range 6 to 8 µm, or of about 7 µm. Amplitudes in this range have been found to be useful for dissociating various tissues into single cells and/or groups of cells without generating excessive amounts of cell debris.

The sonicator may have a liquid bath. The liquid bath may comprise water. Preferably, the liquid bath contains an oil, a hydrocolloid, an echography gel, or a salt solution. The use of these liquids in the liquid bath has been found to modify transfer of energy to the biological sample, which may result in more efficient dissociation.

The oil is not particularly limited. For example, vegetable seed oils may be used. Echography gels are commercially available. The salt solution may be an NaCl solution, for example an aqueous solution comprising 10 % NaCl weight:volume.

Examples of useful hydrocolloids include those selected from: guar gum; xanthan gum; starch; locust bean gum; gum karaya; gum tragacanth; gum Arabic; aliginate; pectin; carrageenan; gelatin; gellan; agar; a cellulose derivative selected from methyl cellulose, ethyl cellulose, hydroxylpropylcellulose, hydroxyethylcellulose, and carboxymethylcellulose; and combinations thereof. The concentration of the hydrocolloid may be selected as appropriate. For example, the hydrocolloid may be dispersed in water in an amount of 0.1 to 10 % weight:volume.

The fixing may comprise sonicating the biological sample. Sonication during the fixing may improve the penetration of the fixative into the biological sample. This may allow for more rapid fixing, especially of samples of firm or robust tissues. Where sonication is applied during the fixing and the fixing is performed as a discrete step, milder sonication may be used during the fixing compared to during the dissociating.

In examples where the fixing comprises sonicating the biological sample, the sonication may be pulse sonication. The pulse sonication may have a pulse cycle wherein the sonicator is active for less than or equal to 20% of the pulse cycle. The amplitude of the sonication may be less than or equal to 2 µm. The pulse cycle may have a duration of 0.5 to 5 seconds, or about 1 second.

In an example where the fixing comprises sonicating the biological sample, the fixative may act as the dissociation medium, and the dissociating may comprise increasing the amount of ultrasound energy applied to the biological sample.

The deep eutectic solvent used in the fixative is not particularly limited. Any of the deep eutectic solvents identified in the detailed description or discussed in US Patent 9,696,247 may be used.

In an example, the deep eutectic solvent of the fixative comprises a first component and a second component, wherein first component is a compound of Formula I: 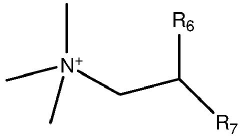 wherein:
R₆ is H or OH;
R₇ is selected from H, CH₃, Cl, Br, a carbonyl oxygen, and 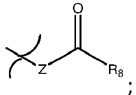
Z is selected from -CH₂-, O and S;
R₈ is R₁₁ or OH; and wherein the second component comprises a compound of Formula II or a salt thereof: 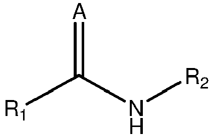 wherein:
   A is selected from O, S, and NH;
   R₁ is selected from H, an alkene group having 1 to 6 carbon atoms, R₉, -NH₂, -NH-(CH₂)ₙCH₃, and -C(R₃)(R₄)(R₅);
      wherein n is 0 or an integer from 1 to 5;
   R₂ is selected from H and linear alkyl group having 1 to 3 carbon atoms;
   R₃ is an optionally substituted 5- or 6- membered aliphatic or aromatic ring, wherein the substituent is R₁₀;
   R₄ and R₅ are each independently H or F; and
wherein R₉, R₁₀, and R₁₁ are each independently selected from alkyl groups having one to three carbon atoms, monochloroalkyl groups having one to three carbon atoms, and mono-, di- or tri-fluoroalkyl groups having one to three carbon atoms.

Most preferably, the first component is trimethylglycine and the second component is trifluoroacetamide. Trimethylglycine : trifluoroacetamide has been observed to be particularly effective for the fixation of cells and for inhibiting the degradation of biomolecules such as RNA, DNA and proteins such as phosphoproteins.

Another example of a DES falling within the above class is a DES wherein the first component is trimethylglycine and the second component is urea.

A still further example of a DES within the above class is a DES wherein the first component is choline chloride and the second component is urea.

The molar ratio of the first component to the second component may be in the range 1 : 1.5 to 1 : 2.5. In an example, the molar ratio of the first component to the second component is about 1: 2.

The most preferred deep eutectic solvent for the fixative is trimethylglycine : trifluoroacetamide at a molar ratio of about 1: 2.

An alternative is the class wherein the deep eutectic solvent of the fixative comprises a first component and a second component, wherein the first component comprises a compound of Formula III: 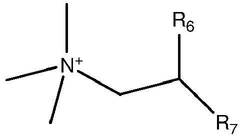 wherein:
R₆ is H or OH;
R₇ is selected from H, CH₃, Cl, Br, a carbonyl oxygen, and 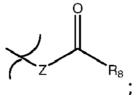
Z is selected from -CH₂-, O and S;
wherein R₈ is selected from OH, an alkyl group having one to three carbon atoms, a monochloroalkyl group having one to three carbon atoms, and a mono-, di- or tri-fluoroalkyl group having one to three carbon atoms; and
wherein the second component is a sugar or a sugar alcohol having at least 3 carbon atoms.

The molar ratio of the first component to the second component may be in the range 1:2 to 2:1.

One particular example of a DES within the above-mentioned class is a DES wherein the first component is trimethylglycine and the second component is glycerol. The molar ratio of trimethylglycine to glycerol may be about 1 : 2.

The fixative may further comprise, as an additive, an acid selected from: formic acid, acetic acid, citric acid, lactic acid, pyruvic acid, tartaric acid, propionic acid, oxalic acid, maleic acid, salicylic acid, ascorbic acid, benzoic acid, butanoic acid, hydrochloric acid, sulfuric acid, phosphoric acid, nitric acid and boric acid. The inclusion of an acid may reduce the viscosity of the DES in comparison with a DES not which does not include the acid additive. Reduced viscosity may make the deep eutectic solvent easier to handle. The inclusion of an acid as an additive in the fixative may also help to maintain cell and tissue volume during fixation. It has also surprisingly been found that, when a biological sample is transferred from such a DES to an aqueous medium, RNA integrity is maintained. Without wishing to be bound by theory, it is believed that acids may efficiently and permanently inactivate RNases, thereby protecting the analyte RNA from degradation by RNases, even when the fixed cell is subsequently transferred to an aqueous buffer as is common for many RNA analytical protocols.

In particular, the acid may be formic acid or acetic acid.

In examples wherein the acid is formic acid, the formic acid may be present in the fixative at a concentration in the range 4 to 9 M, 6 to 8 M, or 7 to 8 M; or of about 8 M. Formic acid concentrations of about 8 M have been found to be particularly effective for maintaining RNA stability. Where formic acid at concentrations in these ranges are used, the biological sample is preferably contacted with the fixative for no more than 10 minutes.

Alternatively, the formic acid may be present in the fixative at a concentration in the range 50 to 500 mM, 150 to 400 mM, or 250 to 350 mM. The use of these concentrations of Formic acid may be preferable when the biological sample is in contact with the fixative for more than 6 hours at room-temperature such as during transport and handling and/or when the biomolecule to be analysed is a protein or DNA molecule, both of which are generally more sensitive to acid hydrolysis than RNA. Additionally, these concentrations can lead to less sample hardening. When formic acid concentrations of less than or equal to 500 mM are used, the method may comprise after the fixing and before at least the dissociating, the biological sample is kept for at least 6 hours at a temperature of at least 18°C or about 24°C (i.e., about room temperature). Typically, the temperature does not exceed 37°C.

In examples where the acid is acetic acid, the acetic acid may be present in the dissociation medium at a concentration of at least 2 M, at least 8 M, at least 11 M, or at least 13 M. It is been found that acetic acid, particularly at concentration of at least 8 M produces, similar benefits to those of formic acid, as detailed above. Acetic acid is preferred for some applications, because it is safer to handle than formic acid. When the acetic acid concentration is at least 13 M, the biological sample is preferably contacted with the fixative for no more than 10 minutes.

Various compositions may be used as the dissociation medium. The dissociation medium may comprise a deep eutectic solvent, a glycol, water, or an ultrasound transmission gel.

In examples where the biological sample is fixed before the dissociating, the nature of the dissociation medium is not particularly limited provided that the fixed cells remain stable in the dissociation medium.

In examples where the biological sample is not fixed before the dissociating, the dissociation medium comprises a deep eutectic solvent. In this arrangement, the fixing and the dissociating may be performed in at the same time.

Preferably, the dissociation medium comprises a deep eutectic solvent.

Any of the deep eutectic solvents reported in US Patent 9,696,247 or identified in the detailed description below may be used as the dissociation medium.

For example, the deep eutectic solvent of the dissociation medium may comprise a first component and a second component, wherein first component is a compound of Formula I: 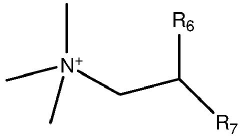 wherein:
R₆ is H or OH;
R₇ is selected from H, CH₃, Cl, Br, a carbonyl oxygen, and 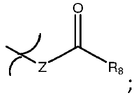
Z is selected from -CH₂-, O and S;
R₈ is R₁₁ or OH; and
   wherein the second component comprises a compound of Formula II or a salt thereof: 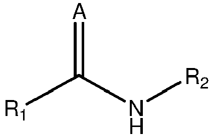 wherein:
   A is selected from O, S, and NH;
   R₁ is selected from H, an alkene group having 1 to 6 carbon atoms, R₉, -NH₂, -NH-(CH₂)ₙCH₃, and -C(R₃)(R₄)(R₅);
      wherein n is 0 or an integer from 1 to 5;
   R₂ is selected from H and linear alkyl group having 1 to 3 carbon atoms;
   R₃ is an optionally substituted 5- or 6- membered aliphatic or aromatic ring, wherein the substituent is R₁₀;
   R₄ and R₅ are each independently H or F; and
wherein R₉, R₁₀, and R₁₁ are each independently selected from alkyl groups having one to three carbon atoms, monochloroalkyl groups having one to three carbon atoms, and mono-, di- or tri-fluoroalkyl groups having one to three carbon atoms.

Preferred deep eutectic solvents within this class include: (i) trimethylglycine : trifluoroacetamide; (ii) trimethylglycine : urea; and (iii) choline chloride : urea.

The molar ratio of the first component to the second component may be in the range 1: 1.5 to 1 : 2.5. In an example, the molar ratio of the first component to the second component is about 1: 2.

Trimethylglycine : trifluoroacetamide at a molar ratio of about 1:2 is particularly preferred.

In another example, the deep eutectic solvent of the dissociation medium comprises a first component and a second component, wherein the first component comprises a compound of Formula III: 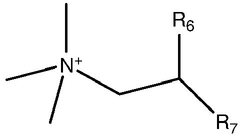 wherein:
R₆ is H or OH;
R₇ is selected from H, CH₃, Cl, Br, a carbonyl oxygen, and 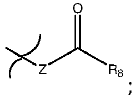
Z is selected from -CH₂-, O and S;
wherein R₈ is selected from OH, an alkyl group having one to three carbon atoms, a monochloroalkyl group having one to three carbon atoms, and a mono-, di- or tri-fluoroalkyl group having one to three carbon atoms; and
wherein the second component is a sugar or a sugar alcohol having at least 3 carbon atoms.

The molar ratio of the first component to the second component may be in the range 1:2 to 2:1.

One particular example of a DES within the above-mentioned class is a DES wherein the first component is trimethylglycine and the second component is glycerol. The molar ratio of trimethyl glycine to glycerol may be about 1 : 2.

The dissociation medium may further comprise water in an amount of up to 50 % by volume of the dissociation medium. For example, the dissociation medium may comprise water in an amount of up to 40 % by volume, or up to 30 % by volume, or up to 15 % by volume, or in the range 5 to 25 % by volume. The water may reduce the viscosity of the dissociation medium. This may be particularly relevant when the dissociating comprises sonication, since the amount of cavitation may be influenced by the viscosity of the medium.

In examples where the dissociation medium comprises a DES, the dissociation medium may further comprises an acid selected from: formic acid, acetic acid, citric acid, lactic acid, pyruvic acid, tartaric acid, propionic acid, oxalic acid, maleic acid, salicylic acid, ascorbic acid, benzoic acid, butanoic acid, hydrochloric acid, sulfuric acid, phosphoric acid, nitric acid and boric acid. As discussed above with reference to the fixative, the acid may be useful for deactivating RNases in the sample, which is useful if the sample is to be exposed to an aqueous medium.

Preferably, the acid is formic acid or acetic acid.

In examples where the acid is acetic acid, the acetic acid may be present in the dissociation medium at a concentration of at least 2 M, at least 8 M, or at least 11 M.

In examples where the acid is formic acid, the formic acid may be present in the dissociation medium at a concentration in the range 4 to 9 M, 6 to 8 M, or 7 to 8 M; or of about 8 M. These concentrations are particularly effective for maintaining RNA stability. When formic acid concentrations in these ranges are used, the biological sample is preferably in contact with the dissociation medium for no longer than 10 minutes.

Alternatively, the formic acid may be present in the dissociation medium at a concentration in the range 50 to 500 mM, 150 to 400 mM, or 250 to 350 mM. These concentrations may be preferred for maintaining DNA or protein stability, or if the biological sample is to be contacted with the dissociation medium for an extended period.

When the dissociation medium comprises a deep eutectic solvent, the fixative and the dissociation medium may each comprise the same deep eutectic solvent. For example, the method may include forming the dissociation medium *in situ* during or after the fixing, by adding one or more components to the fixative. Alternatively, the fixative and the dissociation medium may be identical.

The biological sample may be contacted with the fixative or dissociation medium for a time in the range 3 to 20 minutes or 3 to 10 minutes before starting the dissociating. Contact times within these ranges generally allow for fixing of at least some cells of the biological sample.

Fixing of at least part of the biological sample may occur during the dissociating. In some examples, a portion of the biological sample is fixed before starting the dissociating. In other examples, fixing and dissociating are performed simultaneously. This may be useful for large biological samples, or samples which are slowly penetrated by the fixative.

The dissociation medium may comprise a glycol selected from ethylene glycol, glycerol, diethylene glycol, triethylene glycol, and a polyethylene glycol, PEG, having a molecular weight in the range 200 to 800. For example, the dissociation medium may comprise PEG 200, PEG 300, PEG 400 or PEG 600. When the dissociation medium comprises a glycol, the dissociation medium may further comprises up to 60 % water by weight of the dissociation medium.

The glycol or a mixture of the glycol with water may be used as a dissociation medium. In other words, when the dissociation medium comprises a glycol the dissociation medium does not necessarily comprise a DES.

The use of a mixture of a deep eutectic solvent and a glycol as a dissociation medium is also contemplated. In such mixtures, the glycol may improve dissociation of the sample by softening the sample.

Beads or particles for physically abrading the biological sample may be dispersed in the dissociation medium. Such beads or particles may be useful for dissociating robust samples by allowing additional physical disruption of the sample.

Sonication during the dissociating is not essential. Any appropriate technique may be used to perform the dissociating. Mechanical methods, such as those described in the detailed description below, may be used. Freezing and thawing is one method for dissociating the biological sample. In an example, the fixing is performed before the contacting; the dissociation medium is water; and the dissociating comprises freezing and thawing the biological sample to obtain the single cells and/or groups of cells. The freezing and thawing may be repeated at least 3 times. Freezing and thawing is useful for dissociating robust samples. When freezing and thawing is used the biological sample is preferably a biopsy comprising a tissue selected from: liver, lung, muscle, cornea, tongue, heart, oesophagus, ovaries, skin stomach, a tumour, kidney, testis, brain, thymus, spleen, intestine, pancreas, blood vessel, teeth, cartilage, and bone.

The fixative used with freezing and thawing comprises a DES, and may be any of the fixatives discussed above.

Any of the above methods may further comprise, after the fixing, contacting the biological sample with an acid selected from formic acid, acetic acid, citric acid, lactic acid, pyruvic acid, tartaric acid, propionic acid, oxalic acid, maleic acid, salicylic acid, ascorbic acid, benzoic acid, butanoic acid, hydrochloric acid, sulfuric acid, phosphoric acid, nitric acid and boric acid. In other words, the biological sample may be contacted with an acid separately from the fixative and dissociation medium. It is believed that the acid may have the effects described above in the context of the discussion of the fixative when the acid is introduced at any stage of the method, e.g. in the fixative, in the dissociation medium, and/or as a discrete treatment step.

In an example where the biological sample is contacted with an acid, the acid may be acetic acid and the method may include contacting the biological sample with: (i) glacial acetic acid; or (ii) a solution comprising acetic acid and a solvent selected from a deep eutectic solvent, water, an alcohol, and mixtures thereof, wherein the concentration of acetic acid in the solution is at least 8 M, at least 9 M, at least 11 M, or at least 13 M. Without wishing to be bound by theory, it is believed that acetic acid efficiently and permanently inactivates RNases, thereby protecting the analyte RNA from degradation by RNases, even when the fixed cell is subsequently transferred to an aqueous buffer as is common for many RNA analytical protocols.

The methods described herein may further comprise freezing the biological sample before the fixing; and thawing the biological sample during the fixing. Freezing is one technique for preserving biological samples. If the fixative is present while the sample is being defrosted, damage to cells in the biological sample may be prevented and/or degradation of biomolecules may be inhibited.

The methods described herein may further comprise staining. The staining may be performed at any stage. For example, the biological sample may be stained before, during or after the fixing. Staining of the single cells and/or groups of cells after the dissociating is also contemplated. Staining is useful for various types of analysis, such as histology.

The methods described herein may further comprise, after the fixing and before the dissociating, softening the biological sample using a glycol. Examples of suitable glycols include: ethylene glycol, glycerol, diethylene glycol, triethylene glycol, PEG 200, PEG 300, PEG 400, PEG 600 and mixtures thereof. Softening the biological sample may allow for easier dissociation of the biological sample into single cells and/or groups of cells. This may help to reduce heating of the sample, thereby improving the stability of biomolecules in the sample. The glycol may be included in the dissociation medium, or may be applied separately from the dissociation medium.

In the method of the first aspect, the dissociating may comprise, after the fixing, treating the biological sample with at least one enzyme. It has surprisingly been found that biological samples which have been fixed using a DES may be dissociated by enzymatic digestion while maintaining RNA integrity and/or *in vivo* shape of the cells. Contrastingly, dissociation of fresh, unfixed tissues using enzymes generally results in changes in cell morphology and/or extensive degradation of RNA.

In some examples, treating the biological sample with the at least one enzyme is performed before dissociating the sample using a different technique such as sonication.

Examples of useful enzymes include: collagenase type I, II, III, and IV; trypsin; papain; proteinase K; chymotrypsin; elastase; dispase; a mixture of a collagenase and a neutral protease; pronase; hyaluronidase; DNase, and a lipase. "Pronase" refers to a protease obtainable from *Streptomyces griseus* having CAS number 9036-06-0.

The methods described herein may further comprise, after the dissociating, lysing the single cells and/or groups of cells. Lysing the cells may be useful for various types of analysis. For example, the method may further comprise isolating cell nuclei after the lysing.

Lysing the single cells and/or groups of cells may comprise contacting the single cells and/or groups of cells with a chaotrope selected from sodium perchlorate, potassium iodide, and thiourea; or an organic solvent selected from beta-mercaptoethanol, 1,1,1,3,3,3-hexafluoro-2-propanol, 1,1,1,3,3,3-hexafluoro-2-phenyl-2-propanol, 1,1,1,3,3,3-hexafluoro-2-methyl-2-propanol, dimethyl sulfoxide, and phenol. In one example, the organic solvent is dimethyl sulfoxide. Dimethyl sulfoxide has been found to solubilize selectively the cytoplasm of cells, leaving cell nuclei intact. Isolated nuclei are of particular interest for certain types of analysis. Diethyl sulfoxide and ethyl methyl sulfoxide may show similar behaviour to dimethyl sulfoxide. Dimethyl sulfoxide is preferred.

Lysing the single cells and/or groups of cells may comprises physically disrupting the cells in the presence of a chaotrope selected from potassium iodide, urea, guanidine thiocyanate, and guanidine hydrochloride. The use of these chaotropes in combination with physical disruption has been found to be effective for lysing cells which have been fixed using a deep eutectic solvent.

The methods described herein may further comprise, at one or more time points during the dissociating, isolating groups of cells from the dissociation medium. Different parts of the biological sample may dissociate at different time points during the dissociating. Dissociating may occur at an outer surface of the biological sample, and consequently different groups of cells may be released into the dissociation medium at different times. Sizes of groups of cells may vary during the dissociating, for example a sample may first dissociate into relatively large groups, which then dissociate into progressively smaller groups. Sampling or monitoring the cells which are obtained during the dissociating may provide useful insights.

The biological sample may be a tissue sample. The type of tissue is not particularly limited. The tissue is generally a solid tissue. The biological sample may comprise any of the various tissues identified in the detailed description hereinbelow. Biopsies may be of particular interest for some applications.

The biological sample may include extracellular matrix, and the method further comprises, after the dissociating, isolating at least a component of the extracellular matrix. Extracellular matrix may be separated from the cells and/or groups of cells by filtration, for example. Pieces of extracellular matrix may be larger than individual cells. For example a 40 to 70 µm filter may be used to isolate cellular matrix.

The method described herein may further comprise adding dimethyl sulfoxide to the dissociation medium before, during or after the dissociating. Dimethyl sulfoxide is useful for allowing the separation of nuclei from cell cytoplasm. Dimethyl sulfoxide solubilizes cytoplasm but not nuclei of DES-fixed cells.

The method described herein may further comprise, after the dissociating, sorting the single cells and/or groups of cells. In an example, the sorting comprises staining and flow cytometry. Sorting of cells is useful for various types of analysis. Filtration is another example of a technique for sorting cells.

The method described herein may further comprise, after the dissociating one or more analyses selected from: counting cells; measuring at least one property of cells selected from dimensions, shape, morphology, phenotype; determining the amount and/or identity of one or more RNA, DNA or protein; determining a cell staining pattern; and imaging cells.

The method described herein may further comprise, after the dissociating, analysing or isolating one or more biomolecules of the biological sample, the one or more biomolecules being selected from: a DNA, an RNA, a protein, a phosphoprotein, and combinations thereof. The analysing may comprise single-cell RNA sequencing and/or single cell DNA sequencing. Since deep eutectic solvents are effective for inhibiting the degradation of biomolecules such as RNA and DNA, the methods provided herein are particularly well suited for use in such analysis. For similar reasons, the analysing may comprise localising biomolecules, i.e. analysis of where in a cell particular biomolecules may be found.

The method described herein may further comprise storing the single cells and/or groups of cells for at least 1 day at a temperature in the range 4 to 24 °C. For example, the single cells and/or groups of cells may be stored for up to one month, or longer. Fixation using a deep eutectic solvent may allow for storage of the cells and/or groups of cells for later analysis.

A second aspect of the present disclosure (not in accordance with the present claims) provides an apparatus for dissociating a biological sample into single cells and/or groups of cells, which apparatus comprises: a container for containing a biological sample and a liquid dissociation medium and for transmitting sonication energy to the biological sample and liquid dissociation medium; a conduit having an inlet for removing liquid dissociation medium from the container and an outlet downstream of the inlet; first and second filters arranged in the conduit, the second filter being downstream of the first filter; a pump for pumping liquid dissociation medium through the conduit from the inlet to the outlet; wherein the first filter is a 40 to 70 µm filter, and the second filter is a 1 to 12 µm filter. The apparatus is useful in the practice of the methods provided herein. The first filter may allow for removal of extracellular matrix. The second filter may allow for isolation of single cells.

The outlet may be for returning liquid dissociation medium to the container. This allows recycling of the dissociation medium.

The apparatus may further comprise a plunger arranged in the container. A plunger may movement, gas exchange and evaporation of dissociation medium.

The plunger may be configured for adjusting pressure in the container. For example, there may be an air-tight seal between the plunger and walls of the container. The plunger may be for generating pressures in the range 81 kPa to 405 kPa (0.8 to 4 atm) in the container. It may be useful to adjust pressure in the container because pressure may influence the rate at which a biological sample dissociates into single cells. Typically, the higher the pressure, the slower dissociation occurs.

The plunger may further comprise a heat transfer element, the heat transfer element being operably linked to a temperature adjustment device for controlling temperature in the container. Adjusting the temperature may change the effective viscosity of the dissociation medium. Temperature control is also useful for preventing unnecessary heating of the biological sample, as excessive heat may lead to degradation of the sample or biomolecules present in the sample. Various temperature adjustment devices may be used, such as Peltier controllers. In an example, the heat transfer element and temperature adjustment device are capable of maintaining a temperature within the range 4°C to 24°C.

The plunger may have an abrasive surface for abrading the biological sample. Physical abrasion may be useful for aiding in dissociation of particularly robust samples such as teeth or bone, for example.

The apparatus may further comprise a biopsy cassette in the container. A biopsy cassette is a cage structure having at least one wall which includes a mesh. A biopsy cassette is useful for holding a biological sample in position, for example, holding the biological sample within a cavitation zone or focal region of a sonicator. In examples, the biopsy cassette has a mesh size in the range 5 to 200 µm, for example a mesh size selected from about 200, about 100, about 70, about 50, about 35, about 25, about 20, about 15, about 10, about 8, and about 5 µm (in this context, "about" means to within ±10 %). The mesh of the biopsy cassette may serve as a filter, allowing for the selection of cells of particular sizes.

The apparatus may further comprise a pin, needle or hook for holding a biological sample arranged in the container. Holding the biological sample is useful for keeping all or part of the biological sample within a cavitation zone or focal region of a sonicator. The pin, needle or hook may allow for manipulation of the biological sample during the dissociating. This is useful in examples where it is desired to feed the biological sample progressively into the dissociation medium during the dissociating.

The apparatus may further comprise sensors for monitoring pressure and/or temperature in the container. In particular, a temperature sensor may be present. It may be desirable to measure temperature and/or pressure since these parameters may influence the rate of dissociation. Temperature monitoring also be desirable for avoiding overheating of the biological sample. A thermocouple is one example of a temperature sensor.

The container may have walls of polycarbonate. The material from which the container is constructed is not particularly limited. Polycarbonate is a preferred material because it has been found to be more resistant to shattering by sonication than glass and to allow for efficient transmission of ultrasound. Polycarbonate is also transparent enough to allow visual observation of the sample, which may be useful for monitoring the progress of the dissociation.

The container may be in communication with a source of sonication energy. The container may be at least partially submerged in a sonication bath. Alternatively, the container may be in contact with a block, the block being operably linked to a sonitrode. In another alternative, a sonication probe may extend into the container. Sonication is one technique useful for dissociation of biological samples.

The source of sonication energy may be configured to provide pulse sonication. Pulse sonication may result in reduced heating of the biological sample compared to continuous sonication.

The apparatus may further comprise an analytical device. The nature of the analytical device is not particularly limited, and may be selected as desired. For example, the analytical device may be configured to perform one or more of: counting cells; measuring at least one property of cells selected from dimensions, shape, morphology, phenotype; determining the amount and/or identity of one or more RNA, DNA or protein; determining a cell staining pattern; and imaging cells.

The conduit may further comprise a port, valve or junction for transferring dissociation medium to a further conduit, a further container, or an analytical device. Transfer of dissociation medium, single cells, and/or groups of cells to another device for further processing may be desirable for some applications.

In a third aspect of the present disclosure (not in accordance with the present claims), there is provided a composition comprising a deep eutectic solvent and an additive, wherein the additive is an acid selected from formic acid, acetic acid, citric acid, lactic acid, pyruvic acid, tartaric acid, propionic acid, oxalic acid, maleic acid, salicylic acid, ascorbic acid, benzoic acid, butanoic acid, hydrochloric acid, sulfuric acid, phosphoric acid, nitric acid, and boric acid. It has surprisingly been found that such compositions are particularly well-suited for use as fixatives or dissociation mediums in the method described above. The inclusion of an acid as an additive in the DES may help to maintain cell and tissue volume during cell or tissue fixation. It has also surprisingly been found that, when a biological sample is transferred from such a DES to an aqueous medium, RNA integrity is maintained. Without wishing to be bound by theory, it is believed that acids may efficiently and permanently inactivate RNases, thereby protecting the RNA from degradation, even when the fixed cell is subsequently transferred to an aqueous buffer.

The deep eutectic solvent present in the composition may be any of the deep eutectic solvents described in US Patent 9,696,247, or in the detailed description below.

In preferred examples, the deep eutectic solvent comprises a first component and a second component, wherein first component is a compound of Formula I: 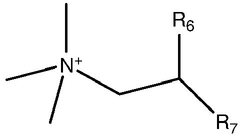 wherein:
R₆ is H or OH;
R₇ is selected from H, CH₃, Cl, Br, a carbonyl oxygen, and 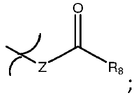
Z is selected from -CH₂-, O and S;
R₈ is R₁₁ or OH; and
   wherein the second component comprises a compound of Formula II or a salt thereof: 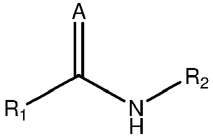 wherein:
   A is selected from O, S, and NH;
   R₁ is selected from H, an alkene group having 1 to 6 carbon atoms, R₉, -NH₂, -NH-(CH₂)ₙCH₃, and -C(R₃)(R₄)(R₅);
      wherein n is 0 or an integer from 1 to 5;
   R₂ is selected from H and linear alkyl group having 1 to 3 carbon atoms;
   R₃ is an optionally substituted 5- or 6- membered aliphatic or aromatic ring, wherein the substituent is R₁₀;
   R₄ and R₅ are each independently H or F; and
wherein R₉, R₁₀, and R₁₁ are each independently selected from alkyl groups having one to three carbon atoms, monochloroalkyl groups having one to three carbon atoms, and mono-, di- or tri-fluoroalkyl groups having one to three carbon atoms.

In more specific examples, (i) the first component is trimethylglycine and the second component is trifluoroacetamide; or (ii) the first component is trimethylglycine and the second component is urea; or (iii) the first component is choline chloride and the second component is urea; or (iv) the first component is choline chloride and the second component is trifluoroacetamide.

Most preferably, the first component is trimethylglycine and the second component is trifluoroacetamide. This DES is particularly effective as a fixative and for stabilising biomolecules.

The molar ratio of the first component to the second component may be in the range 1 : 1.5 to 1 : 2.5. In an example, the molar ratio of the first component to the second component is about 1: 2.

The most preferred deep eutectic solvent for the fixative is Trimethylglycine : trifluoroacetamide at a molar ratio of about 1: 2.

In further examples, the deep eutectic solvent may comprise a first component and a second component, wherein the first component comprises a compound of Formula III: 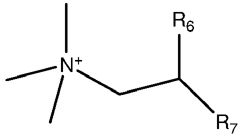 wherein:
R₆ is H or OH;
R₇ is selected from H, CH₃, Cl, Br, a carbonyl oxygen, and 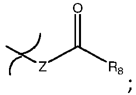
Z is selected from -CH₂-, O and S;
wherein R₈ is selected from OH, an alkyl group having one to three carbon atoms, a monochloroalkyl group having one to three carbon atoms, and a mono-, di- or tri-fluoroalkyl group having one to three carbon atoms; and
wherein the second component is a sugar or a sugar alcohol having at least 3 carbon atoms.

One particular example of a DES within the above class is a DES wherein the first component is trimethylglycine and the second component is glycerol.

The molar ratio of the first component to the second component may be in the range 1 : 1.5 to 1 : 2.5. In an example, the molar ratio of the first component to the second component is about 1: 2.

The acid may be formic acid. The formic acid may be present in the composition at a concentration in the range 50 mM to 12 M. For example, the formic acid may be present in the composition at a concentration in the range 4 to 9 M, 6 to 8 M, or 7 to 8 M; or of about 8 M. Formic acid concentrations of about 8 M are particularly effective for maintaining RNA stability.

For other applications, formic acid concentrations in the range 50 to 500 mM, optionally 150 to 400 mM, and further optionally 250 to 350 mM may be preferred. The use of these concentrations of formic acid may be preferable when the biological sample is in contact for more than 6 hours at room-temperature such as during transport and handling and/or when the biomolecule to be analysed is a protein or DNA molecule, both of which are generally more sensitive to acid hydrolysis than RNA. Additionally, these lower formic acid concentrations can lead to less sample hardening and therefore improve cell lysis and biomolecule purification yield.

The acid may be acetic acid. The acetic acid may be present in the composition at a concentration of at least 8 M, at least 8 M, at least 11 M, or at least 13 M. It is been found that an acetic acid concentration of at least 8 M produces similar benefits to those of formic acid, detailed above. Acetic acid is preferred for some applications, because it may be safer to handle than formic acid.

The composition is useful in a method of fixing a biological sample, which method comprises contacting the biological sample with a composition as described above to form a fixed biological sample. Also provided is the use of the composition to inhibit degradation of a biomolecule (e.g. a method of inhibiting degradation of a biomolecule, comprising contacting the biomolecule with the composition. Inhibiting degradation may include preventing changes in molecular weight of the biomolecule, for example).

A fourth aspect of the present disclosure (not in accordance with the present claims) provides a deep eutectic solvent for use in surgery or in *in vivo* diagnosis. It has been found that deep eutectic solvents may be useful in methods comprising applying the deep eutectic solvent to a region of skin of a patient; applying sonication to the region; and removing the deep eutectic solvent. Cells (e.g. cells from the patient or bacterial cells), viruses, and/or biomolecules may then be recovered from the biological sample for subsequent analysis. The region of skin may, for example, be a region where skin cancer is present or suspected to be present. Recovered cells may be useful for diagnosis of disorders. Analysis of the sample may include inspection of cell morphology and/or analysis of biomolecules such as nucleic acids. The method may include analysis of the sample, and assignment of a diagnosis to the patient.

The deep eutectic solvent may provide fixing of cells obtained. Since deep eutectic solvents allow cells to be separated from one another more easily, effective collection of a sample may be achieved. Injury to the patient may be significantly less than when using techniques such as removal of a biopsy with a scalpel. Stabilisation of biomolecules in the collected sample by the deep eutectic solvent may enable improvements in the analysis of the sample, and corresponding improvements in the accuracy or reliability of a diagnosis based on the analysis.

The deep eutectic solvent for use as provided herein may be applied to a patient to collect a biological sample from the patient. Collecting the biological sample may include applying sonication to the patient.

The most preferred deep eutectic solvent for use in surgery or in *in vivo* diagnosis comprises trimethylglycine and glycerol. An *in vivo* experiment in which this deep eutectic solvent was applied to skin has been performed, and no adverse effects were observed.

The molar ratio of the trimethylglycine to the glycerol may be in the range 1:2 to 2:1, and is preferably about 1 : 2.

The deep eutectic solvent for use in accordance with this aspect may be used to fix the biological sample as it is collected. This may be useful where storage or shipping is to take place before analysis of the sample.

A fifth aspect of the present disclosure (not in accordance with the present claims) provides method of extracting cell nuclei from a biological sample, the method comprising: contacting the biological sample with a deep eutectic solvent to obtain a fixed biological sample comprising fixed cells; and adding a sulfoxide selected from dimethylsulfoxide, diethylsulfoxide, and ethyl methyl sulfoxide to the fixed biological sample to solubilise cytoplasm of the fixed cells thereby extracting the cell nuclei. It has surprisingly been found that nuclei can be efficiently extracted from cells fixed using a DES by treating the fixed cells with a sulfoxide selected from dimethylsulfoxide, diethylsulfoxide and ethyl methyl sulfoxide. Of these, dimethysulfoxide is particularly effective and is most preferred.

The sulfoxide may be added in an amount of at least 25 % based on the volume of the fixed biological sample.

The deep eutectic solvent may be any of the deep eutectic solvents discussed in US Patent 9,696,247 or set out in the detailed description provided herein.

For example, the deep eutectic solvent may comprise a first component and a second component, wherein the first component is a compound of Formula I: 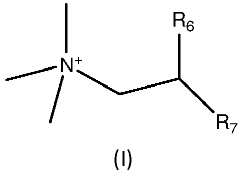 wherein:
R₆ is H or OH;
R₇ is selected from H, CH₃, Cl, Br, a carbonyl oxygen, and 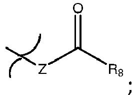
Z is selected from -CH₂-, O and S;
R₈ is R₁₁ or OH; and
   wherein the second component comprises a compound of Formula II or a salt thereof: 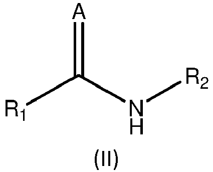 wherein:
   A is selected from O, S, and NH;
   R₁ is selected from H, an alkene group having 1 to 6 carbon atoms, R₉, -NH₂, -NH-(CH₂)ₙCH₃, and -C(R₃)(R₄)(R₅);
      wherein n is 0 or an integer from 1 to 5;
   R₂ is selected from H and linear alkyl group having 1 to 3 carbon atoms;
   R₃ is an optionally substituted 5- or 6- membered aliphatic or aromatic ring, wherein the substituent is R₁₀;
   R₄ and R₅ are each independently H or F; and
wherein R₉, R₁₀, and R₁₁ are each independently selected from alkyl groups having one to three carbon atoms, monochloroalkyl groups having one to three carbon atoms, and mono-, di- or tri-fluoroalkyl groups having one to three carbon atoms.

Examples of preferred deep eutectic solvents within this class include DESs wherein: (i) the first component is trimethylglycine and the second component is trifluoroacetamide; or (ii) the first component is trimethylglycine and the second component is urea; or (iii) the first component is choline chloride and the second component is urea.

The molar ratio of the first component to the second component may be in the range 1 : 1.5 to 1 : 2.5. In an example, the molar ratio of the first component to the second component is about 1: 2.

The most preferred deep eutectic solvent for inclusion in the composition is trimethylglycine : trifluoroacetamide at a molar ratio of about 1: 2.

A sixth aspect of the disclosure provides the use of a deep eutectic solvent as a dissociation medium in a method of dissociating a biological sample into single cells and/or groups of cells. (A method of dissociating a biological sample into single cells and/or groups of cells, comprising contacting the biological sample with a dissociation medium and dissociating the biological sample, wherein the dissociation medium comprises a deep eutectic solvent). In certain more specific aspects, the biological sample may have been fixed using a deep eutectic solvent. The deep eutectic solvent may stabilise a biomolecule selected from an RNA, a DNA, a protein, and a phosphoprotein. Features described with reference to the first aspect are equally applicable to this aspect, provided that the dissociation medium comprises a DES.

A seventh aspect provides the use of single cells obtainable by the method of the first aspect in single-cell RNA sequencing and/or single cell DNA sequencing. In other words, there is provided a method of single-cell nucleic acid sequencing, comprising obtaining a single cell by the method of the first aspect, and sequencing a nucleic acid of the single cell, wherein the nucleic acid is at least one member selected from RNA and DNA. Features described with reference to the first aspect are equally applicable to this aspect. Single-cell RNA and DNA sequencing is an area of particular research interest. Since single cells obtainable by the method of the first aspect are fixed using a deep eutectic solvent, degradation of nucleic acids in the single cells is inhibited. This may allow for more reliable single-cell nucleic acid sequencing.

An eighth aspect provides a method of processing a fixed biological sample to obtain single cells and/or groups of cells as defined in claim 15. The method comprises: contacting the fixed biological sample with composition comprising a deep eutectic solvent; subsequently contacting the biological sample with a dissociation medium; and dissociating the biological sample in the presence of the dissociation medium to obtain the single cells and/or groups of cells. This method is a variant of the method of the first aspect, and the various features discussed with reference to the first aspect apply equally to this aspect; however in accordance with the eighth aspect the fixative does not necessarily comprise a DES.

In one example the method further comprises, before contacting the fixed biological sample with the composition, fixing a biological sample with a fixative to obtain the fixed biological sample, wherein the fixative is not a deep eutectic solvent. It has been found that treating with a DES a biological sample which has already been fixed using an alternative fixative may be useful for preventing further degradation of biomolecules or cells. This can be achieved even if a DES is applied in an intermediate step without being present in the dissociation medium.

Examples of fixatives which are not deep eutectic solvents include: Methanol, Ethanol, Acetic acid, Acetone, PAXgene Tissue, Clarke's, Ridley's, Zamboni's, Davidson's, HOPE, RCL2, UMFIX, BF, Z7, Polyethylenglycol ethyl Alcohol Glycerol Acetic acid, Albumin-Glycerol, Fine-Fixx, Glyo-Fixx, Zenker's, Helly's, B-5, Bouin's, Hollande's, Gendre's, Clarke's, Carnoy's, Methacarn, Michel's, Wright's, Mirsky's, Hartman's, Formaldehyde, Glutaraldehyde, Paraformaldehyde, 4% Neutral buffered formalin, 10% Neutral buffered formalin, Phosphate buffered formalin, Formal calcium, Formal saline, Zinc formalin, Alcoholic formalin and Formol acetic alcohol.

### BRIEF DESCRIPTION OF THE DRAWINGS

The present invention will become more fully understood from the detailed description and the accompanying drawings, in which:

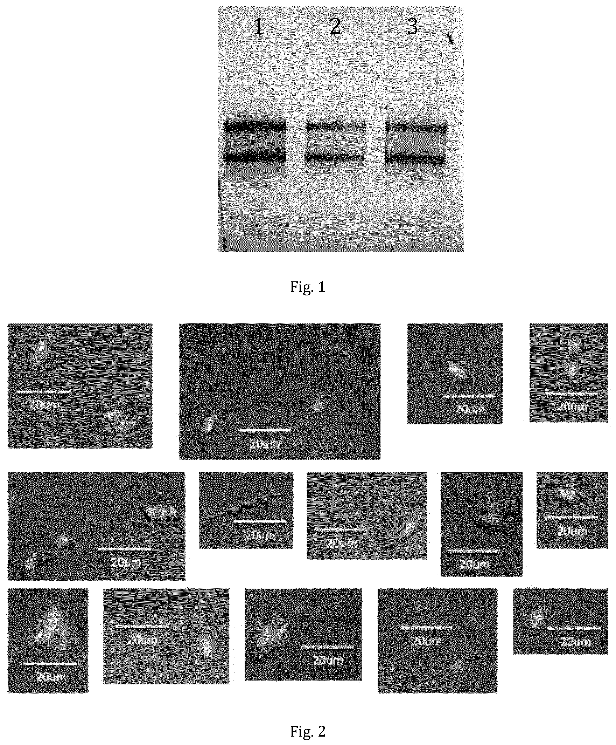

**Figure 1** shows RNA quality from single-cells obtained from mouse liver treated with Trimethylglycine:Trifluoroacetamide (1:2 mol:mol) containing 300mM Formic acid for 24 hours, then dissociated according to Example 15 (35 kHz), for 15 minutes at room temperature. Lane 1, 85% Trimethylglycine: Trifluoroacetamide (1:2 mol:mol) Dissociation buffer, Lane 2. 85% Trimethylglycine:Glycerol (1:2 mol:mol), Lane 3. Postive control, sample not subjected to ultrasound. It can be seen that the ultrasound treatment of the liver sample in either Dissociation buffer had no effect on RNA quality.
**Figure 2** shows Frozen-Thawed Mouse Intestine Thawed in RNAssist and Dissociated Mechanically (DAPI Stain).

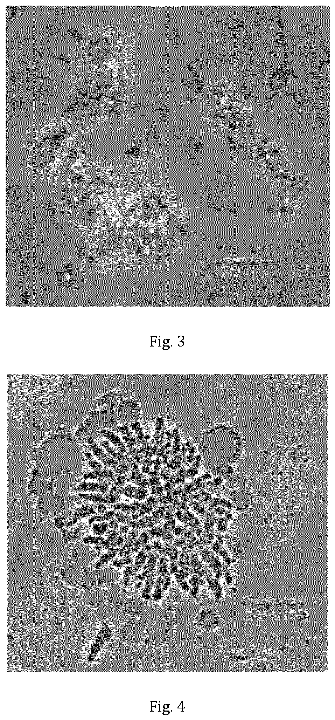

**Figure 3** shows typical cellular debris obtained when dissociating fixed mouse liver in water.
**Figure 4** shows loss of cellular integrity and membrane loss when dissociating fixed mouse intestine in PBS.

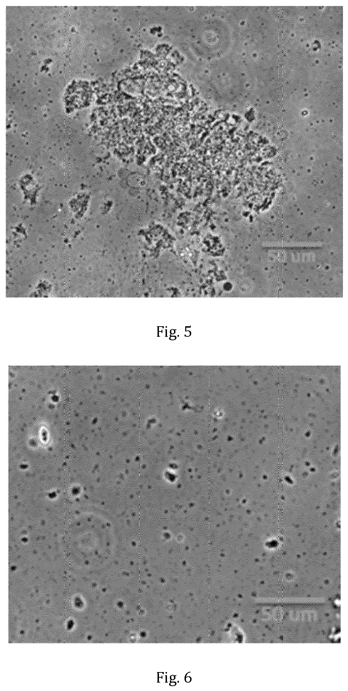

**Figure 5** shows loss of cellular integrity and membrane loss when dissociating RNAlater fixed mouse intestine. RNAlater is a commercially-available composition for inhibiting the degradation of RNA, which does not include a DES.
**Figure 6** shows loss of cellular integrity and membrane loss when dissociating NBF fixed mouse intestine;

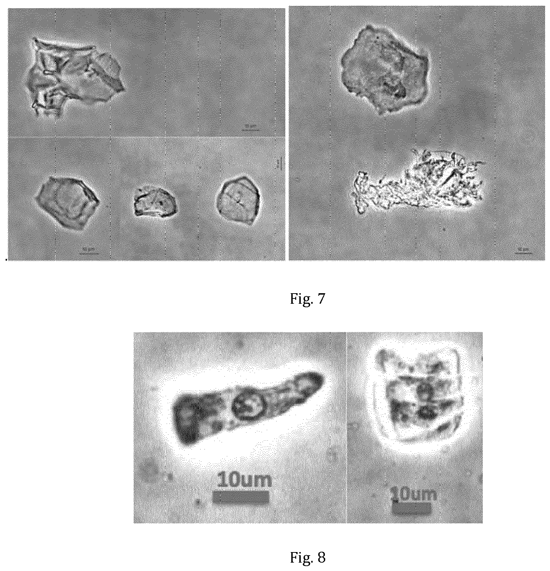

**Figure 7** shows single cells dissociated from human skin using 85% Trimethylglycine:Trifluoroacetamide (2:1 mol:mol), 26 kHz /70% Pulse / 70% Amplitude / 5 minutes at 4°C.
**Figure 8** shows single cells dissociated from frozen-thawed mouse intestine using 85% Trimethylglycine:Trifluoroacetamide (2:1 mol:mol), 26 kHz /70% Pulse / 70% Amplitude / 5 minutes at 4°C;

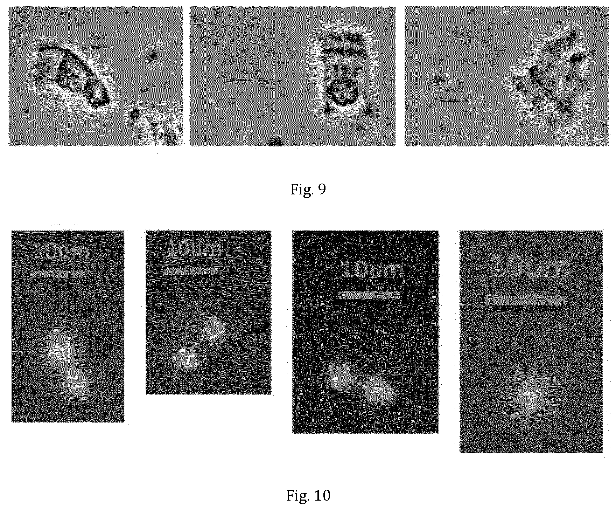

**Figure 9** shows single cells dissociated from frozen-thawed mouse nasal epithelium (brush border) using 85% Trimethylglycine:Trifluoroacetamide (2:1 mol:mol), 26 kHz /70% Pulse / 70% Amplitude / 5 minutes at 4°C;
**Figure 10** shows cells dissociated from frozen-thawed mouse kidney (DAPI stained nuclei) using 85% Trimethylglycine:Trifluoroacetamide (2:1 mol:mol), 26 kHz /70% Pulse / 70% Amplitude / 5 minutes at 4°C;
**Figure 11** shows frozen-thawed mouse liver undergoing dissociation using 85% Trimethylglycine:Trifluoroacetamide (2:1 mol:mol), 37 kHz waterbath for 5 minutes at 4°C;
**Figure 12** shows individual nuclei derived from Trimethylglycine:Trifluoroacetamide (2:1 mol:mol) 300mM Formic acid fixed mouse intestine dissociated in 100% DMSO 37 kHz water bath sonicator for 5 minutes at 24°C;

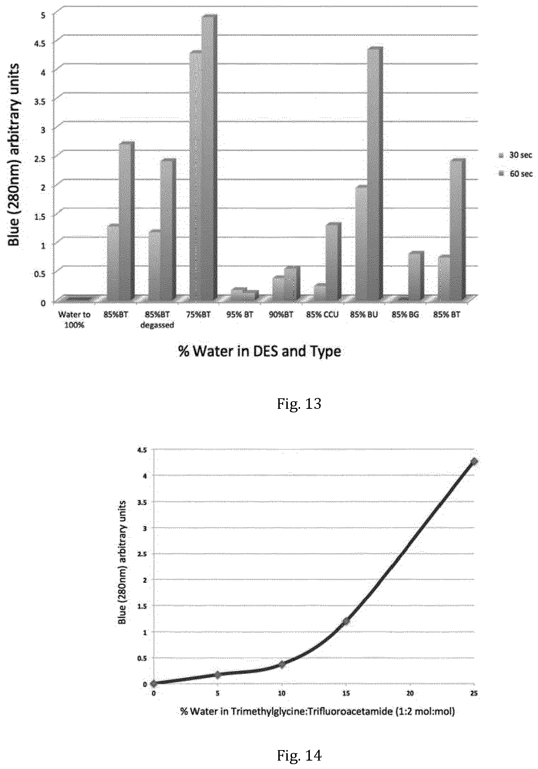

**Figure 13** shows dissolution of dextran blue (2000 Kd) in DESs with varying water content and Dissociation buffers 30 or 60 seconds sonication 35 kHz;
**Figure 14****.** shows dissolution of dextran blue (2000 Kd) in Trimethylglycine:Trifluoroacetamide (2:1 mol:mol) with varying water content using 30 seconds sonication at 35 kHz;

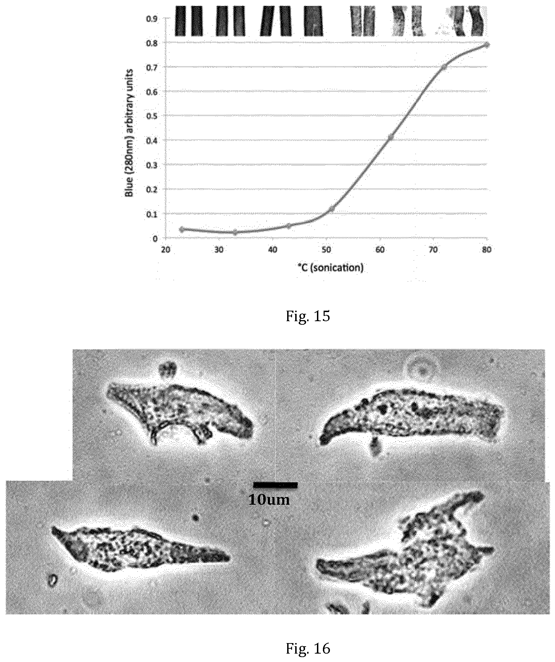

**Figure 15** shows dissolution of carbon blue paper (duplicate strips) at varying temperatures, using 30 seconds sonication in 85% Trimethylglycine:Trifluoroacetamide (1:2 mol:mol) using a 40 kHz water bath;
**Figure 16** shows single cancer cells obtained from dissociated human sarcoma, 4 minutes sonication in 95% Trimethylglycine:Trifluoroacetamide (1:2 mol:mol) using 80% Trimethylglycine:Trifluoroacetamide (1:2 mol:mol) 80% pulse / 80% Amplitude, 26 kHz according to Example 1;
**Figure 17** is a flow chart showing an example method for processing a biological sample;
**Figure 18** is a schematic diagram of an example apparatus.

### DETAILED DESCRIPTION

The terms "dissociation medium" and "dissociation buffer" are used interchangeably herein. A dissociation medium or dissociation buffer may, but does not necessarily, provide pH buffering.

As used herein, the verb 'to comprise' is used as shorthand for 'to include or to consist of'. In other words, although the verb 'to comprise' is intended to be an open term, the replacement of this term with the closed term 'to consist of' is explicitly contemplated.

The word "about" in connection with a numeric value may mean that the value may vary by, for example, ±10 %.

It will be appreciated that some compounds disclosed herein may be ionisable, i.e. some compounds may be weak acids, weak bases, or ampholytes. Representations of the free forms of ionisable compounds are intended to encompass the corresponding ionised forms. For example, representations of carboxylic acid groups encompass the corresponding carboxylate groups.

A multicellular biological sample can be either (i) fixed in a Deep Eutectic Solvent (DES) composition as set out in US Patent 9,696,247 followed by tissue dissociation, or alternatively, (ii) dissociated into single-cells in the presence of a DES composition so that fixation and dissociation occur at the same time to provide a fixed single-cell sample, or (iii) dissociated into single-cells using well-known methods such as proteases and/or mechanical means followed by fixation of the produced single cells as a suspension or pellet.

Most preferably the biomolecules found in and around the cells are stabilized before dissociation, less preferably during dissociation, or least preferably after sample dissociation. Most preferably the biomolecules are stabilized prior to dissociation in order to limit changes to their quantity, quality and distribution during the pre-analytical period. It is also important to limit pre-analytical changes to the cell morphology that may occur during tissue processing. Furthermore it is preferable that the amount, quantity and distribution of biomolecules in and around the cell remains unaltered before, during and after processing. Ideally both the cell morphology (phenotype) and biomolecules resemble, as closely as possible, their quantity, quality and molecular structure *in vivo.*

The specific biological, biophysical, physical and chemical properties of the DES are beneficial for the process of biomolecule stabilization and tissue dissociation. The particular type or combination of DES used for (i) sample fixation, (ii) tissue dissociation and (iii) downstream processing, can be the same, different or a combination of DES at each stage. There can also be more than two DESs used in the process, for example a separate DES to serve as a (i) pre-fixative, (ii) a tissue softener and (iii) a tissue Dissociation buffer, (iv) a secondary Dissociation buffer, and/or (v) a post-Dissociation stabilization buffer and/or (vi) a post-Dissociation solution for single-cell analysis such as for FACS or other types of cell sorting, protein analysis, phosphoprotein analysis, single-cell or single-nuclei sequencing of the RNA and/or DNA.

It will be evident to one skilled in the art that it is possible to supplement the DES with one or more additives to provide additional specific desired properties. The DES containing the additive can then be used for example, as a tissue pre-fixative, tissue fixation, tissue softener, tissue dissociation, post-Dissociation buffer and/or a buffer for downstream analysis of single or groups of cells.

Also provided therefore is the use of an additive in combination with a DES composition to improve tissue dissociation into single-cells or groups of cells for subsequent analysis such as single-cell RNA sequencing (scRNAseq). The purpose of the additive is to improve the properties of the DES composition, for example tissue dissociation into single-cells or groups of cells. By way of example only, the additive could be a (i) colourant or dye to aid in handling or staining or processing the tissue, (ii) an ionic or non-ionic surfactant or detergent, quaternary ammonium salt or Saponin to improve penetration of the cell plasma membrane with the DES mixture and improve tissue dissociation, (iii) anti-microbial such as an antibiotic or antiseptic, (iv) protein precipitant, (v) desiccant to remove excess water from the DES mixture to improve biomolecule stability, (vi) a probe, a hybridising complementary nucleic acid, an aptamer, a peptide, protein, nucleic acid or labelled molecule, an internal control, a blocking sequence or a carrier nucleic acid such as a nucleic acid sequence, (vii) a peptide, enzyme or other protein such as an antibody, (viii) molecules for detecting an analyte, (ix) an anti-oxidant to remove oxygen from the sample and reduce damaging oxidative effects, (x) a ribonuclease inhibitor such as Vanadate ions, RNasin, DTT, DTE, b-mercaptoethanol, a chaotrope such as Thiorurea, Urea, Sodium perchlorate, Lithium perchlorate, Sodium iodide, Potassium iodide, Guanidine thiocyanate, Guanidine HCI, an acid, Sodium trichloroacetate, (xi) buffer to stabilise the pH between 5-8 or more preferably between 6 and 7, used in the range 0.5-20 mM (g) a chelator to remove divalent metal cations, used in the range 0.005-20 mM, (xii) dissolved gas such as Carbon dioxide or Nitrogen (in the final concentration range of 0.01-5 %, a non-reactive gas such as Argon (in the final concentration range of 1-20 %) and or a buffer, (xiii) an alcohol in the range of 1-20% (wt:wt) to reduce DES viscosity such as a tert-butanol and/or (xiv) a glycol such as Glycerol, Ethylene glycol, Propylene glycol, Diethyleneglycol monoethylether acetate (DEGMEA), Polyethylene glycol in the Molecular weight range 100-6000 Daltons, more preferably 150-600 Daltons and most preferably 200-400 Daltons.

A non-exhaustive list of possible Dissociation buffer additives are, but are not limited to, one or more of the following; aqueous solvents, organic solvents, enzymes, antibodies, stains, dyes, markers, salts and chelators. Specific examples of components are water, DMSO, DMF, Urea, Thiourea, Acetamide, Acetone, Dihydroxyacetone, Formamide, Dimethylformamide, Dimethylourea, 1,3-Dimethylurea, 1,1-Dimethylurea, 1,3-Diamino-acetone, Trifluoroacetamide, 1,1,1,3,3,3-Hexafluoro-2-propanol, 1,1,1,3,3,3-Hexafluoro-2-phenyl-2-propanol, 1,1,1,3,3,3-Hexafluoro-2-methyl-2-propanol, 2,2-Difluoropropanediamine, Tetrafluorosuccinamide, 2,3,3,3-Tetrafluoropropianamide, 2,2,3,3-Tetrafluoropropanamide, Trifluoropyruvamide, Difluoropropanamide, Chlorodifluoroacetamide, Trifluoropyruvic acid, Trifluoropyruvamide, N-Hydroxymethyl trifluoroacetamide, 3,3,3-Trifluoropropionamide, Malonamide, Glycinamide, Lactamide, Formaldehyde, Glutaraldehyde, Ammonium p-toluenesulphonic acid, Sodium p-toluenesulphonic acid, Ammonium sulphate, Ammonium chloride, Ammonium thiosulphate, Sodium dodecyl sulphate, Sodium lauryl sulphate, Sodium Benzoate, Dodecyldimethyl(3-sulphopropyl)ammonium hydroxide, Dimethylbenzene sulphonic acid, Congo Red, Giemsa, DAPI, Ethidium bromide, Mallory's stain, Orcein, Aldehyde fuchin, Osmium tetroxide, Chromium trioxide, Chromic acid, Feulgen, Dichromate, Mercuric chloride, Haematoxylin and Eosin stain (H&E), Coomassie Blue, Methylene Blue, Xylene cyanol, Crystal violet, Fuchsin, Acridine orange, DAPI, Carmine, Eosin, Ethidium bromide, Bismarck brown, Hoechst, Malachite green, Methyl green, Neutral blue, Nile blue, Rhodamine or Safranin, Formaldehyde, Glutaraldehyde, Pentanol, Butanol including tert-butanol, Propanol, Ethanol, Methanol, Methyltriphenylphosphonium bromide, Cetyltetrabutylammonium bromide (CTAB), Tetrabutylammonium bromide (TBAB), Tetramethylammonium oxide (TMAO), Sodium deoxycholate, IGEPAL-CA630, Brij-35, Brij-58, NP-40, Triton X-100, Triton X-114, Tween-20, Tween-80, Octyl beta glucoside, CHAPS, Solanine, Sodium dodecyl sulphate (SDS), Sodium lauryl sulphate (SLS), kanomycin, streptomycin or penicillin, Sodium nitrate, Sodium nitrite or Sodium benzoate, kanomycin, streptomycin or penicillin, single-stranded or double-stranded RNA or DNA sequences, including labelled sequences, an aptamer specific to a cellular epitope and containing 5' and 3' polymerase amplification sites, a peptide, enzyme, antibody, biotin, biotin labelled molecule, horseradish peroxidase, avidin, streptavidin, GFP or variant thereof, Luciferase, fluorescent labelled molecule such as Fluorescein, Rhodamine, Texas Red, Alexa Fluor^{™}, LNA, labelled LNA, a Molecular Beacon^{™}, a Scorpion probe^{™}, FISH probe, bDNA, PCR primer, oligo (dT), PNA, anti-oxidant, RNasin, SUPERase•IN^{™}, RNaseOUT^{™}, phenylmethylsulfonyl fluoride (PMSF), diisopropyl fluorophosphate (DFP), aprotinin or Pefabloc SC^{™}, RNasin, SUPERase•IN^{™}, RNaseOUT^{™}, or a protease inhibitor such as phenylmethylsulfonyl fluoride (PMSF), DEPC, diisopropyl fluorophosphate (DFP), aprotinin or Pefabloc SC^{™}, Tris-HCI, PIPES, MES, HEPES, MOPS, MOPSO, CAPS, CAPSO, BIPES, phosphate, imidazole, BAPTA, EDTA, EGTA, D-Penicillamine, a dissolved gas such as a Argon, Nitrogen, Xenon, Air, Carbon dioxide, , Trichloroacetic acid, Sulphosalicyclic acid, Tetramethyl urea, Imidazole, 1-Methylimidazole, 1-Ethylimidazole, 1-Benzylimidazole, 4-Methylimidazole, N-Methylpyrrolidone, N-Ethylpyrrolidone, N-Benzylpyrrolidone, Guanidine, Guanidine HCI, Guanidine isothiocyanate, Ribitol, Rhamnose, Trehalose, D-Sorbitol, L-Sorbitol, Sorbose, Xylitol, Glucose, Sucrose, Lactose, Fructose, Maltose, Mannose, Mannitol, Arabinose, Galactose, Raffinose, Inositol, Erythritol, Xylose, Zinc acetate, Zinc EDTA, Zinc phosphate, Zinc Trifluoroacetate, Zinc citrate, Zinc PTSA, Zinc gluconate, Zinc chloride and/or Zinc sulphate, an oil, a wax, liquid Paraffin, Silica gel, Polyacrylamide, Polyacrylic acid, a Carboxylic acid such as Formic, Acetic, Propanoic, Butanoic, Lactic, Citric acids and halogenated derivatives thereof, Chloroform, Phenol, Glycerol, Ethylene glycol, Diethylene glycol, Triethylene glycol, Polyethylene glycol (PEG) with a MW of 400 to 400,000 Daltons, Beta-mercaptoethanol, DTT, DTE, TCEP, Cysteine, Cystine, a salt such as Sodium chloride, Sodium phosphate, Sodium acetate, Sodium formate, a gel containing a high molecular weight polysaccharide such as Glucomannan, Cellulose, Starch, Inulin, Amylose, a gum such as Xanthan, Arabic or Guar, a protein such as Albumin, Casein or Haemoglobin, an antibody, an enzyme, a dessicant such as Molecular sieves 4A, silica gel, Sodium acrylate, CaCl2 and/or LiCI, hard abrasive particles such as ground glass, hydroxylapatite, magnetic hydroxylapatite or silica, or ceramic, synthetic diamonds of no more than 5 µm, preferably no more than 1µm, even more preferably no more than 100 nm in diameter to serve as enhancers of tissue dissociation during sonication. The dissociation buffer may be degassed.

One particularly preferred type of additive to the DES are acids, preferably carboxylic acids, even more preferably short-chain carboxylic acids such as Propanoic, Acetic acid or most preferably Formic acid, such carboxylic acids can have 1, 2 or more carboxylic groups and contain 1, 2 or more halogen atoms such as in Trifluoroacetic acid. The preferred concentration range of the acid is set out in Example 24. The time that the DES is in contact with the sample is of particular importance and that sustained exposure of the sample to a low pH will damage the DNA, such as gDNA, and to a lesser extent the RNA. It is therefore important to add sufficient acidity to provide the desired property of the DES whilst not so much that the biomolecule is measurably damaged during the period of contact. The damage can be determined by gel electrophoresis as set out extensively in US Patent 9,696,247.

Generally as set out in US Patent 9,696,247 in order to maintain good quality RNA in mammalian samples for a week or more at 37°C it is often necessary to keep the final water content in the DES at 10% or less. It should be noted that in the present methods the RNA and other biomolecules may only need to be stabilized for a few hours or less during the dissociation and therefore the DES composition can contain significant amounts of water or other additives, for example 2, 5, 10, 15, 20, 25, 30, 35, 40, 45, 50, 55, 60, 65, 70 or 75% final concentration of water that would otherwise, over the course of several days lead to RNA degradation.

Also provided is a combination of a DES for fixation and biomolecule stabilization followed by the use of a liquid that is not a DES such as a Glycerol for subsequent dissociation. The DES can also be mixed with varying amounts of a solvent such as water, DMSO, an Alcohol, a Glycol, N-Methylpyrrolidone, Tetramethylurea, or 1-Methylimidazole to give for example a secondary solvent concentration of 99, 90, 80, 70, 60, 50, 40, 30, 20, 10, 1 or less than 1% in the DES. The DES composition can also be composed of more than one DES, for example a mixture of one part of Trimethylglycine:Trifluoroacetamide and one part of Trimethylglycine:Glycerol.

The dissociation step is not particularly limited to one process or procedure and can include mechanical, enzymatic, chemical or a combination of each. Mechanical dissociation is preferred and sonication in particular.

It will be evident to one skilled in the art that it may be necessary to empirically test the most appropriate combination of solvents, including the use of a DES with the most appropriate dissociation process for a specific tissue type. One particular preferred DES composition is Trimethylglycine:Trifluoroacetamide (1:2 mol:mol) with Formic acid as an additive in the range of 50-1000mM, optionally 100-500mM, further optionally 200-400mM, further optionally 250-350mM or 300mM final concentration; or in the range of 1-12M, more preferably 4-9M, more preferably 6-8M and most preferably 7-8M acid.

Other preferred compositions are: (i) Trimethylglycine:Trifluoroacetamide (1:2 mol:mol) with Acetic acid as an additive in the range of 2-17.39M, more preferably 8-17.39M, and most preferably 11-17.39M, or (ii) an aqueous or alcoholic (such as methanol, ethanol or propanol) dilution of Acetic acid in the range of 2-17.39M, more preferably 6-17.39M, and most preferably 9-17.39M, or (iii) pure glacial Acetic acid.

DES compositions containing carboxylic acids such as Formic acid and Acetic acid can be used either as (i) the initial primary fixative of the fresh or the fresh-frozen tissue and prior to dissociation, (ii) subsequent to fixation with a DES that does not contain carboxylic acid, such as Trimethylglycine:Trifluoroacetamide (1:2 mol:mol), or (iii) after dissociation of the DES fixed tissue, such as a secondary fixative of the single-cells produced from a dissociated tissue and preferably carried out either on (a) the cell suspension, (b) on the cells located on a filter, or (c) as a cell pellet.

DES compositions containing carboxylic acids such as Formic acid and Acetic acid can be used either as (i) the initial primary fixative of the fresh or the fresh-frozen tissue and prior to dissociation, (ii) subsequent to fixation with a DES that does not contain carboxylic acid, such as Trimethylglycine:Trifluoroacetamide (1:2 mol:mol), or (iii) after dissociation of the DES fixed tissue, such as a secondary fixative of the single-cells produced from a dissociated tissue and preferably carried out either on (a) the cell suspension, (b) on the cells located on a filter, or (c) as a cell pellet.

Solubilisation of DES fixed tissues and cells is generally an important step in the analysis of the biomolecule composition of a sample. Non-fixed fresh or frozen-thawed cell samples are generally easy to solubilise, meaning a partial or complete lysis of the cell membrane, cytoplasm, cytoplasmic contents with or without lysis of the nuclei. Many protocols require cell lysis to, for example to analyse the protein content (such as Western blotting), the DNA (such as whole genome sequencing), and the RNA (such as for RT-qPCR and scRNAseq).

Common methods to solubilise such samples include using a mild non-ionic detergent, such as Tween-20 or Triton-X100; a hypotonic solution; or stronger methods such as anionic, cationic, zwitterionic detergents; a chaotrope such as urea or guanidine, phenol, and often combined with a physical process such as shaking, bead-beating, vortexing and/or heating.

In contrast to live or frozen-thawed cells, fixed cells such as those fixed with Methanol, Acetone, Formaldehyde or a DES are much more difficult to lyse and may require harsher conditions to do so. As one example Trimethylglycine:Trifluoroacetamide (1:2 mol:mol) fixed samples and cells generally require harsher conditions to lyse them than fresh samples, which can be problematic for protocols such as Drop-Seq scRNAseq (e.g. Chromium, 10X Genomics, USA) which are not compatible with harsh cell lysis conditions (such as strong detergents, solvents and chaotropes) that may lead to both disruption of the oil-water interface of the droplets and inhibit reverse transcriptases. However other methods of scRNAseq such as Seq-Well (Gierahn et al., Nature Methods (2017) 14:395) usefully has a strong chaotrope lysis step for the input single-cells which separates the cell lysis from the sensitive reverse transcription step allowing stronger reagents to be used prior to scRNAseq.

It has been found that the commonly used chaotropes Potassium iodide, Urea, Guanidine thiocyanate and Guanidine HCI are most efficient when combined with physical disruption such as shaking. It has surprisingly been found that the chaotropes 5M Sodium perchlorate, 5M Potassium iodide and 5M Thiourea are efficient at lysing Trimethylglycine:Trifluoroacetamide (1:2 mol:mol) fixed samples. Furthermore the organic solvents beta-mercaptoethanol, 1,1,1,3,3,3-Hexafluoro-2-propanol, 1,1,1,3,3,3-Hexafluoro-2-phenyl-2-propanol, 1,1,1,3,3,3-Hexafluoro-2-methyl-2-propanol, DMSO and phenol, when used in the range of 80-100% final concentration, are particularly effective at effectuating cell lysis. Of note is that whilst DMSO very efficiently solubilises the Trimethylglycine:Trifluoroacetamide fixed cell's cytoplasm but not the nuclei, 1,1,1,3,3,3-Hexafluoro-2-propanol very efficiently solubilises both the cytoplasm and nuclei. This is important for example if studying the RNA component of the cytoplasm, nuclei or the entire cell contents as a selection of the source of the RNA can be made based on the solvent used.

### Sonication

It has been found that tissues that have been fixed with Trimethylglycine:Trifluoroacetamide (1:2 mol:mol) as set out in US patent 9,696,247 are particularly well suited to dissociation of samples into smaller collections of cells or single cells.

Due to the importance of dissociating tissues or groups of cells into single-cells, there are many different protocols that have been developed but most generally rely on either (i) mechanical, (Lima et al., J Vis Exp. (2017) 125 10.3791/55913), (ii) enzymatic (Baron et al; Cell Systems, (2016) 3, 346-360, Muraro et al., Cell Systems, (2016) 3, 385-394) or (iii) a combination of both for dissociation (Martignani et al., Methods Mol Biol. (2018); 1817:137-144).

Mechanical methods for dissociating non-fixed tissues (ie fresh or fresh-frozen) include using devices such as a Dounce homogenizer, dicing with a knife, polytron, ultrathorax, Waring blender, mortar and pestle, trituration with a glass Pasteur pipette, vortexing, specialised devices such as GentleMACS (Miltenyi, Germany) and have been described extensively in the literature (Adams. L. et al., J. Immun. Methods (2015) 426, 42-49). These rely on a variety of physical forces such as shearing, slicing, cutting, vibration, pressure, crushing to pull apart or tear or otherwise separate the tissue sample into smaller pieces such as clumps of cells or individual cells. Such methods are extensively covered in the literature for example; Wiesmann U.N. (1999) Segregating Cells - Proteases in Tissue Culture / Proteolytic Enzymes. Springer Lab Manual., Biofiles (2006) Issue 2, Sigma-Aldrich (available online).

Mechanical methods such as those discussed above may be useful in the dissociating performed in the methods provided herein.

The products of tissue dissociation can be not only single cells but also organelles, cell membranes, nuclei, molecules such as peptides, proteins, phosphoproteins, lipids and fats, RNA and DNA and small molecules of less than 500 Daltons such as sugars, amino-acids and nucleotides. The products can also be multicellular such as clumps of 2 to 99 cells, 100 to 999 cells, 1000 to 9,999 cells, 10,000 to 99,999 cells, 100,000 to 999,999, or more than a million cells. The number of cells in a sample can be determined by using a nuclear stain such as DAPI, or using a cell counter for example the TC20^{™} Automated Cell Counter (Bio-Rad Inc.), Flow cytometry, FACS, weighing the sample and dividing by the average weight of a cell etc.

Dissociation of a tissue can result in not only clumps of cells but also cell matrix, extracellular matrix, connective tissue, collagen rich material, fat, bone, cartilage and cell debris. Whilst cells are of principle interest, other components can also have utility for the diagnostic interpretation of the sample. One example is observing the density, formation, structure and shape of the extracellular matrix of the ascending thoracic aorta of Marfan patients. Another example would be to study the extracellular matrix in a cancer biopsy. Such studies can be accompanied by additional tests such as staining for the presence of collagen, for elastin, for fibrillin, an antibody or a nucleic probe. Staining of cells and tissues is extensively covered in the literature (Ross M.H. and Wojciech P. (2011) Histology. sixth edition, Walters Kluwer).

The sample is biological in origin, such as plant; fungal; animal; bacterial; algal; a parasite such as plasmodium, schistosome, trypanosome; a human blood clot; a mammalian tissue and/or a human clinical sample. The sample is not necessarily a tissue but could be other sample types such as a predominately non-cellular sample such as an egg, cornea, sclera, hair, wool, finger nails, bone, teeth, or wood.

### Definitions

**'Dissociation buffer'** is the medium used to immerse the sample in during dissociation. It can be a liquid, a gel, a wax, an oil, a biphasic or triphasic mixture, a solid or a mixture of a liquid and solid. It can also contain particles such as beads or crystals to aid in the dissociation of the tissue.

Preferably, Deep Eutectic Solvents (DESs) are used as the Dissociation Buffer. DES are particularly useful as Dissociation buffers because they are mostly viscous solutions and the viscosity can be easily modified by for example changing the composition, and therefore the amount of hydrogen bonding, increasing or decreasing the temperature or adding water. As one example, using a single Dissociation buffer composition, it is possible to reduce the viscosity by 10-100 fold by increasing the temperature by 20-80°C or adding 3-50% water. Likewise it is possible to increase the viscosity by reducing the temperature or removing water. It will be evident to one skilled in the art that it is possible to use a Dissociation buffer with a wide variety of desired properties by modifying the composition and the external environment. Properties of the Dissociation buffer that are particularly desired include; (i) viscosity, to promote dissociation of tissues into intact cells or groups of cells rather than breaking, damaging and lysing cells thereby transforming them into debris, (ii) a reducing environment to reduce or prevent oxidative damage to the sample, (iii) the pH in the range of 4-7 to reduce hydrolysis of fragile molecules in particular RNA, (iv) gas content to increase or reduce cavitation and therefore the strength of dissociation, (v) liquid convection to increase the circulation of the sample within the focus of the sonication power, (vi) a stabilizer that protects biomolecules in particular proteins, phosphoproteins, RNA and DNA, (vi) an environment that protects the structure, abundance and localization of cellular and extracellular epitopes so that downstream or *in situ* antibody binding is not compromised.

The Dissociation buffer can also be a (i) single pure substance such as Glycerol or Polyethylene glycol such as PEG200 or PEG600, (ii) a mixture such as an aqueous or organic solvent containing a buffer or a salt, (iii) a DES composition, or (iv) a DES composition containing one or more additives such as those set out herein.

**'Sample':** the sample is generally of biological origin and may comprise one or more living or dead cells or viral particles, bacteria, plant, fungal or animal origin including parasites, nematodes, insects, mollusc, fish, amphibians, reptiles, birds, mammals including human samples and clinical samples such as a cancer biopsy, a sample from an epileptic patient or a post-mortem brain sample.

The mammalian sample is not particularly limited but can be liver, spleen, brain, spinal cord, muscle, heart, oesophagus, testis, ovaries, thymus, kidneys, skin, pancreas, olfactory organ, adrenal glands, lung, smooth, skeletal or cardiac muscle, aorta, blood vessel, blood, plasma, serum, spleen, thymus, salivary gland, uterus, skin, eye, tongue, retina, oesophagus, stomach, intestine, pancreas, gallbladder, lymph nodes, bone, bone marrow, a connective tissue or cartilage.

Furthermore the sample can be or can contain a pathogen such as a prion, viroid, virus, bacteria, fungi or parasite. The sample can also include or consist of viruses such as those found in blood, in particular viruses, bacteria or parasites that are trapped, embedded in or present in blood, dried blood, a blood clot, semen, mucus, faeces, saliva, plasma, serum, amniotic fluid, or other body fluid such as cerebral spinal fluid, urine or pleural effusion.

**'Dissociation'** is the process of reducing the overall size, weight and/or complexity of a biological sample. One of the primary purposes of dissociation is for separating cells from a tissue into smaller groups of cells such as clumps of cells or individual cells. The dissociation may take place in stages, so that distinct parts of the biological sample are separated together, for example a kidney nephron may be separated during the dissociation process, from other nephrons and blood vessels before, optionally further dissociation into smaller groups of cells and/ single-cells. Dissociation can therefore be the progressive reduction in sample size with distinct steps based on tissue or organ structure, with the possibility of sampling and analysis of the partially dissociated sample at any stage. The progressive dissociation of the sample provides a means to collect groups of cells based on their original *in situ* localisation so that single-cells are subsequently obtained that were originally localized in the same part of the sample.

Provided herein are methods for reducing the complexity of a tissue sample, for example by reducing the number of cells that are attached to one another.

**'Cavitation'** is the result of the growth and collapse of a gas filled bubble in a liquid or gel in the presence of sonication energy. The size, growth and energy released when the cavitating bubble collapses is dependent on a large number of parameters including the frequency (kHz), the temperature, the viscosity of the Dissociation buffer, the type of dissolved gas, the ambient pressure in the container, the surface tension of the liquid and the sonicator pulse delay.

**'Sonication'** is the application of sound energy to a sample such as a biological tissue in order to dissociate it into smaller parts such as clumps of cells, single cells or even nuclei. Generally the sound energy has a (ultrasonic) frequency greater than 20 kHz and up to 3 MHz. The most useful frequencies have been found to be 20 kHz to 100 kHz, more preferably 20 to 60 kHz, more preferably between 20 to 40 kHz, and most preferably between 20 to 30kHz. The exact preferred frequency may be dependent on a number of factors such as the tissue type and size to be dissociated, the sample fixative composition and how long it was fixed, the Dissociation buffer composition, the dissociation temperature, the container shape, material and dimensions, and the final size of the dissociated tissue desired (clumps or single-cells). It will be evident to one skilled in the art that the exact frequency preferred has to be determined empirically although the range of frequencies available in commercial sonication devices is somewhat limited, for example 26 kHz, 36 kHz, 46 kHz or 52 kHz. It should be noted that some sonication devices can produce more than one frequency allowing a combination of frequencies to be used during the dissociation process. As examples are Ultrasonic Generator 600W, adjustable multifrequency 20-68 KHz (Cat. No. SMUG600W2068ND2 Steminc, USA), Transonic Tl-H 5, multifrequency 25/45 kHz or 35/130 kHz, and xtra ST 25/45 kHz (Elma Schmidbauer GmbH).

**'Additive'** is any substance that can be dissolved, partially dissolved, is miscible or immiscible in a DES composition, and can be present in amounts greater than, equal to or less than the total amount of the DES mixture (weight:weight). The additive such as an oil or wax may form two or more phases with the DES, or an emulsion as with a hydrophobic solvent. A list of examples of Additives is set out herein.

**'Metabolite'** is any metabolically derived biological substance with a molecular weight of less than or equal to 1000 Daltons.

**'Frequency'** is the number of vibrations created by the transducer per second, also known as Hertz (Hz). Ultrasound commonly refers to frequencies of 20 kHz or more, for example 26 kHz, 36 kHz, 52 kHz etc. and may be as much as 1 MHz, 2 MHz or even 3 MHz. Generally the lower the frequency the greater the mechanical force created, for example a frequency of 20 kHz leads to greater dissociation of a biological sample than 100 kHz or 1 MHz. Higher frequencies such as 1 MHz or more provide a means to dissociate or remove fine particles (<100 nm diameter) from a biological sample such as particles or debris from the outside of a bone or tooth but are generally not suitable for creating sufficient cavitation energy to remove cells or clumps of cells from a biological sample. In general frequencies of 1 MHz or more do not lead to cavitation in any type of Dissociation buffer. Ultrasound is the transmission of pressure waves at frequencies above human hearing, specifically above 20 kHz.

**'Power'** is the intensity (also called power density) of ultrasound and is measured in units of Watts/cm² or Watts per second.

**'Transducer'** is the mechanical device that is the source of ultrasound power, energy for the transducer is provided by the generator. Commonly the transducer is attached to a metal sonotrode or probe that transmits the ultrasound energy either directly or indirectly to the container containing the Dissociation buffer and the sample to be dissociated. Generally a transducer produces only a single frequency of ultrasound which is matched with the physical dimensions and materials of the sonotrode to produce maximum energy transfer.

**'Container'** is the enclosure which contains the Dissociation buffer and biological sample. The container may be in contact with the ultrasound energy source which can be for example a sonotrode, a block holding the container and in contact with a sonotrode, an ultrasound probe or an ultrasonic liquid filled bath. The container may have dimensions such that the ultrasound energy is sufficiently powerful to dissociate the sample, in general smaller internal volumes are preferred for optimal energy intensity, for example no more than 50 times greater, more preferably no more than 25 times and most preferably no more than 15 times the volume of the sample to be dissociated. The container is preferably made of a hard material such as polycarbonate which efficiently transmits sonication energy and is sufficiently transparent to allow the dissociation of the sample to be observed or monitored. Other hard materials include shatter-proof glass (eg. Duran), stainless steel, tungsten alloys, titanium alloys, copper alloys, aluminium alloys, polystyrene, polyamide, polymethylmethacrylate, TPX plastic and/or ceramics. The container can also be composed of 2 or more materials for example a hardwall for transmitting the sonication energy and an inlet and outlet made from flexible tubing. Thickwall carbonate centrifuge tubes manufactured by Beckman Inc., have been found to be suitable containers for sonication as they are available in a variety of sizes and wall thicknesses. Container materials are preferred that are transparent, have a hardness of at least 100 MPa (N/mm2) but are not prone to shattering.

The container can have numerous features including one or more of: an observation and/or measurement window composed of transparent material, an inlet for introducing fresh Dissociation buffer, an outlet for removing Dissociation buffer including single-cells and clumps of cells, a filter and/or membrane for removing cellular and tissue debris, cells and/or clumps of cells, a sealable cap for allowing introduction of the sample, an insert for limiting movement of the sample and/or Dissociation buffer, a mesh cassette with hole sizes of less than 1 mm for maintain the sample within an optimal sonication energy field but allowing cells and/or clumps of cells to escape from the sonication energy to protect the liberated cells from excessive damage such as cell membrane breakage, whereby the cassette fits into a holder within the container and is submerged in the Dissociation buffer, an inlet port for allowing injection or addition of a substance such as an additive, a colourant, a fluorophore, a stain, an antibody, a nucleic acid and/or a sample. The container can include a means to count, quantify the size, volume and 3D shape of a cell, specific morphological features such as brush borders, number of nuclei, mitotic index, localization of a fluorescent dye such as those for ISH and FISH analysis or antibody staining, identify, distinguish and measure organelles such as the golgi and mitochondria, or nuclei. This can be by way of a probe in contact with the Dissociation buffer, an electrode, a means to measure conductivity, a microcapillary, a capillary, an array of nanowells sufficiently proportioned that each well allows entry of a single cell for analysis, a window that is optically transparent to a laser, visible light, ultraviolet wavelengths or infrared wavelengths. The container can include a window for the detection of transmitted, emitted, absorption, reflected and/or generated wavelength frequencies.

**'Sonication Device'** is composed of one or more of the following; an ultrasound generator, a transducer, a means to transfer sonication energy to a container such as a metal block, a probe or, a liquid bath, an orifice of suitable dimensions to allow the entry of a container or vessel containing the Dissociation buffer and sample, a heating and cooling unit such as a Peltier device or water cooler, a tube holder, a pump to circulate and/or filter the Dissociation buffer and single-cells in the container capable of circulating 0.1-100ml per minute, a tube connected from the pump to the inlet of the container, and a tube connected from the outlet of the container to the pump, preferably with a cell filter such as a 1-50um mesh size nylon CellMicroSieve (BioDesign Inc, USA), PluriStrainer (PluriSelect, Germany), or a 1-50um pore size track-etched Isopore polycarbonate filter membranes (EMD Millipore, USA) or the like between the outlet and the pump, a means to quantify the number and size of dissociated material including single cells that are released from the sample into the Dissociation buffer such as lasers and detectors used for flow cytometry quantification, a secondary container where a stain, a colorant, an antibody or nucleic acid probe can be introduced to the single cells, a binding or hybridization reaction occurs, washing steps with a wash buffer, and detection of the reaction product that is detected and quantified such as by the use of a microscope, a laser, multispectral imaging, a spectrophotometer and/or a photometric detector.

The sonication device can be portable for bedside use, and may optionally include internal rechargeable batteries. Alternatively, the sonication device may be immobile for laboratory bench use and may include a touch screen for controlling, setting and adjusting power, frequency and amplitude; a screen and/or printer for observing and recording results; a means to connect to a 'smart-phone', 'tablet' or the like such as a Bluetooth (RTM) and/or wifi connection; one or more holes or orifices to allow introduction of one or more Containers including a sample and Dissociation buffer, and/or samples; a means to insert and remove a cassette or pouch containing one or more liquid filled reservoirs containing one or more of the following: binding, hybridization, reaction reagents, washing and detection reagents such as a detergent containing buffer and an antibody, nucleic acid probe, colourant and/or stain, the cassette connected to a pump or pumping device to allow delivery, mixing and recovery of the reagents added to the sample, or the sample to the reagents, an automated pipette including disposable plastic pipette tips, a waste container for removal and disposal of used reagents, disposable plastics and/or sample, wherein the sample are single-cells derived from an animal such as a clinical human biopsy such a cancer or biopsy from brain sample undergoing neurodegeneration, and/or prion particles, viral particles, bacterial cells, fungi, parasites and the like.

### Deep Eutectic Solvents

Various deep eutectic solvents (DES) are useful in the practice of the methods provided herein, for example as a dissociation medium / Dissociation buffer. The DES may be as described in International patent application publication no. WO 2014/131906 A1 to Goldsborough and Bates.

A particularly preferred DES is Trimethylglycine : Trifluoroacetamide. A preferred molar ratio of Trimethyglycine to Trifluoroacetamide is 1:2.

By way of example, but without limitation, a DES (in particular, the DES used in the dissociation buffer or dissociation medium) may be made by mixing, or mixing and heating, one or more component(s) which may be chosen from the group: Choline nitrate, Choline tetrafluoroborate, Choline hydroxide, Choline bitartrate, Choline dihydrogen citrate, Choline p-toluenesulfonate, Choline bicarbonate, Choline chloride, Choline bromide, Choline iodide, Choline fluoride, Chlorocholine chloride, Bromocholine bromide, lodocholine iodide, Acetylcholine hydroxide, Acetylcholine bitartrate, Acetylcholine dihydrogen citrate, Acetylcholine p-toluenesulfonate, Acetylcholine bicarbonate, Acetylcholine chloride, Acetylcholine bromide, Acetylcholine iodide, Acetylcholine fluoride, Chloroacetylcholine chloride, Bromoacetylcholine bromide, lodoacetylcholine iodide, Butyrylcholine hydroxide, Butyrylcholine bitartrate, Butyrylcholine dihydrogen citrate, Butyrylcholine p-toluenesulfonate, Butyrylcholine bicarbonate, Butyrylcholine chloride, Butyrylcholine bromide, Butyrylcholine iodide, Butyrylcholine fluoride, ChloroButyrylcholine chloride, BromoButyrylcholine bromide, IodoButyrylcholine iodide, Acetylthiocholine chloride, L-Carnitine, D-Carnitine, Betaine, Sarcosine, Trimethylamine N-oxide, Betaine HCI, Cetyl betaine, Cetyltrimethylammonium fluoride, Cetyltrimethylammonium chloride, Cetyltrimethylammonium bromide, Lauryl betaine, N,N-Dimethylenethanolammonium chloride, N,N-diethyl ethanol ammonium chloride, Beta-methylcholine chloride, Phosphocholine chloride, Choline citrate, Benzoylcholine chloride, Lauryl sulphobetaine, Benzyltrimethylammonium chloride, Methyltriphenylphosphonium chloride, Methyltriphenylphosphonium bromide, Methyltriphenylphosphonium iodide, Methyltriphenylphosphonium fluoride, N,N-diethylenethanol ammonium chloride, ethylammonium chloride, Tetramethylammonium chloride, Tetramethylammonium bromide, Tetramethylammonium iodide, Tetramethylammonium fluoride, Tetraethylammonium chloride, Tetraethylammonium bromide, Tetraethylammonium iodide, Tetraethylammonium fluoride, Tetrabutylammonium chloride, Tetrabutylammonium bromide, Tetrabutylammonium iodide, Tetrabutylammonium fluoride, (2-chloroethyl) trimethylammonium chloride, Terbium (III) chloride, Zinc (II) chloride, Zinc (II) bromide, Zirconium (III) chloride, Iron (III) chloride, Tin (II) chloride, Copper (II) chloride, Magnesium (II) chloride; with, one or more other component(s) that can also form a DES, including for example, but without limitation, one or more of the following chemicals chosen from the group: Urea, Formamide, Thiourea, 1-Methylurea, 1,1-Dimethylurea, 1,3-Dimethylurea, Carbohydrazide, Tetramethylurea, 1,3-bis(hydroxymethyl)urea, Benzamide, Girards Reagent T, Lactamide, Acetamide, Fluoroacetamide, Difluoroacetamide, Trifluoroacetamide, Chlorofluoroacetamide, Chlorodifluoroacetamide, Chloroacetamide, Dichloroacetamide, Dichlorofluoroacetamide, Trichloroacetamide, Bromoacetamide, Dibromoacetamide, Tribromoacetamide, Bromofluoroacetamide, Bromodifluoroacetamide, Bromochlorofluoroacetamide, lodoacetamide, Diiodoacetamide, Triiodoacetamide, 2-Methyl-2,2-difluoroacetamide, 2-Methyl-2-fluoroacetamide, 2,2-Dimethyl-2-fluoroacetamide, 2-Ethyl-2,2-difluoroacetamide, 2-Ethyl-2-fluoroacetamide, 2,2-Diethyl-2-fluoroacetamide, 2-Propyl-2,2-difluoroacetamide, 2-Propyl-2-fluoroacetamide, 2,2-Propyl-2-fluoroacetamide, 2-Fluoropropionamide, 3-Fluoropropionamide, 2,2-Difluoropropionamide, 2,3-Difluoropropionamide, 3,3-Difluoropropionamide, 3,3,3-Trifluoropropionamide, 2-Fluoro-3,3,3-trifluoropropionamide, 2-Chloro-3,3,3-trifluoropropionamide, 2,2-Chloro-3,3,3-trifluoropropionamide, 2-bromo-3,3,3-trifluoropropionamide, 2,2-Bromo-3,3,3-trifluoropropionamide, Pentafluoropropionamide, Heptafluorobutyramide, Trimethylacetamide, 1-(Trifluoroacetyl)imidazole, N,O-Bis(trifluoroacetyl)hydroxylamine, Bistrifluoroacetamide, N-Methyl-fluoroacetamide, N-Methyl-difluoroacetamide, N-Methyl-trifluoroacetamide, N-Methyl-chlorofluoroacetamide, N-Methyl-chlorodifluoroacetamide, N-Methyl-chloroacetamide, N-Methyl-dichloroacetamide, D N-Methyl-dichlorofluoroacetamide, N-Methyl-trichloroacetamide, N-Methyl-bromoacetamide, N-Methyl-dibromoacetamide, N-Methyl-tribromoacetamide, N-Methyl-bromofluoroacetamide, N-Methyl-bromodifluoroacetamide, N-Methyl-bromochlorofluoroacetamide, N-Methyl-iodoacetamide, N-Methyl-diiodoacetamide, N-Methyl-triiodoacetamide, N-Methyl-2-methyl-2,2-difluoroacetamide, N-Methyl-2-methyl-2-fluoroacetamide, N-Methyl-2,2-dimethyl-2-fluoroacetamide, N-Methyl-2-ethyl-2,2-difluoroacetamide, N-Methyl-2-ethyl-2-fluoroacetamide, N-Methyl-2,2-diethyl-2-fluoroacetamide, N-Methyl-2-propyl-2,2-difluoroacetamide, N-Methyl-2-propyl-2-fluoroacetamide, N-Methyl-2,2-propyl-2-fluoroacetamide, N-Methyl-2-fluoropropionamide, N-Methyl-3-fluoropropionamide, N-Methyl-2,2-difluoropropionamide, N-Methyl-2,3-difluoropropionamide, N-Methyl-3,3-difluoropropionamide, N-Methyl-3,3,3-trifluoropropionamide, N-Methyl-2-fluoro-3,3,3-trifluoropropionamide, N-Methyl-2-chloro-3,3,3-trifluoropropionamide, N-Methyl-2,2-chloro-3,3,3-trifluoropropionamide, N-Methyl-2-bromo-3,3,3-trifluoropropionamide, N-Methyl-2,2-bromo-3,3,3-trifluoropropionamide, N-Methyl-pentafluoropropionamide, N-Methyl-heptafluorobutyramide, N,N-Dimethyl-2,2,2-trifluoroacetamide, N-Ethyl-2,2,2-trifluoroacetamide, N,N-Diethyl-2,2,2-trifluoroacetamide, N-(Hydroxymethyl)Trifluoroacetamide, Ethyltrifluoroacetate, Dithiothreitol, Dithioerythritol, Beta-mercaptoethanol, Penicillamine, Tiopronin, Acrylamide, Methanol, Ethanol, Propanol, Butanol, Formaldehyde, Glutaraldehyde, Taurine, Aconitic acid, Adipic acid, Benzoic acid, Citric acid, Malonic acid, Malic acid, DL-Maleic acid, Oxalic acid, Phenylacetic acid, Phenylpropionic acid, Succinic acid, Levulinic acid, Tartaric acid, Gallic acid, p-Toluenesulphonic acid, Glycine, Alanine, Valine, Leucine, Isoleucine, Serine, Threonine, Tyrosine, Cysteine, Methionine, Aspartic acid, Asparagine, Glutamic acid, Glutamine, Arginine, Lysine, Histidine, Phenylalanine, Tryptophan, Proline, Ethylene glycol, Triethyleneglycol, Glycerol, Resorcinol, Phenol, 1,2-propanediol, 1,3-propanediol, 1,4-Butanediol, 1,5-Pentanediol, 1,6-Hexanediol, 1,8-Octanediol, 1,12-Dodecanediol, m-Cresol, Imidazole, 1-Methylimidazole, 4-Methylimidazole, N-Methylpyrrolidone, N-Ethylpyrrolidone, N-Benzylpyrrolidone, 2-imidazolindone, tetrahydro-2-pyrimidione, Guanidine, Guanidine HCI, Guanidine isothiocyanate, Guanidine sulphate, Ammonium acetate, Ammonium bicarbonate, Ammonium chloride, Ammonium citrate dibasic, Ammonium formate, Ammonium iodide, Ammonium nitrate, Ammonium phosphate monobasic, Ammonium phosphate dibasic, Ammonium sulfamate, Ammonium sulfate, Ammonium tartrate dibasic, Ammonium isothiocyanate, Ammonium benzoate, Ammonium bromide, Ammonium fluoride, Ammonium hydrogensulphate, Ammonium trifluoroacetate, Ammonium thiosulphate, Adonitol, Ribitol, Rhamnose, Trehalose, D-Sorbitol, L-Sorbitol, Sorbose, Xylitol, Glucose, Sucrose, Lactose, Fructose, Maltose, Mannose, Mannitol, Arabinose, Galactose, Raffinose, Inositol, Erythritol or Xylose.

It should be noted that certain components such as Zinc chloride can form a DES with not only Choline Chloride but also Urea; both Choline chloride and Zinc chloride appear in the same component group above, therefore certain DES mixtures can be prepared from chemicals within the same group.

It should also be noted that the lists above are non-exhaustive, and that the DES components are not particularly limited to any type of molecule or property except hydrogen-bonding between DES components is frequent but not an absolute requirement.

In other arrangements, a deep eutectic solvent may comprise a metal salt and a sugar or sugar alcohol.

It should also be noted that there is no particular maximum number of DES components in the DES mixture, for example 2, 3, 4, 5, 6, 7, 8, 9, 10 or more components can be mixed to produce a DES mixture, usually but necessarily, in integer molar ratios for example in a two component DES mixture, component 1 and component 2 can be mixed in the following ratios: 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10 (mol:mol) or for example in a three component DES mixture, component 1, component 2 and component 3 can be mixed in these ratios: 10:1:1, 9:1:1, 8:1:2, 7:1:3, 6:1:4, 5:1:5, 4:1:6, 3:1:7, 2:1:8, 1:1:9, 1:2:8, 1:3:7, 1:4:6, 1:5:5, 1:6:4, 1:7:3, 1:8:2, 1:9:1, 1:10:1 (mol:mol:mol).

The skilled artisan will understand that other ratios of mixing the components are also possible, for example 20:1 or 1:30 (mol:mol) and that the particular molar ratio leading to the eutectic point is not necessarily the optimum or desired molar ratio for the particular purpose of, for example, stabilising RNA and/or fixing cell morphology. The stabilisation activity of the DES may be related to the hydrogen bonding properties of the DES mixture. There is no particular restriction or limitation to the number of components or the ratio of mixing to make a useful DES mixture for a particular application. Additional physical properties of the DES for example viscosity or density can be modified by either adding a further component, or alternatively, by adding an 'additive' as set-out below. Some DES mixtures can be prepared using a fractional molar ratio, for example ZnCl2:urea has been prepared at 1:3.5 (mol:mol) ratio and there are again no particular restrictions to the molar ratios of component 1 and component 2, or component 1, component 2 and component 3 etc. that are used, and the optimum ratio of the components comprising the DES mixture to be used for the application, such as cell fixation, is most accurately determined empirically. By way of example only, Choline chloride/Trifluoroacetamide can be mixed in a 2:1, 1.75:1, 1.5:1, 1.25:1, 1:1, 1:1.25, 1:1.5, 1:1.75 or a 1:2 mol:mol ratio, or further fractional amounts thereof, and subsequently individually tested to determine which has the optimum RNA stabilization and/or cell fixation property.

Other factors such as the cost or toxicity may also lead the artisan to reduce the proportion of a particular component and to partially replace it with a cheaper alternative. For example only, an Acetylcholine chloride:urea (1:2 mol:mol) DES mixture can, for certain applications be partially replaced with the cheaper Choline chloride as in Acetylcholine chloride:Choline chloride:urea (1:1:4 mol:mol:mol).

It will also be apparent to the artisan that due to, for example, the presence of contaminants in the components used to make the DES, such as water, oxidation, absorption of CO2, contaminating byproducts during synthesis or break-down products of one of the components, and that the desired exact ratio, for example only, of a 1:1 (mol:mol) mixture may practically mean +/- 10%, but more preferably 5% and even more preferably 1% difference in the exact amount of either component 1 or component 2. As way of illustration, a 5% error could potentially give the following final molar ratios: 0.95:1, 1:0.95, 1.05:0.95 or 0.95:1.05 (mol:mol) and the effect of this error can generally only be determined empirically, for example its effect on RNA quality as set-out in Example 1. Generally in order to remove contaminants including water, the well-known procedure of recrystallisation in ethanol can be carried out followed by extensive vacuum and/or chemical drying. Methods are set-out in 'Purification of Laboratory Chemicals' Butterworth-Heinemann (2012).

Typically, but certainly not exclusively, a DES is prepared by mixing a hydrogen bond acceptor from one of the following classes of chemicals; (i) a nitrogen salt with a positively charged cation such as primary, secondary, tertiary or quaternary nitrogen, one example of a nitrogen salt is one with a halide, such as Choline chloride, (ii) a metal salt such as a transition metal salt halide, with, a hydrogen bond donor which may include (iii) an amine, (iv) a hydroxyl, (v) an aldehyde, (vi) an amide, or (vii) carboxylic acid, hydrogen bond donors such as sugars, carboxylic acids, ureas such as Trifluoroacetamide, and alcohols would thus be included.

In one arrangement, the deep eutectic solvent is a type III or a type IV deep eutectic solvent, wherein the RNA extracted from 10 mg of a rat liver sample incubated at 24 ºC for 20 days with 400 mg of the deep eutectic solvent has an RNA integrity number (RIN) of at least 4.0 as measured using an Agilent Bioanalyser 2100.

RNA integrity number may be measured using an RNA 6000 Nano total RNA Kit (Cat. No. 5067-1511, Agilent Technologies, USA) and a Bioanalyser 2100 instrument (Cat. No. G2939AA, Agilent Technologies, USA).

As used herein, the term "type III deep eutectic solvent" refers to a deep eutectic solvent having at least a first component and a second component, wherein the first component is a quaternary ammonium or phosphonium compound such as choline chloride or N,N,N-trimethylglycine (betaine), and wherein the second component is a hydrogen bond donor, such as urea or trifluoroacetamide. A type IV deep eutectic solvent is a deep eutectic solvent comprising at least a first component and a second component, wherein the first component is a metal salt such as zinc chloride and wherein the second component is a hydrogen bond donor, such as urea.

Optionally, the deep eutectic solvent is a type III deep eutectic solvent, wherein the deep eutectic solvent comprises a compound comprising a trifluoromethyl group. The compound comprising the trifluoromethyl group may be present in the deep eutectic solvent in an amount of at least 5 % by weight of the deep eutectic solvent.

In one arrangement, the deep eutectic solvent comprises a first component and a second component, wherein first component is a compound of Formula I: 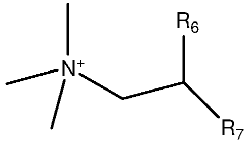 wherein:
R₆ is H or OH;
R₇ is selected from H, CH₃, Cl, Br, a carbonyl oxygen, and 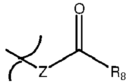
   Z is selected from -CH₂-, O and S;
   R₈ is R₁₁ or OH; and
   wherein the second component comprises a compound of Formula II or a salt thereof: 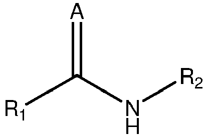 wherein:
      A is selected from O, S, and NH;
      R₁ is selected from H, an alkene group having 1 to 6 carbon atoms, R₉, -NH₂, -NH-(CH₂)ₐCH₃, and -C(R₃)(R₄)(R₅);
         wherein a is 0 or an integer from 1 to 5;
      R₂ is selected from H and linear alkyl group having 1 to 3 carbon atoms;
      R₃ is an optionally substituted 5- or 6- membered aliphatic or aromatic ring, wherein
      the substituent is R₁₀;
      R₄ and R₅ are each independently H or F; and
wherein R₉, R₁₀, and R₁₁ are each independently selected from alkyl groups having one to three carbon atoms, monochloroalkyl groups having one to three carbon atoms, and mono-, di- or tri-fluoroalkyl groups having one to three carbon atoms.

Preferably, in this arrangement, A is selected from O, S, and NH; R1 is selected from H, -CH=CH2, R9, - NH2, -NHCH3, and -C(R3)(R4)(R5); R2 is selected from H and -CH3; R3 is an optionally substituted 5- or 6- membered aliphatic or aromatic ring, wherein the substituent is R10; R4 and R5 are each independently H or F; and R9, R10, and R11 are each independently selected from alkyl groups having one to three carbon atoms, monochloroalkyl groups having one to three carbon atoms, and mono-, di- or tri-fluoroalkyl groups having one to three carbon atoms. Optionally, R7 is selected from H, Br, a 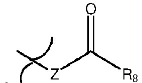 carbonyl oxygen, and
A may be O or S.
R₂ may be H.
R₁ may be selected from H, R₉, -CH=CH₂, and C(R₃)(R₄)(R₅). In this arrangement, it is preferred that R₁ is R₉, wherein R₉ preferably has one carbon atom.

Preferably, the second component is acetamide, or 2-chloroacetamide.

In another arrangement, R₉ is a mono-, di- or tri-fluoromethyl group.

The second component is preferably trifluoroacetamide, trifluorothioacetamide or N-methyltrifluoroacetamide.

In another arrangement, R₉ has two carbon atoms, preferably wherein R₉ is a mono-, di- or trifluoroethyl group.

It is preferred that the second component is 2,2-difluoropropanamide or 3,3,3-trifluoropropanamide. In another arrangement, the second component is formamide or acrylamide.

In a further arrangement, R₁ is C(R₃)(R₄)(R₅), wherein R₄ and R₅ are preferably F.

R₃ may be an optionally substituted 6-membered aromatic ring, such as an optionally substituted phenyl group. Preferably, the first component is 2,2-difluoro-2-phenylacetamide. Alternatively, R₃ comprises a substituent, preferably at the 2-position of the phenyl group.

The substituent is preferably a mono-, di- or tri-fluoromethyl group. Thus, in one arrangement the second component is 2-(trifluoromethyl)phenyl acetamide.

In a further arrangement R₁ is selected from -NH₂ and -NHCH₃. Preferably, the second component is urea, thiourea or 1,3-dimethylurea.

In a further arrangement, A is NH. In this arrangement the second component may be guanidine, optionally wherein the guanidine is present in the form of a hydroisothiocyanate salt.

In a further arrangement, R₇ is H. In this arrangement the first component preferably comprises choline.

In another arrangement, the first component comprises bromocholine.

In another arrangement, the first component is N,N,N-trimethylglycine, optionally wherein the second component is selected from trifluoroacetamide and urea.

In another arrangement R₇ is

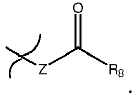

In this arrangement Z may be O or 5 and R₈ may be R₁₁, wherein R₁₁ preferably has one carbon atom.

In this arrangement the first component preferably comprises acetylcholine or acetylthiocholine.

Alternatively R₁₁ may have three carbon atoms, wherein the first component preferably comprises butyrylcholine.

In a further arrangement Z may be CH₂. In this arrangement, R₈ may be OH. In this arrangement, the second component is preferably carnitine.

The first component may comprise a counterion, which counterion is typically a halide anion but can be another anion such as nitrate (NO3-) or tetrafluoroborate (BF4-). The halide anion may be selected from fluoride, chloride, bromide, and iodide, preferably chloride. In this arrangement it is preferred that the first component is choline chloride, optionally wherein the second component is selected from trifluoroacetamide, trifluorothioacetamide; 3,3,3-trifluoropropanamide; 2,2-difluoro-2-phenylacetamide; thiourea and urea, preferably trifluoroacetamide.

The molar ratio of the first component to the second component is in the range 1:3 to 2:1, preferably in the range 1:1.5 to 1:2.5, more preferably 1:2.

In a further arrangement, the deep eutectic solvent comprises a first component and a second component, wherein the first component is a halide salt of choline; and wherein the second component is an optionally-substituted imidazole, wherein the or each substituent is an alkyl group having 1 to 3 carbon atoms. In this arrangement, the molar ratio of the first component to the second component is preferably in the range 2.8:1 to 2:1.

The halide salt of choline is preferably choline chloride and the substituted imidazole is preferably a methyl imidazole, such as N-methylimidazole or 4-methylimidazole. The use of benzyl-substituted imidazoles such as 1-benzylimidazole is also contemplated. Alternatively, the imidazole is unsubstituted.

In a further arrangement, the deep eutectic solvent comprises a first component and a second component, wherein the first component comprises a compound of Formula III: 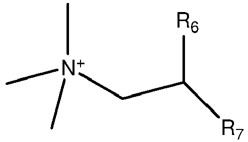 wherein:
R₆ is H or OH;
R₇ is selected from H, CH₃, Cl, Br, a carbonyl oxygen, and 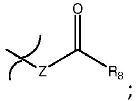
Z is selected from -CH₂-, O and S;
wherein R₈ is selected from OH, an alkyl group having one to three carbon atoms, a monochloroalkyl group having one to three carbon atoms, and a mono-, di- or tri-fluoroalkyl group having one to three carbon atoms; and
wherein the second component is a sugar or a sugar alcohol having at least 3 carbon atoms.

R7 is optionally selected from H, Br, a carbonyl oxygen, and

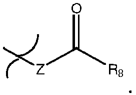

The sugar alcohol may be selected from glycerol, thrietol, xylitol, sorbitol and volemitol, preferably from glycerol, xylitol and sorbitol, and is most preferably sorbitol. Alternatively, the sugar may be trehalose.

In this arrangement, the second component may comprise choline, such as choline chloride. The molar ratio of the first component to the second component may be in the range 1:2 to 2:1, preferably in the range 1:0.8 to 1:1.2.

In a further arrangement, the deep eutectic solvent comprises a first component and a second component, wherein the first component is a zinc (II) halide or zirconium (IV) halide, and wherein the second component is a compound of Formula IV: wherein: 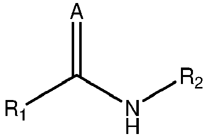
A is selected from O, S, and NH;
R₁ is selected from H, an alkene group having 1 to 6 carbon atoms, R₉, -NH₂, -NH-(CH₂)ₙCH₃, and - C(R₃)(R₄)(R₅);
wherein n is 0 or an integer from 1 to 5;
R₂ is selected from H and linear alkyl group having 1 to 3 carbon atoms;
R₃ is an optionally substituted 5- or 6- membered aliphatic or aromatic ring, wherein the substituent is R₁₀;
R₄ and R₅ are each independently H or F; and
wherein R₉ is selected from alkyl groups having one to three carbon atoms, monochloroalkyl groups having one to three carbon atoms, and mono-, di- or tri-fluoroalkyl groups having one to three carbon atoms.

The preferred zinc (II) halide is ZnCl₂. The preferred zirconium (IV) halide is ZrCl₄. The second component is preferably urea. The molar ratio of the first component to the second component may be in the range 1:3 to 1:4.

The second component is preferably urea.

In a further arrangement, the deep eutectic solvent comprises a first component and a second component, wherein the first component is a compound of Formula V: 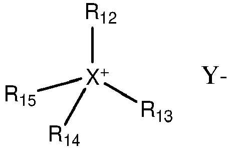 wherein:
Y⁻ is Cl⁻ or Br⁻;
X is N or P;
R₁₂, R₁₃, R₁₄ and R₁₅ are each independently a linear alkyl group having 1 to 16 carbon atoms, a linear alcohol group having 1 to 16 carbon atoms, a benzyl group, or a phenyl
group;
   wherein the second component is a compound of Formula I or a salt thereof: 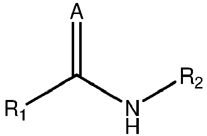 wherein:
   A is selected from O, S, and NH;
   R₁ is selected from H, -CH=CH₂, R₉, -NH₂, -NHCH₃, and -C(R₃)(R₄)(R₅);
   R₂ is selected from H and -CH₃;
   R₃ is an optionally substituted 5- or 6- membered aliphatic or aromatic ring, wherein the substituent is R₁₀; and
   R₄ and R₅ are each independently H or F;
wherein R₉ and R₁₀ are each independently selected from alkyl groups having one to three carbon atoms, monochloroalkyl groups having one to three carbon atoms, and mono-, di- or tri-fluoroalkyl groups having one to three carbon atoms.

Y⁻ may be Cl⁻. In alternative arrangements, Y⁻ may be any suitable counterion, such as a halide, nitrate, or tetrafluoroborate.

X may be N; in this arrangement, the compound of Formula V is a quaternary ammonium salt.

Optionally, R₁₂, R₁₃, R₁₄ and R₁₅ are each independently a linear alkyl group having 1 to 16 carbon atoms, a benzyl group, or a phenyl group

R₁₂, R₁₃, R₁₄, and R₁₅ may be each independently selected from linear alkyl groups having 1 to 4 carbon atoms, and are preferably each methyl groups. In this arrangement the first component is preferably tetramethylammonium chloride.

Alternatively, R₁₂, R₁₃, R₁₄, and R₁₅ are each ethyl groups. In this arrangement the first component is preferably tetraethylammonium chloride.

Alternatively R₁₂, R₁₃, R₁₄, and R₁₅ are each butyl groups. In this arrangement the second component is tetrabutylammonium chloride or tetrabutylammonium bromide.

Alternatively, the first component comprises:

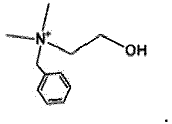

Alternatively, the first component comprises:

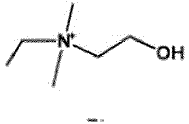

In another arrangement X may be P.

At least one of R₁₂, R₁₃, R₁₄, and R₁₅ may be a phenyl group. In this arrangement the first component preferably comprises methyltriphenylphosphonium and may be methyltriphenylphosphonium bromide.

In another arrangement A may be O or S.

R₁ may be selected from -NH₂ and -NHCH₃ and the second component is preferably urea. Alternatively the second component may be trifluorothioacetamide. Alternatively the second component may be trifluoroacetamide.

The molar ratio of the first component to the second component may be 1:1.5 to 1:2.5, preferably 1:1.8 to 1:2.2.

In a further arrangement the deep eutectic solvent comprises a first component and a second component, wherein the first component comprises choline and wherein the second component is an alkanediol having 5 to 7 carbon atoms. The alkanediol is preferably hexanediol.

In a still further arrangement the deep eutectic solvent comprises a first component and a second component, wherein the first component comprises choline and wherein the second component comprises an N-alkyl pyrrolidone, wherein the N-alkyl group has 1 to 5 carbon atoms. Preferably, the N-alkyl pyrrolidone is N-methylpyrrolidone. Preferably, the choline is choline chloride. In this arrangement, the molar ratio of the first component to the second component may be 1:2.

In another arrangement, the deep eutectic solvent comprises a first component and a second component, wherein the first component comprises choline, and wherein the second component comprises beta-mercaptoethanol. In this arrangement, the choline may be choline chloride and/or the molar ratio of the first component to the second component may be about 1:2.

In another arrangement, the deep eutectic solvent comprises a first component and a second component, wherein the first component comprises choline, and wherein the second component comprises dithiothreitol. The choline may be choline chloride. The ratio of the first component to the second component may be about 1:2.

The eutectic point of a two or more component DES mixture is the point where the proportion of component 1 relative to the other component(s) leads to the lowest melting temperature. For example the eutectic point of a Choline chloride:Urea DES mixture is (1:2 mol:mol) with a reported freezing point (Fp) of 12°C. From a practical stand-point this means that the DES can be handled as a liquid at room-temperature but will slowly solidify in the fridge. Other DES mixtures, particularly those containing Choline chloride mixed with Glycerol, Ethylene glycol and Trifluoroacetamide or Phenylacetic acid have much lower Fp, remaining liquid below 0°C or even -20°C. Although handling liquids provides certain advantages such as ease of measuring exact volumes and mixing, the present methods are not limited to DES mixtures that are liquid at room-temperature of any other particular temperature. Heating and mixing the components of a DES provides a means for individual atoms and molecules to establish hydrogen bonds or other interactions with other atoms and molecules and therefore produce the particular property of the DES mixture. Once this has occurred, whether the DES mixture is a liquid, gel or solid does not necessarily change its capacity to, for example, stabilise RNA in a tissue sample. Simple contact between the tissue sample and the DES mixture will occur even when the DES is a solid at room temperature and rapid stabilisation can occur. Although traditionally RNA stabilisation reagents are liquids such as RNAlater, AllProtect, PAXgene Tissue or formalin, the methods described here can make use of the liquid, gel or solid DES RNA stabilisation and/ or cell fixation properties. Indeed the use of a solid form of a DES can be advantageous when handling tissue samples; a solid block of DES can be formed by heating the DES solid above its eutectic point so that it melts and then pouring it into a suitable receptacle to cool and solidify or, molded or cut to fit a specific container and the tissue sample placed on the solid DES surface to bring about stabilisation. Conveniently wells, depressions or grooves can be made in the solid DES surface to serve as specific storage locations for each sample, one well receiving one sample etc, advantageously with the use of a well, the surface area in contact between the solid DES and the sample is increased, whilst an additional layer of a DES solid or liquid above the sample located in the well will further increase the total amount of DES available and surface area of contact with the sample.

It may be useful to use an additive in combination with a DES mixture. The purpose of the additive is to improve the properties of the DES mixture, for example, RNA, DNA and/or protein stabilisation and/or cell and tissue fixation or another application. The effects of various additives on cell morphology for example are set out in Table A. The purpose of the additive is to enhance the property of the DES mixture for the particular application, for example RNA stabilisation, storage, transport, and/or cell or tissue fixation. By way of example only, the additive could be a (i) colourant or dye to aid in handling or staining or processing the tissue such as H&E stain, Coomasie Blue, Methylene Blue, Xylene cyanol, Crystal violet, fuchsin, Acridine orange, DAPI, Carmine, Eosin, Ethidium bromide, Bismarck brown, Hoechst, Malachite green, Methyl green, Neutral blue, Nile blue, Osmium tetroxide, Rhodamine or Safranin, (ii) detergent, quaternary ammonium salt or saponin to improve penetration of the cell plasma membrane with the DES mixture, such as SDS, sodium lauryl sulphate (SLS), cetyltetrabutylammonium bromide (CTAB), tetrabutylammonium bromide (TBAB), sodium deoxycholate, Brij-35, Brij-58, NP-40, Triton X-100, Triton X-114, Tween-20, Tween-80, Octyl beta glucoside, CHAPS, Solanine, (iii) anti-microbial such as an antibiotic or antiseptic, such as kanomycin, streptomycin or penicillin, sodium nitrate, sodium nitrite or sodium benzoate, (iv) protein precipitant such as Trichloroacetic acid, Ammonium sulphate, Sulphosalicyclic acid, Zinc salt such as ZnCl2 or ZnSO4., (v) desiccant to remove excess water from the DES mixture such as Molecular sieves 4A, silica gel, CaCl2 or LiCI, (vi) a probe, a hybridising complementary nucleic acid, a peptide, protein, nucleic acid or labelled molecule, an internal control, a blocking sequence or a carrier nucleic acid such as (a) ss or ds RNA or DNA sequences, (b) a peptide, enzyme or other protein such as an antibody, (c) molecules for detecting an analyte such as a biotin, horseradish peroxidase, avidin, streptavidin, fluorescent labelled molecule such as fluorescein, Texas Red, Alexa Fluor^{™} labelled LNA, (d) a Molecular Beacon^{™}, a Scorpion probe^{™}, (vii) anti-oxidant to remove oxygen from the sample and reduce damaging oxidative effects during storage on for example the RNA or DNA nucleobases, such as Vitamin C or glutathionine, (viii) ribonuclease inhibitor such as RNasin, SUPERase•IN^{™}, RNaseOUT^{™}, or a protease inhibitor such as phenylmethylsulfonyl fluoride (PMSF), diisopropyl fluorophosphate (DFP), aprotinin or Pefabloc SC^{™}, (ix) buffer to stabilise the pH between 5-8 or more preferably between 6 and 7, used in the range 0.5-20mM such as Tris-HCI, PIPES, MES, HEPES, MOPS, MOPSO, CAPS, CAPSO, BIPES, phosphate, imidazole, (x) chelator to remove divalent metal cations, used in the range 0.5-20mM such as BAPTA, EDTA, EGTA, citric acid, D-Penicillamine, (xi) dissolved oxygen (in the final concentration range of 2-20%), and/or CO2 (in the final concentration range of 0.01-5%, a non-reactive gas such as Argon (in the final concentration range of 1-20%) and or a buffer, nutrients (such as glucose and amino-acids) to enhance cell, tissue and organism viability, and/or (xii) an alcohol in the range of 1-20% (wt:wt) to reduce DES viscosity such as a tert-butanol.

**Table A. Effects of Various DES Mixtures with or without additives on Cell Morphology.**

| | **Component 1** | **Component 2** | **Ratio (mol:mol)** | **Additive Final %** | **Effect on HeLa Morph ology** |
|---|---|---|---|---|---|
| 1 | Choline chloride | Trifluoroacetamide | 1:1.8 | - | ++++ |
| 2 | Choline chloride | Trifluoroacetamide | 1:2 | - | ++++ |
| 3 | Choline chloride | Trifluoroacetamide | 1:2.25 | - | ++ |
| 4 | Choline chloride | Trifluoroacetamide | 1:2.5 | - | ++++ |
| 5 | Choline chloride | Trifluoroacetamide | 1:2.75 | - | ++++ |
| 6 | Choline chloride | Trifluoroacetamide | 1:3 | - | +++++ |
| 7 | Choline chloride | Trifluoroacetamide | 1:2 | H2O (17%) | +++++ |
| 8 | Choline chloride | Trifluoroacetamide | 1:2 | H2O (13%) | +++ |
| 9 | Choline chloride | Trifluoroacetamide | 1:2 | H2O (10%) | +++ |
| 10 | Choline chloride | Trifluoroacetamide | 1:2 | H2O (5%) | ++++ |
| 11 | Choline chloride | Trifluoroacetamide | 1:2 | H2O (2.5%) | +++ |
| 12 | Choline chloride | Trifluoroacetamide | 1:2 | Ethylene glycol (17%) | ++ |
| 13 | Choline chloride | Trifluoroacetamide | 1:2 | 1,6-Hexanediol (17%) | +++ |
| 14 | Choline chloride | Trifluoroacetamide | 1:2 | Ethanol (17%) | ++++ |
| 15 | Choline chloride | Trifluoroacetamide | 1:2 | Methanol (17%) | +++ |
| 16 | Choline chloride | Trifluoroacetamide | 1:2 | Dimethylformamide (17%) | + |
| 17 | Choline chloride | Trifluoroacetamide | 1:2 | Dimethylsulphoxide (17%) | +++ |
| 18 | Choline chloride | Trifluoroacetamide | 1:2 | N-Methyl pyrrolidone (17%) | +++++ |
| 19 | Choline chloride | Trifluoroacetamide | 1:2 | N-Ethyl pyrrolidone (5%) | +++++ |
| 20 | Choline chloride | Trifluoroacetamide | 1:2 | Ethyleneurea (5%) | ++ |
| 21 | Choline chloride | Trifluoroacetamide | 1:2 | Pivalamide (5%) | +++ |
| 22 | Choline chloride | Trifluoroacetamide | 1:2 | 1,3-Dimethylurea (5%) | ++ |
| 23 | Choline chloride | Trifluoroacetamide | 1:2 | N,N'-Dimethylourea (5%) | ++ |
| 24 | Choline chloride | Trifluoroacetamide | 1:2 | Isopropanol (17%) | ++++ |
| 25 | Choline chloride | Trifluoroacetamide | 1:2 | Butanol (17%) | ++++ |
| 26 | Choline chloride | Trifluoroacetamide | 1:2 | Glycerol (17%) | ++ |
| 27 | Choline chloride | Trifluoroacetamide | 1:2 | 1-Methylimidazole (33%) | +++++ |
| 28 | Choline chloride | Trifluoroacetamide | 1:2 | 1-Methylimidazole (5%) | ++ |
| 29 | Choline chloride | Trifluoroacetamide | 1:2 | 1-Ethylimidazole (5%) | ++ |
| 30 | Choline chloride | Trifluoroacetamide | 1:2 | 1-Benzylimidazole (2.5%) | +++++ |
| 31 | Choline chloride | Trifluoroacetamide | 1:2 | 1-Benzylimidazole (5%) | +++++ |
| 32 | Choline chloride | Trifluoroacetamide | 1:2 | Tetramethylurea (1%) | +++++ |
| 33 | Choline chloride | Trifluoroacetamide | 1:2 | Tetramethylurea (5%) | +++++ |
| 34 | Choline chloride | Trifluoroacetamide | 1:2 | Ethylene carbonate (33%) | ++ |
| 35 | Choline chloride | Trifluoroacetamide | 1:2 | Imidazole 33%) | ++++ |
| 36 | Choline chloride | Trifluoroacetamide | 1:2 | Lithium acetate (33%) | ++ |
| 37 | Choline chloride | Trifluoroacetamide | 1:2 | 4-Formyl morpholine (33%) | +++++ |
| 38 | Choline chloride | Trifluoroacetamide | 1:2 | Acetonyl acetone (20%) | ++ |
| 39 | Choline chloride | Trifluoroacetamide | 1:2 | Guanidine HCI (3.4%) | ++ |
| 40 | Choline chloride | Acrylamide | 1:2 | - | ++ |
| 41 | Choline chloride | 2-Chloroacetamide | 1:2 | - | ++ |
| 42 | Choline chloride | Bistrifluoroacetami de | 1:2 | - | ++++ |
| 43 | Choline chloride | 2,2-Difluoropropanamid e | 1:2 | - | +++ |
| 44 | Choline chloride | 2,2,2-Trifluorothioacetam ide | 1:2 | - | ++ |
| 45 | Choline chloride | Formamide | 1:2 | - | ++ |
| 46 | Choline chloride | Methanol | 1:2 | - | ++ |
| 47 | Choline chloride | Ethanol | 1:2 | - | ++ |
| 48 | Choline chloride | Trifluoroacetamide | 1:2 | Sorbitol (5%) | ++ |
| 49 | Choline chloride | Trifluoroacetamide | 1:2 | Xylitol (5%) | ++ |
| 50 | Choline chloride | Urea | 1:2 | - | ++ |
| 51 | Choline chloride | Urea | 1:2 | Na cacodylate (10%) | +++ |
| 52 | Choline chloride | Urea | 1:2 | SDS (5%) | ++ |
| 53 | Choline chloride | Urea | 1:2 | Na p-Toluene sulphonic acid (6%) | + |
| 54 | Choline chloride | Urea | 1:2 | Triton TX-45 (12%) | + |
| 55 | Choline chloride | Urea | 1:2 | Na benzoate (8%) | +++ |
| 56 | Choline chloride | Urea | 1:2 | Guanidine isothiocyanate (7%) | + |
| 57 | Choline chloride | Urea | 1:2 | Sulpho salicylic acid (10%) | + |
| 58 | Choline chloride | Urea | 1:2 | CTAB (8%) | ++ |
| 59 | Choline chloride | Urea | 1:2 | Zinc chloride (11%) | +++ |
| 60 | Choline chloride | Urea | 1:2 | Methyl p-toluenesulphonate (25%) | ++ |
| 61 | ZnCl2 | Ethylene glycol | 1:4 | - | + |
| 62 | ZnCl2 | Ethylene glycol:Trifluoroacet amide | 1:3:1 | - | + |
| 63 | ZnCl2 | Urea | 1:3.5 | - | + |
| 64 | ZnCl2 | Trifluoroacetamide | 1:3.5 | - | ++ |
| 65 | Choline chloride | Sorbitol | 1:1 | - | ++++ |
| 66 | Choline chloride | Guanidine isothiocyanate | 2:1 | - | + |
| 67 | Choline chloride | Phenylacetic acid | 1:2 | - | + |
| 68 | Choline chloride | Malonic acid | 1:2 | - | + |
| 69 | Choline chloride | Boric acid | 1:1.5 | - | +++ |
| 70 | Acetylcholine chloride | Urea | 1:2 | - | + |
| 71 | Acetylcholine chloride | Trifluoroacetamide | 1:2 | - | ++ |
| 72 | Choline bromide | Urea | 1:2 | - | + |
| 73 | Choline bromide | Trifluoroacetamide | 1:2 | - | ++++ |
| 74 | Beta-methylcholine chloride | Trifluoroacetamide | 1:2 | - | + |
| 75 | Carnitine | Trifluoroacetamide | 1:2 | - | ++ |
| 76 | Taurine | Trifluoroacetamide | 1:2 | - | + |
| 77 | Methyltriphenylph osphonium bromide | Trifluoroacetamide | 1:3 | - | + |
| 78 | Grignard Reagent T | Trifluoroacetamide | 1:2 | - | +++ |
| 79 | Chloroethyltrimet hyl ammonium chloride | Trifluoroacetamide | 1:2 | - | +++ |
| 80 | Cetyltrimethylam monium chloride | Trifluoroacetamide | 1:2 | - | ++ |
| 81 | Tetramethyl ammonium oxide | Trifluoroacetamide | 1:2 | - | + |
| 82 | Choline chloride | Trichloroacetamide | 1:2 | - | ++ |
| 83 | Benzyltrimethylam monium chloride | Trifluoroacetamide | 1:2 | - | +++ |
| 84 | Betaine | Trifluoroacetamide | 1:2 | - | +++ |

| | | | | | |
|---|---|---|---|---|---|
| Effect on HeLa cell morphology, scale + (worst) to +++++ (best). | | | | | |

An 'additive' is defined here as any substance that can be dissolved in a particular DES mixture, and can be present in amounts greater than, equal to or less than the total amount of the DES mixture (weight:weight). By way of example only, Choline chloride:Urea:Zinc chloride (1:2:0.5 mol:mol:mol), the ZnCl2 is an additive, or Choline chloride: Trifluoracetamide:Urea (1:2:0.1 mol:mol:mol), the Urea is an additive. It should also be noted that the additive does not necessarily have any particular effect on the freezing point of the DES mixture or form hydrogen bonds with any of the DES components but endows the DES mixture with a unique property.

A non-exhaustive list of possible additives to a DES mixture are: Ammonium p-toluenesulphonic acid, Sodium p-toluenesulphonic acid, Ammonium sulphate, Ammonium chloride, Ammonium thiosulphate, Sodium dodecyl sulphate, Sodium lauryl sulphate, Sodium Benzoate, Dodecyldimethyl(3-sulphopropyl)ammonium hydroxide, Dimethylbenzene sulphonic acid, Congo Red, Giemsa, DAPI, Ethidium bromide, Mallory's stain, Orcein, Aldehyde fuchin, Osmium tetroxide, Chromium trioxide, Chromic acid, Feulgen, Dichromate, Mercuric chloride, Haematoxylin and Eosin stain (H&E), Formaldehyde, Glutaraldehyde, Acetone, Ethanol, Methanol, Methyltriphenylphosphonium bromide, Cetyltetrabutylammonium bromide (CTAB), tetrabutylammonium bromide (TBAB), sodium deoxycholate, Brij-35, Brij-58, NP-40, Triton X-100, Triton X-114, Tween-20, Tween-80, Octyl beta glucoside, CHAPS, Solanine, kanomycin, streptomycin or penicillin, sodium nitrate, sodium nitrite or sodium benzoate, ss or ds RNA or DNA sequences, ss or ds RNA or DNA labelled sequences, an aptamer, a peptide, enzyme, antibody, biotin, biotin labelled molecule, horseradish peroxidase, avidin, streptavidin, GFP or variant thereof, Luciferase, Fluorescein, Rhodamine, Texas Red, Alexa Fluor^{™}, LNA, labelled LNA, a Molecular Beacon^{™}, a Scorpion probe^{™}, FISH probe, bDNA, PCR primer, oligo (dT), PNA, anti-oxidant, RNasin, SUPERase•IN^{™}, RNaseOUT^{™}, phenylmethylsulfonyl fluoride (PMSF), diisopropyl fluorophosphate (DFP), aprotinin or Pefabloc SC^{™}, Tris-HCI, PIPES, MES, HEPES, MOPS, MOPSO, CAPS, CAPSO, BIPES, phosphate, imidazole BAPTA, EDTA, EGTA, citric acid, D-Penicillamine, O2, CO2, N2, Argon, propanol, tert-butanol, Trichloroacetic acid, sulphosalicyclic acid, Water, Methanol, Ethanol, Propanol, Butanol, Tetramethyl urea, Imidazole, 1-Methylimidazole, 1-Ethylimidazole, 1-Benzylimidazole, 4-Methylimidazole, N-Methylpyrrolidone, N-Ethylpyrrolidone, N-Benzylpyrrolidone, Guanidine, Guanidine HCI, Guanidine isothiocyanate, Ribitol, Rhamnose, Trehalose, D-Sorbitol, L-Sorbitol, Sorbose, Xylitol, Glucose, Sucrose, Lactose, Fructose, Maltose, Mannose, Mannitol, Arabinose, Galactose, Raffinose, Inositol, Erythritol, Xylose, Zinc acetate, Zinc EDTA, Zinc phosphate, Zinc Trifluoroacetate, Zinc citrate, Zinc PTSA, Zinc gluconate, Zinc chloride and/or Zinc sulphate. Non-dissolvable additives to DES mixtures include but not limited to: Paraffin, Silica gel, Sodium sulphate or Molecular Sieves^{™}, Polyacrylamide, Aerogel, Polyacrylic acid and/or a Quantum Dot.

It will be apparent that there are many types of DES mixture that can be made and the choice of a particular DES for the purpose of preserving RNA in a sample will be determined by one or more of the following preferable features of the DES: (1) compatibility with RNA, preferably it should have a pH between pH 4.5 and 8.5, more preferably between pH 5 and 8, more preferably between pH 5.5 and 7.5 and most preferably between pH 6 and 7 when mixed with the sample, (2) compatibility with the RNA purification reagents and protocol, for example it should not interfere with the binding between the RNA and silica spin column such as RNeasy (RNeasy Mini Kit, Cat. No. 74106, Qiagen, Germany) or alter the partitioning of RNA in phenol containing reagents such as TRIzol (Cat. No. 15596018 Life Technologies, USA), (3) stabilising RNA in the tissue sample sufficiently quickly as to substantially alter the activity of ribonucleases and/or accessibility of RNA to hydrolysis. Alternatively RNA is stabilised in the tissue sufficiently quickly that the RNA Integrity Number (RIN) is not reduced by more than 2 RIN units, more preferably not more than 1 RIN units, even more preferably not more than 0.5 RIN units and most preferably less than 0.1 RIN units during the initial 18-24 hours storage with the DES mixture at room temperature compared with RNA extracted from a fresh tissue sample, without storage, of the same type, (4) have a capacity to stabilise RNA when the tissue represents at least 10%, more preferably 20% and even more preferably at least 50% of the weight of the DES mixture (weight:weight), (5) not reduce RNA yield by more than 20% compared with fresh tissue, (6) provide stabilisation of DNA, proteins and the phosphate groups of phospho-proteins, (7) be chemically stable, non-flammable, non-toxic to the user and the environment, biodegradable, not react with bleach or reagents used during RNA purification to form toxic compounds, (8) have a shelf-life of at least 6 months at room temperature, (9) fix and stabilise native cell morphology and histology including antibody epitopes and sub-cellular organisation and organelles, whilst stabilising RNA and other biomolecules, (10) fix and stabilise cells in such a way as to subsequently allow histological and immunohistochemistry applications for example with antibodies and stains such as Hoechst or H&E stain, (11) stabilise RNA in FFPE samples to protect and enhance the reversal of formalin cross linking at temperatures greater than 50°C and to allow the melting and therefore the easy removal of paraffin from the fixed sample, (12) fix and stabilise circulating tumour cells in whole blood to allow their purification, detection and molecular analysis.

It will be evident to one skilled in the art that various DES can be tested for their suitability by adding them to the sample at a ratio of at least 10:1 (wt:wt). Comparisons of the relative RNA stabilisation can be made using a control solution of RNAlater (10:1 (vol:wt) used according to the manufacturer's instructions (Cat. No. 76106, Qiagen, Germany). Following incubation at, for example 37°C for one day or more, the RNA is extracted according to a standard protocol such as RNeasy (RNeasy Mini Kit, Cat. No. 74106, Qiagen, Germany) and its intactness analysed by gel electrophoresis or by using a a RNA 6000 Nano total RNA Kit (Cat. No. 5067-1511, Agilent Technologies, USA) and Bioanalyser 2100 or other suitable method such as RT-qPCR as described. RNA yields can be determined by OD260nm *uv* spectrometry and purity by the well-known OD260/230 and OD260/280 ratios. A Nanodrop ND2000 (ThermoScientific Inc., USA) is one example of a suitable spectrometer for such determination. Once suitable DES mixtures have been identified their optimum amount, purity, amount of contaminating water and use can be determined by further such tests.

Generally the most important single factor for optimisation is the relative molar ratios of the individual components in the DES mixture. In the first instance mixing only two components of the DES mixture is the most straightforward means to identify approximately, and by empirical means as described above, components that result in the desired effect, such as RNA stabilisation, yield, purity and suitability for downstream applications such as RT-PCR or hybridisation. However it will also be apparent to one skilled in the art that a blend of more than two components may be desirable to further enhance or add novel properties to the DES mixture. There are obviously a very large number of potential mixtures of components that can lead to a DES mixture, and although it is easiest to start with the preparation of, in the first instance simple stoichiometric molar ratios of the DES components such as, by way of example only, a 2:1, 1:1 or 1:2 molar ratio (mol:mol) of choline chloride and urea. It is thought that the depression in freezing point of two component DES mixtures is dictated, at least in part, by the hydrogen bonding between the available hydrogen bond donor (e.g. choline chloride) and acceptor (e.g. urea). However it should be noted that the preferred components of the DES mixture and their optimal ratio of mixing for a particular application such as RNA preservation or cell fixation is not necessarily related to the extent of the observed freezing point depression. Identifying the optimum DES mixture for an application can therefore be determined empirically and consequently trial and error testing may be involved. It will be apparent that identifying the best blend for a particular purpose of a greater than two component DES mixture may involve significant effort. Therefore, in the first instance and in the interest of rapidly defining suitable molar ratios of components, stoichiometric ratios may be used as described above. Once approximate molar ratios have been identified, further refinement involving fixing the molar amount of one component whilst varying the other two or more can be made. It should be noted that the molarity of the various DES components is variable and depends on the density, molar ratios and molecular weight of the individual components. By way of example, the Choline chloride concentration in a Choline chloride:Urea (1:2) mixture is approximately 5M and the Urea 10M, compared with a Choline chloride concentration of 3.6M and a Trifluoroacetamide of 7.2M in a Choline chloride:Trifluoroacetamide (1:2) mixture.

In one aspect of the present disclosure, there is provided a deep eutectic solvent, DES, comprising an additive, wherein the additive is Formic acid. The inclusion of Formic acid may reduce the viscosity of the DES in comparison with a DES not which does not include the Formic acid additive. Reduced viscosity may make the deep eutectic solvent easier to handle. The inclusion of Formic acid as an additive in the DES also helps to maintain cell and tissue volume during cell or tissue fixation. It has also surprisingly been found that, when a biological sample is transferred from such a DES to an aqueous medium, RNA integrity is maintained. Without wishing to be bound by theory, it is believed that Formic acid efficiently and permanently inactivates RNases, thereby protecting the analyte RNA from degradation by RNases, even when the fixed cell is subsequently transferred to an aqueous buffer as is common for many RNA analytical protocols.

The DES may be any of deep eutectic solvents described hereinabove. Preferably, the DES is trimethylglycine : trifluoroacetamide. For example, the DES may be trimethylglycine : trifluoroacetamide at a molar ratio of 1:2 mol:mol.

The concentration of Formic acid in the DES is not particularly limited. Typically, the concentration of Formic acid is in the range 50 mM to 12 M.

For some applications, Formic acid concentrations in the range 4 to 9 M, optionally 6 to 8 M, further optionally 7 to 8 M may be preferred. This is because Formic acid concentrations of about 8 M have been found to be optimal for maintaining RNA stability.

For other applications, Formic acid concentrations in the range 50 to 500 mM, optionally 150 to 400 mM, and further optionally 250 to 350 mM may be preferred. The use of these lower concentrations of Formic acid may be preferable when the biological sample is in contact for more than 6 hours at room-temperature such as during transport and handling and/or when the biomolecule to be analysed is a protein or DNA molecule, both of which are generally more sensitive to acid hydrolysis than RNA. Additionally, these lower Formic acid concentrations can lead to less sample hardening and therefore improve cell lysis and biomolecule purification yield.

In one aspect of the present disclosure, there is provided a deep eutectic solvent, DES, comprising an additive, wherein the additive is Acetic acid. The Acetic acid may be present at a concentration of at least 8 M. It is been found that an Acetic acid concentration of at least 8 M produces similar benefits to those of Formic acid, as detailed above. Acetic acid is preferred for some applications, because it is safer to handle than Formic acid.

The DES may be any of deep eutectic solvents described hereinabove. Preferably, the DES is trimethylglycine : trifluoroacetamide. For example, the DES may be trimethylglycine : trifluoroacetamide at a molar ratio of 1:2 mol:mol.

The concentration of the Acetic acid is at least 8 M, optionally at least 9 M, further optionally at least 11 M. It is believed that there is no upper limit on the useful concentration of Acetic acid, although the concentration of Acetic acid will be less than 17.4 M (i.e., the concentration of pure glacial Acetic acid). For example, the concentration of Acetic acid may be in the range 8 to 17.39 M, 9 to 17.39 M, or 11 to 17.39 M; or 8 to 17 M, 9 to 17 M, or 11 to 17 M.

Any of the various methods and uses described herein may be practiced using a DES comprising a Formic acid additive or an Acetic acid additive.

For example, there is provided a method of fixing a biological sample, which method comprises contacting the biological sample with a DES to form a fixed biological sample, wherein the method further comprises contacting the biological sample with an additive, and wherein the additive is Formic acid or Acetic acid.

The DES may be any of the DESs described herein, for example, trimethylglycine : trifluoroacetamide. The biological sample may be contacted with both the DES and the additive in a single step, in other words contacted with a mixture comprising the DES and the additive.

More preferably, the method comprises a fixation step comprising fixing the biological sample using the DES and then a post-fixation step of contacting the additive with the biological sample. In arrangements where the additive is Acetic acid, the post-fixation step may comprise contacting the biological sample with glacial Acetic acid, or a solution further comprising a solvent selected from a DES, water, an alcohol, and mixtures thereof. The alcohol may be a C1 to C6 alkanol, such as methanol, ethanol, or isopropanol. The DES may be the same as or different to the DES used in the fixing step. Where a solution of Acetic acid is used, the concentration of Acetic acid in the solution is at least 8 M, preferably at least 9 M, more preferably at least 11 M. The concentration of Acetic acid in the solution will be less than the concentration of glacial Acetic acid (approximately 17.4 M) due to the presence of the solvent, but is otherwise not particularly limited.

Optionally, the biological sample is contacted with the DES for a period of 6 hours or less for example 1-15, 15-60 minutes, 1-2 or 2-6 hours, typically the sample is contacted for 15-20 minutes at room temperature. It has been found that limiting the contact time to 6 hours or less helps to maintain nucleic acid integrity.

The biological sample may be any of the various biological samples disclosed hereinabove, e.g. a tissue sample.

The fixed biological sample may be subjected to further process steps. For example, the fixed biological sample may be dissociated into single cells using any of the techniques taught herein. The further process steps may include contacting the fixed biological sample with an aqueous medium.

A fixed biological sample may be dissociated by sonication, for example, sonication using a bath sonicator, as discussed above. The liquid present in the bath may be water, an aqueous salt solution (e.g. a sodium chloride solution, such as a 5-15 wt%, optionally 10 wt%, sodium chloride solution based on the total weight of the solution), an oil such as a vegetable seed oil or an echography gel. Preferably, the liquid is a hydrocolloid, such as a 0.1 - 10% (weight:volume) solution of guar gum, xanthan gum, starch, locust bean gum, gum karaya, gum tragacanth, gum Arabic, cellulose derivatives, alginate, pectin, carrageenan, gelatin, gellan and agar. Using a hydrocolloid in water has the advantage of significantly increasing the transfer of sonication energy from the sonicator to the vial or container containing the Dissociation buffer and the sample to be dissociated. This has the advantage of increasing the rate of the desired tissue dissociation and the production of single cells. An advantage of a water bath sonicator is a wider variety of hard-walled tubes, vessel or containers can be used such as glass which might otherwise shatter and break in a metal block type sonicator as well as simpler temperature control.

Another aspect provides the use of a DES to inhibit degradation of a biomolecule, wherein the DES comprises an additive, wherein the additive is Formic acid.

A still further aspect provides the use of a DES as a Dissociation buffer Dissociation buffer as defined herein, wherein the DES comprises an additive, wherein the additive is Formic acid or Acetic acid.

Although the above aspects have been described with reference to Formic acid, in variants alternative acids may be used instead of, or in addition to, Formic acid. For example, an acid selected from acetic acid, citric acid, or lactic acid may be used as the additive. Further alternative acids which may be suitable for use as the additive include carboxylic acids selected from pyruvic acid, tartaric acid, propionic acid, oxalic acid, maleic acid, salicylic acid, ascorbic acid, benzoic acid or butanoic acid; and mineral acids selected from hydrochloric acid, sulfuric acid, phosphoric acid, nitric acid, and boric acid.

### EXAMPLES

### Example 1

### Dissociation of Mammalian Samples

To 1 ml of Trimethylglycine:Trifluoroacetamide (1:2 mol:mol) was added 5-50 mg (at least one dimension of the sample has a thickness of no more than 4mm) of a freshly dissected mouse, rat or human clinical sample such as liver and incubated for at least 2 hours at room temperature with occasional mixing at approximately 24°C. Following this fixation and stabilization step, the sample can either be used immediately or stored at -80, -20, 4 or 24° C for at least one week prior to recovery of the tissue sample and use as follows.

Excess liquid around the fixed tissue sample is briefly removed using an adsorbent material and then immediately placed in a 300ul thickwall polycarbonate centrifuge tube (Beckman Cat. No. 343775 (7 x 20mm) containing 100-300ul of 95% Trimethylglycine:Trifluoroacetamide (1:2 mol:mol), 15% water by volume, and the tube sealed using a self-adhesive silicone closure. The tube is then placed in a VialPress fitted to a Vial-Tweeter-Sonotrode (S26d11x10), using an Ultrasonic generator (UP200St-G) 200W, 26 kHz, Ultrasonic Transducer (UP200St-T), run at Constant Power, 70% Pulse, 70% Amplitude (100% amplitude = 10um) for 3 minutes at 4°C (Hielscher GmbH Germany).

Alternatively, the 95% Trimethylglycine:Trifluoroacetamide can be replaced with 65, 70, 75, 80, 85, 90, or 100% Trimethylglycine:Trifluoroacetamide. Preferably 85-100% is used with 95% most preferred. The pulse can be varied between 10-100% although it is found that the optimum is be in the range 50-90%, whilst the amplitude can be varied in the range of 20-100% with an optimum in the range 60-100%.

The temperature of the sample should be maintained in the range of -20 to 37°C more preferably -5 to +10°C and most preferably at 4°C to maintain Dissociation buffer viscosity and biomolecule stabilization.

The time typically necessary for sufficient sample dissociation is dependent on a number of factors including the amount and type of sample, the tube size, the pause time, the amplitude, the power, temperature, the Dissociation buffer used, the additive(s) used, the percentage water in the Dissociation buffer, fixation time and the fixative type.

The extent of tissue dissociation can be followed by a variety of means including visual inspection of the opacity of the liquid in the tube and decrease in size of the tissue under white light either by eye or in more detail using a binocular microscope at 20x magnification, by an increase in the optical density (OD600nm), using a surrogate indicator such as a sonication sensitive dye such as Crystal violet or solid matrix such as carbon blue copy paper.

A general method for using a waterbath type sonication device for dissociation is as follows; the fixed biological sample in the range of for example, 1-150mg but preferably less than 50mg, is placed in a Container such as a thickwall polycarbonate centrifuge tube such as a 8 x 51mm (Beckman Coulter^{™} Cat. No. 355657) containing 25-500ul of 75-95% Trimethylglycine:Trifluoroacetamide (1:2 mol:mol) containing 5-25% water by volume and the tube sealed using a self-adhesive silicone closure. The tube is then placed in a floating tube holder and floated on the surface of a waterbath type sonicator such as Elmasonic P30H (37/80 kHz) used at 37kHz, or preferably an Elmasonic Tl-H F MF2 (25/45 kHz) used at 25 kHz, containing water equilibrated at 4-24°C. Waterbath type sonicators should be used with a frequency ≤ 40Kz and most preferably ≤30 kHz. Initially the sonicator should be used at 50% of the total power setting for 1 minute and the extent of tissue dissocation can be be approximately ascertained by observing the opacity of the Dissociation buffer. An increase in opacity is associated with an increase in the number of the desired cells released from the tissue sample. The exact number of cells can be determined using a 10 microlitre portion of the Dissociation buffer and a cell counter such as Countess^{™} II Automated Cell Counter (ThermoFisher Inc. Cat. No. AMQAX1000). If sufficient cells have not been dissociated from the sample, the dissociation can be continued for 2, 3, 4, 5 minutes or by increasing the power setting from 50% to 60, 70, 80, 90 100, or even 120% (if the device is equipped with a pulse function), until sufficient cells have been produced. Care should be taken to maintain the temperature at or below 24°C, preferably below 10°C (ice can be used to maintain the temperature during use). Other waterbath sonicator functions such as 'degas' and 'sweep' can also be tested to determine if tissue dissociation is more efficient.

Single cells collected in this manner are suitable for; scRNAseq, scDNAseq, flow cytometry including FACS and cell staining for example immunofluorescence and DAPI nuclear staining applications.

### Example 2

### Dissociation of Mouse or Rat Tissues

To 1 ml of Trimethylglycine:Trifluoroacetamide (1:2 mol:mol) was added 10-100 mg (at least one dimension of the sample has a thickness of no more than 4 mm) of a freshly dissected mouse or rat tissue such as, brain, salivary glands, tongue, retina, olfactory organ, skeletal muscle, lungs, blood vessels including the aorta, heart, oesophagus, testis, ovaries, spleen, thymus, kidneys, testis, ovaries, lymph glands, skin, stomach, intestine, gall bladder, pancreas, adrenal glands, bone or bone marrow and incubated for at least 2 hours at room temperature (approximately 24°C) with mixing.

Tissue dissociation was carried out by sonication according to Example 1 except that the total time was dependent on the tissue type. As an approximate guide the relative sonication energy necessary to dissociate different tissue types is set out in Table 1.

One of the important variables in the production of single cells from a sample is the type of tissue used, for example tissues which are rich in fibrous connective tissue and structural proteins such as muscle can require additional sonication energy input to completely dissociate the sample. It has been found that striated muscle such as leg muscle dissociates into myofibrils with individual cells difficult to identify in part because muscle cells are multinucleate.

The following table is an approximation of the relative energy typically needed for sample dissociation. 1 (easiest) to 10 (most difficult).

**Table 1.**

| **Tissue Type** | **Relative Energy Typically Required** |
|---|---|
| brain | 5 |
| salivary glands | 4 |
| tongue | 9 |
| retina | 3 |
| olfactory organ | 6 |
| skeletal muscle | 8 |
| lungs | 10 |
| blood vessels | 7 |
| heart | 10 |
| oesophagus | 10 |
| ovaries | 8 |
| spleen | 6 |
| thymus | 5 |
| kidneys | 6 |
| testis | 2 |
| lymph glands | 3 |
| skin | 10 |
| stomach | 8 |
| intestine | 7 |
| gall bladder | 5 |
| pancreas | 5 |
| adrenal glands | 5 |
| bone marrow | 1 |
| bone | 10 |
| teeth | 10 |

### Example 3

### Dissociation of Human Tissues

To 1 ml of Trimethylglycine:Trifluoroacetamide (1:2 mol:mol) was added 10-100 mg (at least one dimension of the sample has a thickness of no more than 4mm) of a freshly dissected human tissue such as, brain, salivary glands, tongue, retina, olfactory organ, skeletal muscle, lungs, blood vessels including the aorta, heart, oesophagus, testis, ovaries, spleen, thymus, kidneys, testis, ovaries, lymph glands, skin, stomach, intestine, gall bladder, pancreas, adrenal glands, bone, bone marrow or a biopsy such as a cancer biopsy, and incubated for at least 2 hours at room temperature (approximately 24°C) with mixing. Dissociation was carried out as set out in Example 1.

### Example 4

### Dissociation of a Cancer Biopsy

To 1 ml of Trimethylglycine:Trifluoroacetamide (1:2 mol:mol) was added 10-100 mg (no dimension of the sample has a thickness of more than 4mm) of a human cancer biopsy and incubated for at least 2 hours at room temperature with mixing.

The cancer biopsy can be as way of example a: (i) Carcinoma including (a) adenocarcinomas and (b) squamous cell carcinoma, (ii) Sarcoma including (a) Osteosarcoma or osteogenic sarcoma, (b) Chondrosarcoma, (b) Leiomyosarcoma (c) Rhabdomyosarcoma (d) Mesothelial sarcoma or mesothelioma, (e) Fibrosarcoma, (f) Angiosarcoma or hemangioendothelioma, (g) Liposarcoma, (h) Glioma or astrocytoma, (i) Myxosarcoma and Mesenchymous or mixed mesodermal tumor; (iii) Leukemias, (iv) Lymphomas including (a) Hodgkin Lymphoma and, (b) Non-Hodgkin Lymphoma.

Dissociation was carried out by sonication as set out in Example 1. Following dissociation the single cells were examined by microscopy.

### Example 5 (a reference example)

### Sample collection of Analytes from Human Skin

100 µl of Trimethylglycine:Glycerol (1:2 mol:mol) containing 10% water was spotted onto the lower forearm of a patient and gently rubbed into the skin for 1 minute and then a piezoelectric disc such as a 15 mm 30 KHz transducer (Cat. No. SMUN15K12F30 Steminc, USA) or 35 mm Piezo ceramic disc 56kHz (Cat. No. SMD35T4S311, Steminc, USA) was placed onto the prepared skin zone, maintained in place with an adhesive strip, the disc attached to a suitable electric generator and sonication carried out for 5 minutes with constant gentle pressure applied to the disc, after which the disc was removed and an adsorbent disc was immediately placed on the sonicated skin zone and held, with firm pressure for 1 minute to recuperate the liquid containing the DES composition and any skin cells, bacteria, viruses, particles, blood, plasma, nucleic acids, fats, proteins, phosphoproteins, and/or metabolites that have been extracted from the skin surface. After removal from the skin, to the adsorbent disc bearing the sample was added 150ul of Trimethylglycine:Glycerol (1:2 mol:mol), saline solution or water and then the disc rolled and placed vertically in a 1.5ml polypropylene centrifuge tube and centrifuged for 60 seconds at 12,000g and the recovered liquid used as a source of analyte material for a test such as a glucose test, a therapeutic or doping drug test, a viral or bacterial diagnostic test, a genetic test, an allergy test, a test for the presence of a specific patient antibody, a blood chemistry test such as for cholesterol or haemoglobin, a cancer test for example for CEA or PSA antigens, or as a source of DNA such as genomic DNA, circulating free DNA or circulating foetal DNA for a genetic test. The type of analytical test is not particularly limited to a chemistry, haematology, microbiology or a molecular based analytical test.

Alternatively the analytical test is carried out within the adsorbent disc that has been in contact with the patients skin, for example specific antibodies are added to adsorbent disc for a lateral flow test or other assay to detect a specific antigen.

### Example 6

### Dissociation of Varying Tissue Sizes

The energy transferred from the sonication device to the container or vessel containing the sample is proportional to the mass of the sample. It has been found that larger tissue pieces may require a larger energy input to achieve sufficient dissociation. However, when tissue pieces smaller than 10mg are placed in the cavitation zone of the Dissociation buffer they tend to be pushed by the cavitation bubbles to parts of the Dissociation buffer where there is less cavitation thereby reducing the rate of sample dissociation. To avoid this issue with smaller tissue pieces, a smaller diameter tube is preferred thereby physically constraining the sample to the cavitation zone. Alternatively, the sample can be attached or restricted to the focal area of the cavitation zone using a tethering structure within the tube. This can be a vertical needle attached to the tube lid and descending into the dissociation liquid with the tissue sample firmly skewered on it thereby limiting its movement. Another alternative is to place the tissue sample in a plastic cage or mesh within the container so that the Dissociation buffer and cavitation energy can freely contact the sample but the sample is held firmly within a defined cavitation zone of the tube.

Large pieces of tissue of up to 10 g can be dissociated by sonication but this has been found to be most easily accomplished in a continual manner from one edge of the tissue that is presented towards the cavitation zone, and as the tissue is dissociated and therefore reduced in size, the tissue is progressively 'fed' into the cavitation zone in a successive manner until the entire sample is dissociated. It will be evident to one skilled in the art that more sonication power is typically important for larger samples and this may also require longer Dissociation buffer cooling periods during the process.

It has been found that tissue samples of very variable starting weight can be dissociated, as way of example only; 1 to 100 mg, 100 to 1000 mg or 1 g to 10 g.

### Example 7

### Dissociation of Tissue in Different Dissociation Buffers

Many different types of Dissociation buffers can be used with the sonication device, preferably these buffers have the following characteristics (i) water content less than 50%, more preferably less than 40% and even more preferably less than 30%, and most preferably less than 15%, (ii) a viscosity of at least 5 centipoise (cP), more preferably 10 cP, even more preferably 25 cP, and most preferably between 50-125 cP, and (iv) protection and stabilization of biomolecules such as RNA, DNA, proteins and phsophoproteins, (v) softening of the sample to improve dissociation, (vi) good transmission of sonication power to the sample, (vii) high cavitation bubble formation to improve the rate of dissociation, (viii) protection of single cells from breaking down into sub-cellular components and debris, and, (ix) high coefficient of heat energy transfer from the container to the surrounding environment. Dissociation buffers composed of DESs have been found to be particularly effective for this purpose.

Specific examples include 65-100% Trimethylglycine:Trifluoroacetamide (1:1.5 mol:mol, 1:2 mol:mol or 1:3 mol:mol), 65-100% Trimethylglycine:Glycerol (1:2 mol:mol), 65-100% Choline chloride:Urea (1:2 mol:mol), 65-100% Trimethylglycine:Urea (1:2 mol:mol). As set out herein, the use of additives to the DES can provide improved dissociation properties such as by the addition of a detergent.

Although protecting and stabilizing biomolecules such as RNA less well than DESs, glycols may serve as useful Dissociation buffers, for example Ethylene glycol, Glycerol, Diethylene glycol, Triethylene glycol, PEG 200, PEG 300, PEG 400, PEG 600 and PEGs with a MW in excess of 800 with 0-60% water to provide a room temperature liquid with a viscosity of at least 5 cP.

### Example 8

### Softening Tissue Using Glycols Prior to Dissociation

It has been found that fixed tissues that are difficult to dissociate can be rendered softened and therefore facilitate the dissociation by sonication into single cells by soaking the sample in at least 20 volumes of a 20-100% solution of a glycol for 1 or more hours at room-temperature. The following room temperature liquid glycols with a molecular weight of less than 1000 Daltons have been found to be effective for tissue softening; ethylene glycol, glycerol, diethylene glycol, triethylene glycol, PEG 200, PEG 300, PEG 400 or PEG 600.

A 20 mg sample of mouse liver was treated for 1 week with 100% Trimethylglycine:Trifluoroacetamide (1:2 mol:mol), in order to fix and stabilize the RNA, DNA and protein content, the fixed sample was then removed, excess fixative removed and then placed in 500 µl of glycerol (TCI France) and softened overnight at room-temperature. The sample was then removed and added to 300 µl of 95% Trimethylglycine:Trifluoroacetamide (1:2 mol:mol) in a 1 ml (8 × 51mm) thickwall Polycarbonate centrifuge tube (Beckman Cat. No. 355657), the tube was then placed in the VialPress attached to a VialTweeter as described in Example 1 and run at 70% Pulse with 70% amplitude for 3 minutes at 4°C.

An alternative method is to carry out the dissociation step in the presence of a softener. A 20 mg sample of mouse liver was treated for 1 week with 100% Trimethylglycine:Trifluoroacetamide (1:2 mol:mol) in order to fix and stabilize the RNA, DNA and protein content, the fixed sample was then removed, excess fixative removed and then placed in 300 µl of 60% Trimethylglycine:Trifluoroacetamide (1:2 mol:mol), 25% PEG200 (TCI France) and 15% water in a 1 ml (8 x 51mm) thickwall Polycarbonate centrifuge tube (Cat. No. 355657 Beckman, USA), the tube was then placed in the VialPress attached to a VialTweeter as described in Example 1 and run at 70% Pulse with 70% amplitude for 3 minutes at 4°C.

### Example 9

### Different Types of Tubes / Dissociation Containers

A 20mg sample of mouse liver was treated for 6 hours with 100% Trimethylglycine:Trifluoroacetamide (1:2 mol:mol) in order to fix and stabilize the RNA, DNA and protein content. The mouse liver sample was then removed and added to 300 µl of 95% Trimethylglycine:Trifluoroacetamide (1:2 mol:mol) in a 1ml (8 × 51mm) thickwall Polycarbonate centrifuge tube (Beckman Cat. No. 355657), the tube was then placed in the VialPress attached to a VialTweeter as described in Example 1 and run at 70% Pulse with 70% amplitude for 3 minutes at 4°C.

A comparison was made by replacing the Polycarbonate tube with a 1.5 ml 'Eppendorf' Polypropylene tube and running the sonication under identical conditions. It was found that the extent of tissue dissociation and therefore the number of cells released, (which is a measure of the efficiency of energy transfer) was approximately 30% less with a Polypropylene tube compared with a Polycarbonate tube. It was also found that on extended sonication the Polypropylene tube tended to become softer and even deform and melt. It was further found that thick walled Polycarbonate tubes were particularly well suited to transferring energy to the sample.

It was found that when the tube is in direct contact with the metal block of the VialTweeter, and sonication energy applied, that glass, even thick walled glass designed for laboratory use which has excellent sonication energy transmitting properties nevertheless shattered and failed regularly, similarly polystyrene 8ml tubes (Cat. No. 352027, Corning Inc, USA) tended to form stress and fracture lines in the walls which eventually led to leakage. Therefore polycarbonate was found to be an excellent compromise between the energy transmitting properties of glass and the strength and low cost of a polymer, with the additional benefit that the sample can be easily observed through the transparent walls during the dissociation procedure.

### Example 10

### Varying the Temperature of the Dissociation Buffer

The temperature of the dissociation process is of great importance, firstly the viscosity of the Dissociation buffer is highly dependent on the temperature. In the case of 100% Trimethylglycine:Trifluoroacetamide (1:2 mol:mol) or aqueous dilutions of, viscosity is greatly reduced as the temperature increases. As the viscosity has a marked effect on cavitation by altering the growth and collapse of the cavitation bubble, heating the Dissociation buffer increases the bubble size formed and the dissociation effect. However, increasing the temperature also negatively effects the RNA, DNA, protein and phosphoprotein quality therefore it is preferable to keep the temperature during sonication below 24°C, more preferably below 12°C and even more preferably at about 4°C or less. The majority of the sonication energy is converted into heat at the site of cavitation, or as friction between the tube holding the sample and the metal VialTweeter block (Hielscher, Germany).

Maintaining steady temperature is most easily accomplished by pre-chilling equipment such as the metal components, block, water bath, sample and tubes at 4°C, and if possible running the dissociation experiment in a 4°C 'cold-room' so excess container heat is rapidly dissipated. Also by running the sonicator in 'pulse mode' such as 20, 40, 60 or 80% (20% pulse means 20/100^{th} of a second on, 80/100^{th} of a second off for each cycle), rather than at 100% leads to less heat generation per minute. It will be evident to one skilled in the art that there are various well known methods to remove heat from an object such as by using heat sinks, blowing cool air, water cooling for example with a water jacket, or by Peltier cooling. Preferably the Dissociation buffer and sonication of the sample is carried out at 4°C. Control of the temperature is one means to control viscosity and therefore the rate of dissociation.

### Example 11

### Varying the Water Content and/or Pressure of the Dissociation Buffer

The final water content in the Dissociation buffer is one factor which may influence tissue dissociation, the number of single cells to debris, sample heating, cavitation, and the rate of decomposition of the analytes such as RNA.

Cavitation is dependent on both the amount of dissolved gases but also the viscosity of the Dissociation buffer. It has been found that it in order to maintain RNA quality, final water concentration in the DES should not exceed 35%, more preferably it should be no more than 30%, even more preferably no more than 25%, even more preferably no more than 20%, even more preferably no more than 10% and most preferably no more than 5% final volume. Viscous solvents such as pure glycerol have been found to be ineffective at promoting cavitation without added water present, preferably in the order of 15% final volume.

It has been found that when water is present at 5-10% final volume in the Dissociation buffer that there is a tendency for larger portions of the tissue sample to dissociated leading to larger clumps of cells than when a higher proportion of water, for example 25%. Increasing the water content to 50% or more leads to a massive increase in the formation of cell debris at the cost of a reduction in the number of single cells. Therefore it will be evident to one skilled in the art that it is possible to fine tune both the rate of tissue dissociation but also the number of single-cells produced during dissociation.

Pressure can be easily altered using a syringe attached to the Dissociation container, increasing the pressure to 4 atmospheres markedly reduces the amount and size of cavitation bubbles and the amount of dissociation whilst reducing the pressure to 0.8 atmospheres increases the number and size of cavitation bubbles. It will be evident to one skilled in the art that it is possible to vary the pressure in the dissociation container to regulate the degree of sample dissociation either between different samples runs or during a single dissociation experiment.

### Example 12

### Varying the Viscosity of the Dissociation Buffer

The viscosity of the Dissociation buffer can be altered in a number of different ways; (i) the solvent type, (ii) percentage of water as described in Example 11 above, (iii) temperature, the latter being particularly marked for solvents with strong hydrogen bonding such as glycerol, ethylene glycol, water and DESs. It has been found that the temperature of the reaction container should preferably be kept at room temperature or more preferably at 4-12°C both to reduce RNA degradation but also to reduce viscosity. When the viscosity decreases, cavitation increases and may lead to a disequilibrium of the cell debris:single cell product ratio. The viscosity of the Dissociation buffer can either be determined empirically, or for pure solvents, by consulting published online sources such as Pubchem (NIH). It will be understood by one skilled in the art that by careful selection of solvents with a desired viscosity at a set temperature that the extent of dissociation and the type of products can be attained.

### Example 13

### Varying the Pulse Time and Amplitude of the Sonication Device

Both the pulse time and the amplitude are important factors for the control of the tissue dissociation. This has an important effect on dissociation as the tissue sample is more effectively dissociated when the pulse time is between 65 to 95% rather than 100%. The pulse of cavitation energy causes the tissue to dissociate faster than with a continual energy input which was unexpected. The amplitude is the distance traveled in micrometres by the sonitrode and therefore also the metal block or device holding the container with the sample in it. As one example, the amplitude of the VialTweeter is 10 µm at 100%. By reducing the amplitude there is a corresponding decrease in the amount of energy applied to the sample. It will be evident to one skilled in the art that it is possible to vary both the pulse time and the amplitude in order to obtain the amount of dissociation appropriate for the sample being dissociated. It has been found that a suitable pulse time is 70% and amplitude 70%.

### Example 14

### Enzymatic Pre-treatment Prior to Tissue Dissociation

Protease digestion of tissues to dissociate them into single-cells is well known. Most of the published studies have been carried out with fresh tissues with the goal of using the single cells for tissue culture. There are dozens of proteases that have been used for tissue dissociation which are either general proteases or proteases specific towards proteins such as collagen. By way of reference some of the more widely used proteases are; (i) Liberases (Roche, UK), (ii) Collagenase Type I, II, III or IV, (iii) Trypsin, (iv) Papain, (v) Proteinase K, (vi) Chymotrypsin, (vii) Elastase, (viii) Dispase and/or (ix) Pronase (Sigma-Aldrich, UK). Other types of enzymes can also be used such as Hyaluronidase, DNase and Lipases to facilitate dissociation and reduce sample viscosity.

One of the advantages of using proteases is that they can separate cells from connective tissue, however there are many disadvantages to using proteases including the removal of cell surface epitopes that would otherwise be used as markers of cell identity (Garg A, et al., Cytotherapy. (2014);16:545-559), or on prolonged incubation the cell integrity can be lost. A particularly negative consequence of using proteases is that they generally require incubation at 37°C or more for prolonged periods (e.g. one to several hours) in non-physiological conditions that inevitably lead to profound changes to the transcriptome and other analytes which can be radically altered from their original *in vivo* levels during enzymatic treatment.

Additionally cells released from fresh tissues treated with proteases fail to maintain the morphology that is characteristic of their original *in vivo* shape so this information is lost during the procedure despite have great value for cellular identification. The advantage of tissue fixation prior to tissue dissociation to overcome these issues is therefore profound, however few fixatives are capable of maintaining RNA integrity during tissue dissociation.

To 1 ml of Trimethylglycine:Trifluoroacetamide (1:2 mol:mol) was added 50 mg of a freshly dissected mouse liver sample and incubated for at least 2 hours at room temperature (approximately 24°C). The sample was removed and any excess fixation solution blotted away on a paper towel, the sample was then placed in a thickwall polycarbonate tube (Cat. No. 343775, Beckman USA) containing 200 ul of a 65-100% DES, preferably at least 85% DES, Trimethylglycine:Trifluoroacetamide (1:2 mol:mol) solution containing 0.5-5mg Pronase enzyme powder (Cat. No. 10165921001, Roche, UK) and incubated for 20 min to 4 hours at room temperature with constant gentle mixing. The tube was then transferred to the sonication device as set out in Example 1 and dissociation carried out.

Alternatively, Proteinase K (Cat. No. 70663-4) Trypsin (Cat. No. T4799), Collagenase (Cat. No. C0130) Liberase, Collagenase (Type I, II, III or IV), Papain (Cat. No. 76218), Chymotrypsin (Cat. No. 1.02307), Elastase (Cat. No. E7885), and/or Dispase (Cat. No. D4818), powder (0.05-5mg Sigma-Aldrich, UK) was used to digest the sample instead of Pronase.

Alternatively the enzymatic digestion solution can be, but is not limited to 65-100% Choline chloride:Urea (1:2 mol:mol), Trimethylglycine:Urea (1:2 mol:mol) and/or Trimethylglycine:Glycerol (1:2 mol:mol).

### Example 15

### Dissociation of Tissue Using Indirect Sonication

To 1 ml of Trimethylglycine:Trifluoroacetamide (1:2 mol:mol) was added 50 mg of a freshly dissected mouse or rat liver sample and incubated for at least 2 hours at room temperature.

Excess liquid around the fixed tissue sample was briefly removed using an adsorbent material and then immediately placed in a standard polypropylene 1.5ml Eppendorf centrifuge tube containing 200 µl of 85-100% Trimethylglycine:Trifluoroacetamide (1:2 mol:mol), 15% water, the tube was then transferred to a Banderex 1.5L 35 kHz sonicator water bath and run constantly for 3-30 min depending on the tissue type at 4 - 24°C (temperature can be adjusted by adding ice to the bath). Indirect sonication was stopped once sufficient cell dissociation was obtained as determined visually or by cell counting.

Alternative indirect sonication water baths include the Elmasonic P series Dual frequency (37/80 kHz 2.75L Cat. No. P30H Elma Schmidbauer GmbH) or multi-frequency ultrasonic cleaning unit TI-H-5 MF-2 25/45 kHz, or MF-3 35/130 kHz .

Instead of tap water in the water bath, a solution of 10% NaCl, vegetable seed oil or echography gel (eg. Aquasonic Clear Ultrasound gel, Parker Inc Cat. No. 03-50) can be used.

Alternatively a hydrocolloid such as 0.1 - 10% (weight:volume) solution of guar gum, xanthan gum, starch, locust bean gum, gum karaya, gum tragacanth, gum arabic and cellulose derivatives, alginate, pectin, carrageenan, gelatin, gellan and agar can be dispersed in water and used as a replacement for tap or distilled water in the water bath sonicator, for example the Elma Ultrasonics Model P30H. Using a hydrocolloid in water has the advantage of significantly increasing the transfer of sonication energy from the sonicator to the vial or container containing the Dissociation buffer and the sample to be dissociated. This has the advantage of increasing the rate of the desired tissue dissociation and the production of single cells. An advantage of a waterbath sonicator is a wider variety of hard-walled tubes, vessel or containers can be used such as glass which might otherwise shatter and break in a metal type block sonicator as well as simpler temperature control and on cost grounds.

Preferably the tube or container has an insert or plunger which has an adjustable height within the container, allowing it to be placed in contact with the Dissociation buffer and thereby limiting the movement, gas exchange and evaporation of Dissociation buffer within the tube, vessel or container. The insert or plunger may contain a means to cool the sample, such as a metallic heat transfer element to equilibrate the Dissociation buffer temperature, or a filter allowing the removal of the Dissociation buffer and sample from the tube, vessel or container. The insert or plunger may also have an abrasive surface to improve sample dissociation which is particularly important for robust samples such as the lung, muscle and eye cornea. The insert or plunger also provides a means to either increase or decrease the ambient pressure above the Dissociation buffer thereby controlling the force of cavitation as set out in Example 11.

It has been found that a better transmission of the ultrasound energy to the sample tube can be obtained using these alternative liquids and gels. Tap water can also be degassed by boiling for 15 minutes and then cooling before use, or alternatively, it can be degassed under vacuum or using the 'degassing' function of certain water baths such as the Elmasonic P series. Degassing improves sonication power transmission to the sample container.

When using certain water bath sonicators, care should be taken to position the tube containing the sample in the correct part of the bath as indirect sonication water baths can have a very unequal distribution. Sonicators with a sweep function such as the Elmasonic P series (Elma Schmidbauer GmbH) are preferred because there is not one focal point of high energy rather the energy is distributed throughout the bath.

It is also advantageous to have more than one ultrasound frequency as is available with Elmasonic P series (37 and 80 kHz), generally the low frequency is capable of removing larger blocks of cells from the tissue mass whilst the higher frequency removes cell debris and cleans the cells of broken debris. Other devices with multifrequency output is TI-H5 (Elma Schmidbauer GmbH) operating at either 25 and 45 kHz (Model MF2), or alternatively 35 and 130 kHz (Model MF3). Using more than one frequency provides additional control of the dissociation process.

### Example 16

### Dissociation of Tissue Using Direct Sonication

To 1 ml of Trimethylglycine:Trifluoroacetamide (1:2 mol:mol) was added 50 mg of a freshly dissected mouse or rat liver sample and incubated for at least 2 hours at room temperature (approximately 24°C).

Excess liquid around the fixed tissue sample was briefly removed using an adsorbent material and then immediately placed in a standard polypropylene 1.5 ml Eppendorf centrifuge tube containing 400 µl of 85-100% Trimethylglycine:Trifluoroacetamide (1:2 mol:mol), 15% water, was placed in an ice bath and a sonication probe (3mm tip diameter, 180 µm amplitude) Model 120 sonic 20 kHz dismembrator (Cat. No. FB4422, Fisher Scientific, UK) was then placed in direct contact with the tube contents and run constantly for 3-30min depending on the tissue type. Direct sonication was stopped once sufficient cell dissociation has occurred as determined optically. It was found that dissociation was rapidly achieved using direct sonication but both overheating and contamination of the sample with the probe were significant drawbacks.

### Example 17

### Device for Holding the Tissue Sample During Dissociation

It has been observed that the sonication energy in the Dissociation buffer causes the tissue sample to be displaced to a part of the tube with a lower ambient energy and therefore less dissociation results. In order to avoid this displacement, the tissue sample can be physically constrained within the energy field. It was found that a number of different constraints for the sample were suitable such as impaling the sample on a hook or screw like metal wire and suspended in the Dissociation buffer at the focal point of the energy. Alternatively a cassette can also be used such as those used for paraffin embedding tissue PrintMate^{™} Biopsy Cassettes (Cat.No. A84210111, ThermoFisher Scientific), Micromesh^{™} (Cat. No. M507-2, Simport, USA) conveniently allows the sample to be dissociated and smaller clumps of cells pass easily through the mesh (0.38mm mesh size) but not the sample which is held within the cassette. It will be evident to one skilled in the art that various commercially available mesh sizes can be used to enclose the sample so that using a mesh size for example of 8 µm will only allow cells, for example erythrocytes and immune cells, of less than that diameter to pass through and be collected whilst larger mesh sizes for example 50 µm will allow the majority of mammalian single-cells to pass through and be collected whilst maintaining the tissue sample within the cassette. A variety of woven plastic mesh sizes (200, 100, 70, 50, 35, 25, 20, 15, 10, 8 and 5 µm were found to be particularly useful (Cat. No. N50S, BioDesign Inc, USA) at removing large clumps of cells and other large debris from the desired single-cells which pass through the filter and can be collected.

### Example 18 (a reference example)

### Device for Continual Flow

The device consists of a square or round container with an opening for inserting the tissue sample and addition of a Dissociation buffer, and in addition an inlet and an outlet both of which are connected to the interior of the container and the Dissociation buffer. The Dissociation buffer can thus be circulated out of the container where the single cells from the dissociation process collected for analysis and an inlet feeding fresh Dissociation buffer back into the container in a continual process. The overall interior volume of the container is about 100 - 500 µl. The container, preferably constructed of polycarbonate is shaped such as to fit into, and make good contact with a metal block attached to a sonotrode, or alternatively, a sonication bath. Preferably the contents of the container are visible to the operator such that the dissociation process can be followed visually, either by the naked eye or using a stereomicroscope (Stereo Discovery.V20 or Smartzoom 5, Carl Zeiss, Germany). The liquid is moved by way of a small pump (e.g. Model 9QX Peristaltic Pump, Boxer GmbH) and tubing (e.g. 9000.714 diameter 1.0 mm, Boxer GmbH, Germany) that can be controlled in its rate of flow. Single cells can be removed from the circulating Dissociation buffer by filtration, centrifugation, flow cytometry, Trimethylglycine:Trifluoroacetamide (1:2 mol:mol) CS etc for subsequent staining with a stain such as Crystal Violet, Methylene Blue, Trypan Blue, antibody or nucleic acid hybridization probe, extraction of proteins, phosphoproteins, DNA or RNA, microscopical observation and digital analysis.

A variety of woven plastic mesh sizes (200, 100, 70, 50, 35, 25, 20, 15, 10, 8 and 5 µm such as Cat. No. N50S have been found to be particularly useful (BioDesign Inc, USA).

### Example 19 (a reference example)

### Device for Tissue Dissociation and Automatic Separation of Single Cells and Display Results on Monitor

It is convenient for the dissociation and analysis of multiple samples such as in a clinical setting to reduce the number of manual steps necessary to complete the diagnosis. Ideally the sample, for example a biopsy, such as a prostate or breast cancer biopsy, is removed from the patient and immediately placed in 20 volumes of Trimethylglycine:Trifluoroacetamide (1:2 mol:mol) and the sample dissociated as set out in Example 1 for sufficient time to dissociate the entirety of the biopsy. The cells are then removed from the outlet and separated from the Dissociation buffer by filtration and tissue debris removed by way of filtration or density gradient centrifugation. The filtered cells are transferred by means of a pump into a separate container where, in suspension, they are stained with a colourant, a stain, a primary and secondary labelled antibody and/or nucleic probe to aid in their identification. Alternatively the cells are transferred and attached onto a glass microscope slide where they can be stained with for example the nuclear stain DAPI or an antibody and observed by means of a fluorescent microscope. The presence of abnormal cells as judged by their morphology, their staining pattern or both associated with the disease phenotype can then be analysed by a pathologist, preferably with the aid of an appropriate software such as HALO developed by Indica Labs, UK that can determine the shape, size or abnormal staining pattern.

Once the tissue sample has been dissociated into single cells, it is then possible to use the single cells for a number of and diverse number of applications. There are a number of ways to select single cells including using antibodies either as fluorescent markers in the FACS procedure, or using antibodies bound to a solid phase such as a bead a fiber or magnetic beads including MACS (Miltenyi GmbH, Germany). It will be evident to one skilled in the art that there are a great number of protocols and products available for binding to single cells.

The analytical procedure can depend on counting the relative and absolute number of cells of various types such as T cells and B cells CD4 positive and CD8 positive cells. This in itself can be the diagnostic purpose of the experiment or diagnostic essay.

Following selection the single cells can be used for a number of molecular tests, for example RNAseq with the RNA transcript of each individual cells is determined. Several commercialized RNA-seq platforms exist including 10X Chromium, Fluidigm C1, BioRad ddSEQ and Dolomite RNA-seq system.

Alternatively the single cells can be used for proteome analysis by extracting protein. As one example, this could be for studying the phosphoprotein component of the cell. Conveniently it is been found that Trimethylglycine:Trifluoroacetamide (1:2 mol:mol) fixation and 85-100% Trimethylglycine:Trifluoroacetamide (1:2 mol:mol) tissue Dissociation buffer protects the phosphate group on the protein including Serine, Threonine and Tyrosine phosphate proteins. The single cells can also be used for genomics sequencing to ascertain the sequence of a genome.

### Example 20

### Dissociation of Tissue, Separation and Identification of Single Cells Based on Morphology

To a 100 mg of mouse large intestine sample was added 1 mL of Trimethylglycine:Trifluoroacetamide (1:2 mol:mol) and incubated for 12 hours at ambient temperature to fix the sample. The tissue sample was then removed and placed in 500 µL of 85-100% Trimethylglycine:Trifluoroacetamide (1:2 mol:mol) in a 1 mL polycarbonate tube. Sonication was carried out for 15 seconds as set out in Example 1, 500 µL of the dissociation buffet was then removed and a fresh 500 µL of 85-100% Trimethylglycine:Trifluoroacetamide (1:2 mol:mol) was added and sonication carried out again, for 60 seconds. This process was repeated with a sonication time of 60 seconds per cycle using using 70% pulse and 70% amplitude. The Dissociation buffer was passed through an 8 µm CellSieve mesh filter to remove debris and to capture the single cells. The single-cells were washed off the filter and resuspended in water before FACS analysis and sorting based on cell size or shape. On recovery and examination of the filtered cells using a microscope it was found that the initial dissociation cycles led initially to the dissociation of epithelial cells followed by the intestinal luminal cells and finally muscle cells and connective tissue.

### Example 21

### Staining the Tissue Sample Prior to, During, or After Dissociation

It is possible to carryout tissue fixation and staining in a variety of ways: (i) staining followed by fixation, (ii) fixation followed by staining, or (iii) staining and fixation at the same time. It is also possible to combine staining, fixation and dissociation at the same time by using the Dissociation buffer containing a stain as the sample fixative solution. A fresh tissue sample or a fresh sample that has been snap-frozen was placed placed in a 1 ml thick-wall polycarbonate centrifuge tube (Beckman Cat. No. 355657) containing 200 µl of 85-100% Trimethylglycine:Trifluoroacetamide (1:2 mol:mol) and 0.02% Methylene blue, and the tube sealed using a self-adhesive silicone closure. The tube is then placed in a VialPress fitted to a Vial-Tweeter-Sonotrode (S26d11x10), using an Ultrasonic generator (UP200St-G) 200 W, 26 kHz, Ultrasonic Transducer (UP200St-T, Hielscher GmbH, Germany), run at Constant Power, 10% Pulse, 10% Amplitude for 60 minutes at 4°C. It was found that single-cells were both well fixed, intact and stained with Methylene blue.

Preferably the dissociation and fixation solution is at least 95% Trimethylglycine:Trifluoroacetamide (1:2 mol:mol) and the sonication power is such that the unfixed cells break open and lyse before they are fixed. It is therefore preferred that both the Pulse and Amplitude are limited to a maximum of 20% to favourise initial tissue fixation followed by dissociation. It has been found that gentle sonication in this manner can lead to faster fixation than in its absence, therefore the sonication serves the role of speeding up the fixation as well as causing the tissue to dissociate into single cells.

It is also possible to include a stain during this process so that tissue fixation, staining and dissociation all occur at the same time. The stain can be a colourant, an antibody or a nucleic acid probe.

### Example 22

### Dissociation of Tissue into Separated Groups or Clumps of Cells for Subsequent Dissociation into Single Cells

Partial dissociation of the tissue sample leads to large collections of cells together. The cells making up the clump of cells are by definition, cells that were spatially located in the original tissue sample together. By separating these collections of cells into individual containers by, for example microfluidic means, filtering, density gradient centrifugation, manual sorting or by use of a colourant, a stain such as an antibody or fluorescent probe, and then their subsequent dissociation into single cells allows the analysis of cells that were spatially localized together in the original sample this allows a comparison of, for example, gene expression patterns of neighbouring cells. It will also be apparent that this approach is applicable not only for large collections of cells (greater than a million) but also for very small numbers, for example groups of 10 or less, five or less, or even two or less cells joined together. Such a progressive method of tissue dissociation provides a means to understand the spatial structure of the original biopsy and therefore cell to cell variation of, as one example, mRNA expression patterns.

### Example 23

### Dissociation of Fixed Tissue into Single Nuclei Using DMSO

Traditional methods for purifying nuclei require long and arduous protocols involving multiple centrifugation steps and detergents (Kizer, K., Mds (2006) 40:296-302). It has surprisingly been found that the addition of the polar aprotic solvent Dimethylsulphoxide (DMSO) at 25% or more final concentration to a DES fixed sample is particularly efficient at the solubilization and removal of the cell membrane and cytoplasm but not the nucleus, which remains intact. Such intact nuclei can be sorted, pelleted selected, and their contents analysed either in entire nuclei by using, for example, labeled antibodies specific to nuclear epitopes such as Ki-67 or, by using fluorescent nucleic acid probes for detecting RNA and DNA. Alternatively the purified nuclei can be lysed and their contents including proteins, DNA and RNA released for further analysis such as genomic DNA sequencing, protein-protein interactions, intron splicing, CHIP analysis, chromosome analysis and/or RNA sequencing. As an example, single nuclei have been successfully used for RNA-seq (Habib, N. et al., Nat. Methods (2017) 10:955-958) and with the 10X Chromium sequencing platform (10X Genomics, USA).

DMSO at 25% or more can be added to a sample at one or more steps; (i) after the sample has been fixed with a DES; the sample, for example, tissue culture cells or a tissue is treated with DMSO, and optionally, vortexing, pipetting, or using other mechanical means included to improve cytoplasmic solubilisation, or (ii) DMSO is added to the Dissociation buffer during sonication, or (iii) after tissue dissociation is complete the single cell suspension is treated with DMSO to produce a pure population of nuclei.

The ability of DMSO (and, to a lesser extent, diethylsulfoxide; ethyl methyl sulfoxide may show similar behavior) to dissolve a DES fixed cell but not the nuclei is unexpected and unusual; no other solvents, alcohols, organic solvents or hydrocarbons have been identified that have this property. The differential solubility of cytoplasm and nuclei in DMSO is particularly unexpected given that DMSO treatment of living cells is well known as a cryoprotectant to maintain cell viability during freezing.

As an alternative to purifying nuclei, DMSO can also be usefully employed to solubilize the cell cytoplasm in order to efficiently release cytoplasmic biomolecules, for example, cytoplasmic RNA such as mRNA, or proteins and phosphoproteins, without contaminating nuclear protein, RNA and/or genomic DNA (gDNA) which is a serious problem in many purification protocols. As one example, purified RNA from cells and tissues are frequently treated with DNase or another DNA removal process to remove the omnipresent contaminating co-purifying gDNA prior to RT-PCR. In this example therefore, following DMSO treatment of the DES fixed sample, the nuclei can either be filtered out using, for example a 0.25um filter, or pelleted away from the desired solubilized cytoplasmic fraction of the cell to leave a substantially DNA free sample.

As another example, cells and tissues fixed with DES, particularly Trimethylglycine:Trifluoroacetamide (1:2 mol:mol) fixed samples can be resistant to efficient solubilisation with detergents that are used standardly with fresh samples leading to low yields particularly of proteins. By the addition of DMSO at 25% or more to the standard solubilisation buffer, the cytoplasm but not the nuclei are efficiently dissolved and solubilised so that recovery yields of proteins and other biomolecules are greatly improved compared with the non-DMSO treated sample.

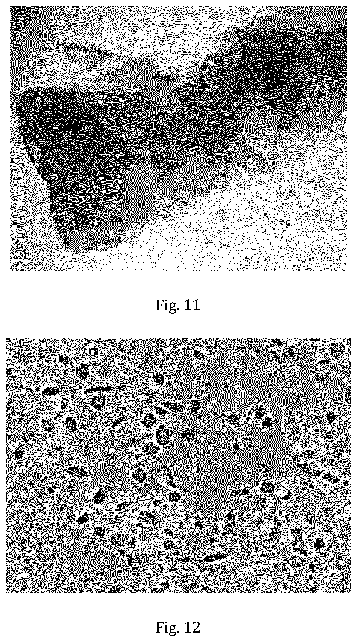

It has also been found that the nuclei that are released from the DES fixed cell maintain a great deal of their morphology, for example muscle nuclei from DES fixed muscle samples have long spindle shapes (Figure 12), whilst liver nuclei are round and of variable volume as has been observed from Formalin Fixed Paraffin Embedded (FFPE) H&E stained slides of these tissues. Therefore the nuclei maintain their *in vivo* shape and size which is representative of the cell type of origin. This has clinical applications, for example if a cancer biopsy is fixed with a DES, preferably Trimethylglycine:Trifluoroacetamide (1:2 mol:mol) as set out in US Patent 9,696,247 and then treated with a 25% solution of DMSO or more to dissolve the cytoplasm and recover nuclei by filtration using a 0.25 µm filter or other means, the nuclear shape, diameter, volume, symmetry, density, opacity and/or texture can be observed with a microscope and is highly representative of the type and stage of cancer or other diseases (Carleton NM., J. Cell Biochem. (2018) doi: 10.1002/jcb.27156), and can therefore have a useful diagnostic application. Indeed, changes to the shape of the nucleus may precede the appearance of cancer. It will be evident to one skilled in the art that producing and analyzing such nuclei is also much faster than processing a biopsy with FFPE and cutting sections and staining. Additionally, observation of nuclei in FFPE sections are only in 2D and can be partially obscured by the cytoplasm, whilst when prepared with DMSO, the nuclei can be observed in their entirety in 3D.

It will be evident to one skilled in the art that DMSO at 25% or more can be used either in aqueous solution such as water, a buffer, a kosmotrope, a chaotrope, a detergent, or alternatively as part of another solvent such as a DES, an alcohol, a phenol or an organic solvent. One advantage of using DMSO dissolved in a DES is that the RNA and other biomolecules are protected from degradation. One preferred DES is Trimethylglycine: Trifluoroacetamide (2:1 mol:mol). One less preferred alternative to DMSO is Diethylsulphoxide.

### Example 24

### Dissociation of Tissue Fixed With Trimethylglycine:Trifluoroacetamide and Formic Acid

It has been observed that certain mammalian tissues that have fixed with Trimethylglycine:Trifluoroacetamide (1:2 mol:mol) and then transferred and immersed in 20 volumes or more of Phosphate Buffered Saline (PBS) or other salt solutions such as 20 mM NaCl, KCI, LiCI or NH4CI have suboptimal RNA stability. The removal of the DES post-fixation and its replacement with an aqueous buffer and/or salt solution may lead to a reactivation of nucleases, or a chemical hydrolysis of the RNA. This issue has not been observed when the sample has first been treated with Choline chloride:urea or Trimethylglycine:urea (1:2 mol:mol) before being transferred to PBS. If the Trimethylglycine:Trifluoroacetamide fixed sample needs to be transferred to an aqueous solution for example for FACS or antibody binding reaction, it is preferred that the solution (i) does not contain a salt or a buffer such as Tris-HCI, or the minimum necessary is used, (ii) the sample is handled at no more than 4°C, (iii) Dithioerythritol, Beta-mercaptoethanol, TCEP or other such reductant is added in the range 10-100mM, (iv) the aqueous solution has a pH in the range of 3-5, (v) contains a detergent such as Triton X-100, CTAB, NP40, CHAPS, Tween-20 or other detergent in the range 0.01 - 1%, (vi) contains a PEG such as PEG200 in the range 20 - 90%, (vii) contains at least 60%, preferably at least 70%, and even more preferably 75-100% Trimethylglycine:Trifluoroacetamide, Choline chloride:urea or Trimethylglycine:urea (1:2 mol:mol), (viii) contains at least 60%, preferably at least 70%, and even more preferably 75-100% alcohol such as Methanol, Ethanol, Propanol or Butanol such as 1-Butanol.

Alternatively, it has been found that the acidification of the DES fixative in particular Trimethylglycine:Trifluoroacetamide (1:2 mol:mol) before its addition to the sample for fixation, with an acid such as Hydrochloric acid, Sulphuric acid, Phosphoric acid, Nitric acid, Boric acid, but most preferably a Carboxylic acid such as Citric, Lactic, Pyruvic, Butanoic acid, Propanoic, Acetic, and most preferably Formic acid are particularly effective. The acidification of the DES should not lead to a pH of less than 1.5 or the biomolecules may become hydrolysed, therefore, preferably the pH should be in the range pH 2 or more, more preferably in the range pH 2 - 5.5 and most preferably in the range pH 4 - 5. Longer term storage of the sample in acidified DES should be avoided and the fixation time should be less than 1 week at room temperature, preferably less than 3 days to avoid DNA hydrolysis. Formic acid is the preferred acid when added in the range 1-12M, more preferably 4-9M, more preferably 6-8M and most preferably 7-8M Formic acid final concentration in Trimethylglycine:Trifluoroacetamide (1:2 mol:mol). Alternatively, Formic acid may be present It has therefore been surprisingly found that Trimethylglycine:Trifluoroacetamide (1:2 mol:mol) containing 7-8M Formic acid has been found to protect RNA, DNA, proteins and phosphoproteins whilst fixing the cells in the fixed sample even when it is subsequently transferred to PBS. Advantageously the addition of Formic acid greatly reduces the viscosity of the DES.

It has additionally been found that tissues that have been fixed with Trimethylglycine:Trifluoroacetamide (1:2 mol:mol) containing 7-8M Formic acid dissociate easily during sonication as set out in Example 1, providing a means to produce single cells with high quality RNA suitable for single-cell RNA seq applications. Therefore it has surprisingly been found that high concentrations of Formic acid in Trimethylglycine:Trifluoroacetamide are compatible with RNA and DNA integrity if treatment time is limited to 6 hours or less. It has also been found that treatment of the sample with Trimethylglycine:Trifluoroacetamide (1:2 mol:mol) with 1-12M, more preferably 4-9M, more preferably 6-8M and most preferably 7-8M Formic acid leads not only to excellent cell morphology but also leads to permanent protection of RNA even when the cell is transitioned into an aqueous buffer such as water or PBS.

The use of Trimethylglycine:Trifluoroacetamide (1:2 mol:mol) Formic acid as the fixative has several advantages, in particular (i) permanent protection of RNA when the fixed sample is incubated in an aqueous buffer such as PBS, and (ii) the volume of the fixed sample is retained and does not shrink thereby improving the histological detail of the tissue and morphological detail of the cells.

It will be evident to one skilled in the art that a variety of acids can be used for acidification of the DES, preferably Trimethylglycine:Trifluoroacetamide (1:2 mol:mol) but acids with a smaller molecular weight are preferred as are low molecular weight Carboxylic acids including halogenated Carboxylic acids.

### Example 25

### Dissociation of Pre-frozen Tissue into Single cells

It has been found that Trimethylglycine:Trifluoroacetamide (1:2 mol:mol) consolidates and binds together cells and tissues that have previously been frozen. It is known that frozen tissues and cells when thawed out show ice-damage, breakage, membrane damage and loss of cell integrity. It was found that individual cells despite having been frozen and thawed, maintained structural integrity and retained their cellular morphology when, during or following thawing they are placed in contact with Trimethylglycine:Trifluoroacetamide (1:2 mol:mol). This has great utility as many clinical samples are routinely frozen and stored in biobanks but cellular morphology and quality suffers greatly as a result of thawing non-fixed cells. Generally cells and tissues are protected from ice-damage by the application of cryoprotectants such as DMSO prior to freezing and thawing but this process is rarely used routinely with clinical samples prior to freezing. One example of the direct consequence of freeze-thawing tissues is the inability to maintain brain cell integrity for scRNAseq, therefore only the RNA component found in the nucleus is currently analysed but not the cytoplasmic RNA which is lost following thawing the sample.

Therefore, provided herein is a means to maintain cell integrity of previously frozen samples for downstream applications including dissociation, collection, sorting, isolation, manipulation, handling, processing, identification, staining, labeling with a dye, an antibody, an aptamer, a fluorophore, a MassTag, an enzymatic reaction, a nucleic acid probe, sorting, separation, purification, identification, FACS sorting, flow cytometry, centrifugation, gradient centrifugation, microfluidics, handling, manipulation such as those associated with morphological analysis, droplet sequencing (Drop-Seq), Seq-Well and other single-cell analysis platforms used for determining the composition, diversity and/or sequence of RNA and/or DNA, and/or protein and/or phosphoprotein analysis.

It has been found that Trimethylglycine:Trifluoroacetamide (1:2 mol:mol) is excellent at fixing frozen samples, maintaining individual complete cells.

250 mg of mouse liver was frozen at -80°C for one week, the frozen sample was then removed from the freezer and immediately immersed and allowed to thaw in 1 mL of Trimethylglycine:Trifluoroacetamide (1:2 mol:mol) and fix for 6 hours at room temperature. The fixed mouse liver tissue was then placed in 300 µL of 95% Trimethylglycine:Trifluoroacetamide (1:2 mol:mol) and sonicated to obtain single cells as described in Example 1. Alternatively, the frozen-thawed-Trimethylglycine:Trifluoroacetamide fixed sample can be processed into paraffin and sectioned according to standard paraffin embedding procedures used for FFPE sections.

### Example 26

### Alternative Tissue Fixation Procedures

One of the advantages of using Formic acid mixed with Trimethylglycine:Trifluoroacetamide (1:2 mol:mol) is that the mixture can be used in a number of different ways during tissue fixation, dissociation and manipulation of single cells. As representative examples, a tissue sample such as 20 mg of mouse liver was treated with 20 volumes of (i) Trimethylglycine:Trifluoroacetamide (1:2 mol:mol) 8M Formic acid for 20 minutes to 12 hours and then transferred into 50 volumes of Trimethylglycine:Trifluoroacetamide (1:2 mol:mol) to wash out and dilute the Formic acid then left for 1 hour to 1 month at room temperature before dissociation into single-cells as set out for example in Example 1 using 80-95% Trimethylglycine:Trifluoroacetamide (1:2 mol:mol) as the Dissociation buffer. It was found that the RNA was of excellent quality even after the cells were incubated in an aqueous buffer such as PBS for 2 hours; (ii) Trimethylglycine:Trifluoroacetamide (1:2 mol:mol) for 20 minutes to 1 month and then transferred into 20 volumes of Trimethylglycine:Trifluoroacetamide (1:2 mol:mol) 8 M Formic acid and left for 1 to 12 hours at room temperature before dissociation into single-cells as set out for example in Example 1 using 80-95% Trimethylglycine:Trifluoroacetamide (1:2 mol:mol) as the Dissociation buffer; (iii) Trimethylglycine:Trifluoroacetamide (1:2 mol:mol) 8M Formic acid for 20 minutes to 12 hours and then transferred directly into 80-95% Trimethylglycine:Trifluoroacetamide (1:2 mol:mol) as the sonication Dissociation buffer as set out in Example 1; or (iv) Trimethylglycine:Trifluoroacetamide (1:2 mol:mol) 8 M Formic acid for 20 minutes to 12 hours and then transferred into 100 mM Tris, 10 mM EDTA buffer pH 7.4 to neutralize the acidity prior to transfer into 80-95% Trimethylglycine:Trifluoroacetamide (1:2 mol:mol) as the sonication Dissociation buffer as set out in Example 1. It will be evident that single-cells such as tissue culture cells can also be treated in a similar sequence but without the dissociation step.

### Example 27

### Alternative Tissue Dissociation Procedures

It has been found that either Trimethylglycine:Trifluoroacetamide (1:2 mol:mol) or Trimethylglycine:Trifluoroacetamide (1:2 mol:mol) 8 M Formic acid 2 hour fixed mouse liver (20 mg) can be dissociated into single cells by first washing the fixed tissue in 500 volumes of water, removing excess water and then placing in 100 µl of water on for example an aluminium foil support and then freezing either in a freezer at -20°C, -80°C or dry ice, then allowing the frozen sample to thaw at room temperature and repeating the freeze-thaw five times. Whilst this method was found to be efficient for liver samples, it was not useful for brain samples which underwent general lysis. When using Trimethylglycine:Trifluoroacetamide (1:2 mol:mol) 8M Formic acid fixed tissues, the water can be replaced with a number of other aqueous buffers such as PBS, 1X TE or SSC. RNA quality as determined by RNA purification and gel electrophoresis was found to be excellent following such dissociated mouse liver.

### Example 28 (a reference example)

### Storage of Fixed Cell in PBS

It has been found that Trimethylglycine:Trifluoroacetamide (1:2 mol:mol) 8M Formic acid 2 hour fixed mouse liver (20 mg) was washed in 500 volumes of PBS, excess PBS removed by blotting with a tissue then placed in 500 µl of PBS and incubated for 1, 4, or 12 hours in PBS at room temperature before RNA purification (RNeasy, Qiagen) and analysis by gel electrophoresis showed no detectable RNA degradation. RNA stability in fixed cells and tissues is important for protocols and applications using aqueous buffers for extended periods such as FACS analysis, cell sorting, antibody binding and scRNAseq.

### Example 29

### Lysis of Fixed Cells

20 mg of mouse liver was fixed in 20 volumes of Trimethylglycine:Trifluoroacetamide (1:2 mol:mol) 8 M for 2 hour at room-temperature and dissociated into single-cells according to Example 1. The dissociated cells were then briefly stained using 0.02% bromophenol blue and 500 cells were placed in individual wells of a 96-well plate and treated with various solvents and aqueous solutions and the effect on cell lysis observed after 5 min, 2 hours or 4 days incubation. The lysis results are shown in the following table:

| **Reagent** | **Cell lysis effect (4 day treatment)** |
|---|---|
| Pronase (30mg/ml) | Complete Lysis |
| 2% SDS | Complete Lysis |
| 0.1% SDS | >15% cell lysis |
| 8M Urea | >95% cell lysis (not nuclei) |
| 1% CHAPS | >80% cell lysis |
| 1% Triton X100 | >60% cell lysis |
| 1% NP40 | >50% cell lysis |
| 1% CTAB | 100% cell lysis (not nuclei) |
| 1% Tween-20 | >60% cell lysis |
| 10% IGEPAL-CA630 | No lysis |
| 30% IGEPAL-CA630 | No lysis |
| 50% IGEPAL-CA630 | 100% cell lysis |
| 4M Guanidine thiocyanate | >50% cell lysis |
| 100% PEG200 | No lysis |
| 10mg/ml BSA (acetylated) | No lysis |
| 100% Acetone | No lysis |
| 10% Beta-mercaptoethanol | No lysis |
| 20% Beta-mercaptoethanol | No lysis |
| 40% Beta-mercaptoethanol | No lysis |
| 60% Beta-mercaptoethanol | >50% cell lysis |
| 80% Beta-mercaptoethanol | >80% cell lysis |
| 100% Beta-mercaptoethanol | 100% cell lysis |
| 1M LiCl | No lysis |
| 100% Ethanol | No lysis |
| 100% Glycerol | No lysis |
| 100% 1-Methylimidazole | No lysis |
| 100% 1-Ethylimidazole | No lysis |
| 100% TRlzol | 100% cell lysis |
| 100% N-ethyl pyrrolidone | No lysis |
| 50% N-methyl pyrrolidone | No lysis |
| 100% Formyl morpholine | No lysis |
| 100% Formamide | No lysis |
| 100% Tetramethylurea | No lysis |
| 100% DMSO | >70% cell lysis |
| 100% 1,1,1,3,3,3-Hexafluoro-2-propanol | Complete lysis including nuclei |
| 90% 1,1,1,3,3,3-Hexafluoro-2-propanol | Complete lysis including nuclei |
| 50% 1,1,1,3,3,3-Hexafluoro-2-propanol | >40% cell lysis |
| 10% 1,1,1,3,3,3-Hexafluoro-2-propanol | No lysis |
| 50% 1,1,1,3,3,3-Hexafluoro-2-propanol 50% DMSO | >80% Lysed |
| Saturated Trifluoroacteamide | No lysis |
| Saturated Tetrafluoropropionamide | No lysis |
| Saturated N,N-Diethyl-2,2,2-Trifluoroacetamide | No lysis |
| Saturated N,N-Methyl-2,2,2-Trifluoroacetamide | No lysis |
| 50% Tetramethyl ammonium oxide | 100% cell lysis |
| 5M Tetramethyl ammonium chloride | No lysis |
| 5M Tetrabutyl ammonium chloride | No lysis |
| 5M Sodium perchlorate | 100% cell lysis |
| 5M Guanidine thiocyanate | >50% cell lysis |
| 5M Guanidine HCI | >50% cell lysis |
| 5M Potassium iodide | >50% cell lysis |
| 1M Potassium iodide | No lysis |
| Saturated Thiourea | 100% cell lysis |

### Example 30

### Post-Tissue Fixation Procedures using Either Pure Acetic acid (17.4M) or dilutions of Acetic acid in Trimethylglycine:Trifluoroacetamide or Water

20 mg of mouse liver was treated with 20 volumes of Trimethylglycine:Trifluoroacetamide (1:2 mol:mol) for 20 minutes to 12 hours at ambient temperature and then transferred into 50 volumes of a secondary post-fixative containing either (i) pure glacial Acetic acid, or (ii) an aqueous or alcoholic dilution to give a final concentration of 9-17.39M Acetic acid, or (iii) a Trimethylglycine:Trifluoroacetamide (1:2 mol:mol) dilution to give a final concentration of 11-17.39 M Acetic acid then incubated for 10 minutes to 1 hour at room temperature before dissociation into single-cells as set out for example in Example 1 using 75-95% Trimethylglycine:Trifluoroacetamide (1:2 mol:mol) as the Dissociation buffer. The Acetic acid component can then be neutralized using Trimethylglycine:Trifluoroacetamide (1:2 mol:mol) as set-out for Formic acid in Example 26.

Alternatively and conveniently, dissociated single cells generated as set-out in Example 1, in the Dissociation buffer can be poured over and captured on a filter such as a Pluriselect 1-4 µm filter, and then the cells on the filter are treated with either (i) pure Acetic acid, or (ii) a 9-17.39M dilution of Acetic acid in water or alcohol, or, (iii) a 11-17.39M dilution of Acetic acid in Trimethylglycine:Trifluoroacetamide (1:2 mol:mol) for 10-60 minutes before removal of the acid by filtration and neutralization with 50 volumes of Trimethylglycine:Trifluoroacetamide (1:2 mol:mol). Alternatively, the cells can be recovered by the addition of 1 volume of ethanol and centrifugation at 600g for 5 minutes to pellet the cells.

It was found that the RNA was of excellent quality even after the Acetic acid treated cells were incubated in an aqueous buffer such as PBS for 2 hours.

### Example 31

### Dissociation using Ultrasound Transmission Gel

To 1 ml of Trimethylglycine:Trifluoroacetamide (1:2 mol:mol) was added 10-100 mg of a freshly dissected mammalian tissue such as liver and incubated for at least 30 minutes at room temperature (approximately 24°C).

Tissue dissociation was then carried out by sonication according to Example 1 except the Dissociation buffer was 50-500 µl of Aquasonic^{®} 100 Ultrasound Transmission Gel (Parker Laboratories Inc., USA) with a viscosity of 130,000-185,000 centipoise.

It was found that the Transmission gel was an efficient medium for transferring ultrasound energy to the sample and led to good tissue dissociation into single cells.

### Example 32

### Dissociation in Small Volumes of Dissociation Buffer

Fixation of the biological sample was carried out according to Example 1 except the volume of Dissociation buffer was reduced to between 5-100 µl, with a preference of 10-20 µl. With such small volumes the sample was not completely covered with the Dissociation buffer but was in contact with the container such as the polycarbonate tube and therefore the sonication energy via the thin layer of Dissociation buffer. It was found that Dissociation was rapid and effective and provided a simple means to concentrate the single-cells in a small volume for subsequent manipulation such as pelleting or filtration, and was particularly useful for very small amounts of sample.

### Example 33

### Illustrative method of processing a biological sample

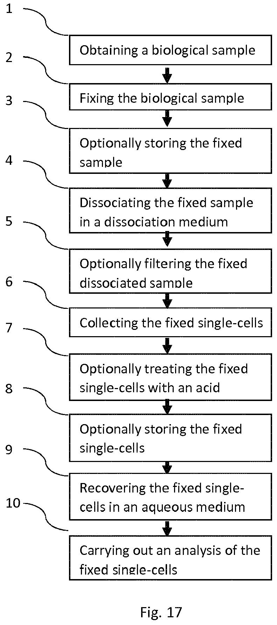

Fig. 17 is a flow chart illustrating general steps of an example method for processing a biological sample.

The method includes obtaining (1) a biological sample such as a fresh or frozen human or animal tissue. The sample is fixed (2) using a fixative by placing the sample in contact with the fixative. The sample is then optionally stored (3) in the fixative.

The sample is then transferred to a dissociation medium and dissociated (4). Larger dissociated pieces of tissue are optionally removed by filtering (5).

Single cells are collected (6) by for example pelleting the cells or by filtration. The collected cells are optionally treated (7) with a secondary fixative such as an acid.

The single cells may be stored (8), resuspended (9) in an aqueous medium, and then analysed (10).

### Example 34 (a reference example)

### Illustrative Apparatus

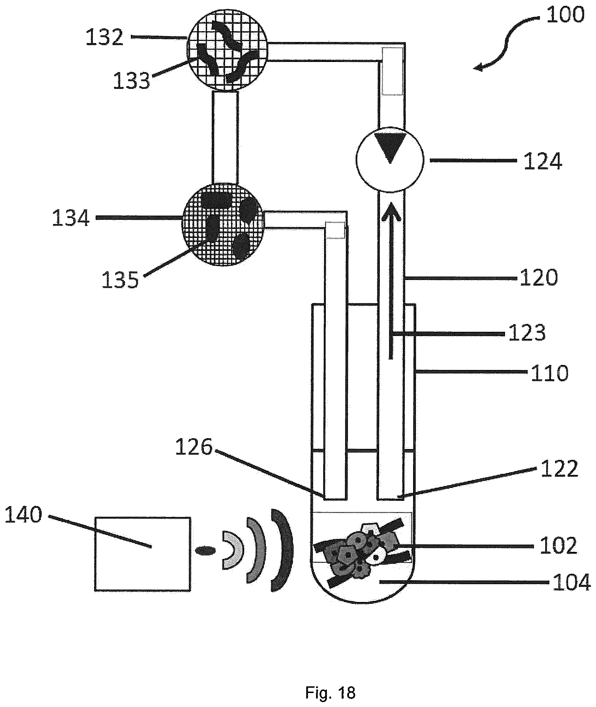

Fig. 18 is a schematic diagram of one example of an apparatus 100 useful for processing a biological sample 102 to obtain single cells and/or groups of cells.

Apparatus 100 includes a container 110 for containing a biological sample 102 and a dissociation medium 104. The apparatus also includes a conduit 120, for example a tube. Conduit 120 has an inlet 122 for removing liquid dissociation medium from container 110, and an outlet 126 downstream of inlet 122. In this example, outlet 126 is for returning liquid dissociation medium to the container. This allows for recycling of dissociation medium.

First and second filters 132, 134 are arranged in the conduit. First filter 132 is a 40 to 70 µm filter, in other words, apertures in the filter have sizes in the range 40 to 70 µm. In this way, the first filter is configured to trap larger pieces of dissociated material such as fragments of extracellular matrix 133. Second filter 134 is downstream of first filter 132. Second filter 132 is a 1 to 12 µm filter. Filter sizes in this range are useful for isolating single cells 135. Cell sizes may vary, and the filter size may be selected as desired within this range.

A pump 124 for pumping liquid dissociation medium from the inlet 122 of the conduit 120 to the outlet 126 of the conduit 120.

In use, a biological sample 102 to be dissociated and an aliquot of a dissociation medium 104 such as a deep eutectic solvent are placed in the container 110. Sonication energy provided by a sonication source 140 is applied to the container, and is transmitted to the biological sample 102 via the dissociation medium. The sonication causes cavitation of the dissociation medium 104. The cavitation causes the biological sample 102 to be dissociated, producing single cells and/or groups of cells along with pieces of extracellular matrix. The single cells and/or groups of cells are suspended in the dissociation medium.

Pump 124 is activated, drawing dissociation medium and the single cells, groups of cells and extracellular matrix into the inlet 122 of conduit 120. The dissociation medium flows through the conduit in downstream direction 123. Pieces of extracellular matrix 133 are too large to fit through first filter 132, and are separated from the stream of dissociation medium. The dissociation medium then reaches second filter 134, where single cells 135 are separated from the dissociation medium. After passing through second filter 134, dissociation medium is returned to the container via outlet 126 of the conduit.

The biological sample is contacted with a deep eutectic solvent during the processing. This could be before or during the dissociating. The nature of the biological sample is not particularly limited. It may be fresh, frozen, or fixed. For fixed samples, fixation may be with a deep eutectic solvent or a deep eutectic solvent may be applied post-fixation either as dissociation medium or in an intermediate step.

Single cells obtained using the apparatus may be used in, for example, single cell DNA sequencing or single cell RNA sequencing, amongst other applications.

## Claims

1. A method of processing a biological sample to obtain single cells and/or groups of cells, the method comprising:
fixing cells of the biological sample using a fixative;
contacting the biological sample with a dissociation medium;
dissociating the biological sample in the presence of the dissociation medium to obtain the single cells and/or groups of cells;
wherein the fixative comprises a deep eutectic solvent.

2. The method according to claim 1, wherein dissociating the biological sample comprises sonication of the biological sample using a sonicator, optionally wherein the method further comprises constraining at least a part of the biological sample to a cavitation zone during the sonication, and further optionally wherein the constraining comprises feeding the biological sample into the cavitation zone as the biological sample is reduced in size.

3. The method according to claim 1 or claim 2, wherein the fixing comprises sonicating the biological sample, optionally wherein:
i) the sonication is pulse sonication, the pulse sonication having a pulse cycle wherein the sonicator is active for less than or equal to 20% of the pulse cycle, and the amplitude of the sonication is less than or equal to 2 µm; and/or
ii) the sonication is performed at a temperature in the range -20 to 37°C; and/or
iii) the sonication is applied for a time in the range 10 to 60 minutes.

4. The method according to claims 2 or 3,
wherein the sonicator has a liquid bath, optionally wherein the liquid bath contains an oil, a hydrocolloid, an echography gel, or a salt solution.

5. The method according to any preceding claim, wherein the deep eutectic solvent of the fixative comprises a first component and a second component, wherein first component is a compound of Formula I: 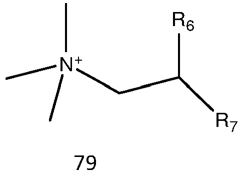 wherein:
R₆ is H or OH;
R₇ is selected from H, CH₃, Cl, Br, a carbonyl oxygen, and 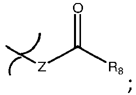
Z is selected from -CH₂-, O and S;
R₈ is R₁₁ or OH; and
wherein the second component comprises a compound of Formula II or a salt thereof: 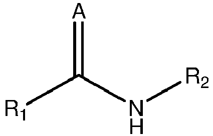 wherein:
A is selected from O, S, and NH;
R₁ is selected from H, an alkene group having 1 to 6 carbon atoms, R₉, -NH₂, -NH-(CH₂)ₙCH₃, and -C(R₃)(R₄)(R₅);
wherein n is 0 or an integer from 1 to 5;
R₂ is selected from H and linear alkyl group having 1 to 3 carbon atoms;
R₃ is an optionally substituted 5- or 6- membered aliphatic or aromatic ring, wherein
the substituent is R₁₀;
R₄ and R₅ are each independently H or F; and
wherein R₉, R₁₀, and R₁₁ are each independently selected from alkyl groups having one to three carbon atoms, monochloroalkyl groups having one to three carbon atoms, and mono-, di- or tri-fluoroalkyl groups having one to three carbon atoms;
optionally wherein:
i) the first component is trimethylglycine and the second component is trifluoroacetamide; or
ii) the first component is trimethylglycine and the second component is urea; or
iii) the first component is choline chloride and the second component is urea.

6. The method according to any of claims 1 to 4, wherein the deep eutectic solvent of the fixative comprises a first component and a second component, wherein the first component comprises a compound of Formula III: 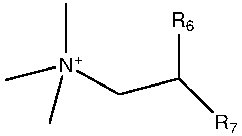 wherein:
R₆ is H or OH;
R₇ is selected from H, CH₃, Cl, Br, a carbonyl oxygen, and 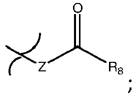
Z is selected from -CH₂-, O and S;
wherein R₈ is selected from OH, an alkyl group having one to three carbon atoms, a monochloroalkyl group having one to three carbon atoms, and a mono-, di- or tri-fluoroalkyl group having one to three carbon atoms; and
wherein the second component is a sugar or a sugar alcohol having at least 3 carbon atoms;
optionally wherein the first component is trimethylglycine and the second component is glycerol.

7. The method according to any preceding claim, wherein the fixative further comprises an acid selected from: formic acid, acetic acid, citric acid, lactic acid, pyruvic acid, tartaric acid, propionic acid, oxalic acid, maleic acid, salicylic acid, ascorbic acid, benzoic acid, butanoic acid, hydrochloric acid, sulfuric acid, phosphoric acid, nitric acid and boric acid; optionally
i)wherein the acid is acetic acid, and the acetic acid is present in the dissociation medium at a concentration of at least 2 M, at least 8 M, at least 11 M, or at least 13 M; or
ii) the acid is formic acid, and the formic acid is present in the fixative at a concentration in the range 4 to 9 M, 6 to 8 M, or 7 to 8 M; or of 8 M; or
iii) the acid is formic acid, and the formic acid is present in the fixative at a concentration in the range 50 to 500 mM, 150 to 400 mM, or 250 to 350 mM.

8. The method according to any preceding claim, wherein the dissociation medium comprises a deep eutectic solvent, optionally wherein:
i) the dissociation medium further comprises water in an amount of up to 50 % by volume of the dissociation medium; and/or
ii) the dissociation medium further comprises an acid selected from: formic acid, acetic acid, citric acid, lactic acid, pyruvic acid, tartaric acid, propionic acid, oxalic acid, maleic acid, salicylic acid, ascorbic acid, benzoic acid, butanoic acid, hydrochloric acid, sulfuric acid, phosphoric acid, nitric acid and boric acid.

9. The method according to claim 8, wherein the deep eutectic solvent of the dissociation medium comprises a first component and a second component, wherein first component is a compound of Formula I: 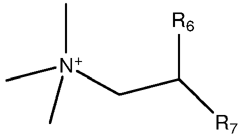 wherein:
R₆ is H or OH;
R₇ is selected from H, CH₃, Cl, Br, a carbonyl oxygen, and 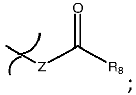
Z is selected from -CH₂-, O and S;
R₈ is R₁₁ or OH; and
wherein the second component comprises a compound of Formula II or a salt thereof: 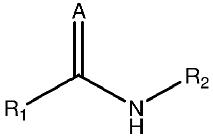 wherein:
A is selected from O, S, and NH;
R₁ is selected from H, an alkene group having 1 to 6 carbon atoms, R₉, -NH₂, -NH-(CH₂)ₙCH₃, and -C(R₃)(R₄)(R₅);
wherein n is 0 or an integer from 1 to 5;
R₂ is selected from H and linear alkyl group having 1 to 3 carbon atoms;
R₃ is an optionally substituted 5- or 6- membered aliphatic or aromatic ring, wherein
the substituent is R₁₀;
R₄ and R₅ are each independently H or F; and
wherein R₉, R₁₀, and R₁₁ are each independently selected from alkyl groups having one to three carbon atoms, monochloroalkyl groups having one to three carbon atoms, and mono-, di- or tri-fluoroalkyl groups having one to three carbon atoms;
optionally wherein:
i) the first component of the deep eutectic solvent of the dissociation medium is trimethylglycine and the second component is trifluoroacetamide; or
ii) the first component of the deep eutectic solvent of the dissociation medium is trimethylglycine and the second component is urea; or
iii) the first component of the deep eutectic solvent of the dissociation medium is choline chloride and the second component is urea.

10. The method according to claim 8, wherein the deep eutectic solvent of the dissociation medium comprises a first component and a second component, wherein the first component comprises a compound of Formula III: 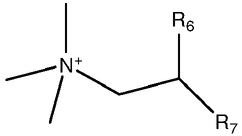 wherein:
R₆ is H or OH;
R₇ is selected from H, CH₃, Cl, Br, a carbonyl oxygen, and 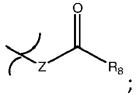
Z is selected from -CH₂-, O and S;
wherein R₈ is selected from OH, an alkyl group having one to three carbon atoms, a monochloroalkyl group having one to three carbon atoms, and a mono-, di- or tri-fluoroalkyl group having one to three carbon atoms; and
wherein the second component is a sugar or a sugar alcohol having at least 3 carbon atoms; optionally wherein the first component of the deep eutectic solvent of the dissociation medium is trimethylglycine and the second component is glycerol.

11. The method according to any preceding claim, wherein the dissociation medium comprises a glycol selected from ethylene glycol, glycerol, diethylene glycol, triethylene glycol, and a polyethylene glycol, PEG, having a molecular weight in the range 200 to 800, optionally wherein:
i) the dissociation medium comprises PEG 200, PEG 300, PEG 400 or PEG 600; and/or
ii) the dissociation medium further comprises up to 60 % water by weight of the dissociation medium.

12. The method according to any preceding claim, further comprising:
freezing the biological sample before the fixing; and
thawing the biological sample during the fixing.

13. The method according to any preceding claim, further comprising, after the fixing and before the dissociating, softening the biological sample using a glycol, optionally wherein the glycol is selected from: ethylene glycol, glycerol, diethylene glycol, triethylene glycol, PEG 200, PEG 300, PEG 400, PEG 600 and mixtures thereof.

14. The method according to any preceding claim, further comprising:
i)after the dissociating, lysing the single cells and/or groups of cells, optionally wherein the method further comprises isolating cell nuclei after the lysing; and/or
ii) at one or more time points during the dissociating, isolating groups of cells from the dissociation medium; and/or
iii) adding dimethyl sulfoxide to the dissociation medium before, during or after the dissociating.

## Patentansprüche

1. Verfahren zur Verarbeitung einer biologischen Probe, um einzelne Zellen und/oder Zellgruppen zu erhalten, wobei das Verfahren aufweist:
Fixieren von Zellen der biologischen Probe unter Verwendung eines Fixiermittels;
Kontaktieren der biologischen Probe mit einem Dissoziationsmedium;
Dissoziieren der biologischen Probe in Gegenwart des Dissoziationsmediums, um die einzelnen Zellen und/oder Zellgruppen zu erhalten;
wobei das Fixiermittel ein tief eutektisches Lösungsmittel aufweist.

2. Verfahren nach Anspruch 1, wobei das Dissoziieren der biologischen Probe die Beschallung der biologischen Probe unter Verwendung eines Beschallers aufweist, wobei optional das Verfahren ferner ein Einschränken wenigstens eines Teils der biologischen Probe auf einer Kavitationszone während der Beschallung aufweist, und wobei ferner optional das Einschränken ein Einspeisen der biologischen Probe in die Kavitationszone aufweist, während die biologische Probe in ihrer Größe reduziert wird.

3. Verfahren nach Anspruch 1 oder Anspruch 2, wobei das Fixieren ein Beschallen der biologischen Probe aufweist, wobei optional:
i) die Beschallung eine Impulsbeschallung ist, wobei die Impulsbeschallung einen Impulszyklus hat, bei dem der Beschaller für weniger als oder gleich 20% des Impulszyklus aktiv ist und die Amplitude der Beschallung weniger als oder gleich 2µm ist; und/oder
ii) die Beschallung bei einer Temperatur im Bereich von -20 bis 37°C durchgeführt wird; und/oder
iii) die Beschallung für eine Zeit im Bereich von 10 bis 60 Minuten durchgeführt wird.

4. Verfahren nach Anspruch 2 oder 3, wobei der Beschaller ein Flüssigkeitsbad hat, wobei optional das Flüssigkeitsbad ein Öl, ein Hydrokolloid, ein Echographiegel oder eine Salzlösung enthält.

5. Verfahren nach einem der vorhergehenden Ansprüche, wobei das tief eutektische Lösungsmittel des Fixiermittels eine erste Komponente und eine zweite Komponente aufweist, wobei die erste Komponente eine Verbindung der Formel I ist: 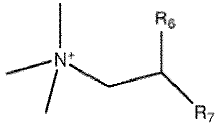 worin:
R₆ ist H oder OH;
R₇ ist ausgewählt aus H, CH₃, CI, Br, einem Carbonylsauerstoff, und 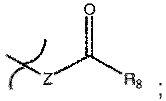
Z ist ausgewählt aus -CH₂-, O und S;
R₈ ist R₁₁ oder OH; und
wobei die zweite Komponente eine Verbindung der Formel II oder ein Salz davon aufweist: 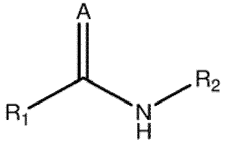 worin:
A ist ausgewählt aus O, S und NH;
R₁ ist ausgewählt aus H, einer Alkengruppe mit 1 bis 6 Kohlenstoffatomen, R₉, -NH₂, -NH-(CH₂)ₙCH₃, und -C(R₃)(R₄)(R₅);
wobei n 0 oder eine ganze Zahl von 1 bis 5 ist;
R₂ ist ausgewählt aus H und einer linearen Alkylgruppe mit 1 bis 3 Kohlenstoffatomen;
R₃ ist ein gegebenenfalls substituierter 5- oder 6-gliedriger aliphatischer oder
aromatischer Ring, wobei der Substituent R₁₀ ist;
R₄ und R₅ sind jeweils unabhängig voneinander H oder F; und
wobei R₉, R₁₀ und R₁₁ jeweils unabhängig voneinander ausgewählt sind aus Alkylgruppen mit ein bis drei Kohlenstoffatomen, Monochloralkylgruppen mit ein bis drei Kohlenstoffatomen, und Mono-, Di- oder Tri-Fluoralkylgruppen mit ein bis drei Kohlenstoffatomen;
wobei optional:
i) die erste Komponente Trimethylglycin ist und die zweite Komponente Trifluoracetamid ist; oder
ii) die erste Komponente Trimethylglycin ist und die zweite Komponente Harnstoff ist; oder
iii) die erste Komponente Cholinchlorid ist und die zweite Komponente Harnstoff ist.

6. Verfahren nach einem der Ansprüche 1 bis 4, wobei das tief eutektische Lösungsmittel des Fixiermittels eine erste Komponente und eine zweite Komponente aufweist wobei die erste Komponente eine Verbindung der Formel III aufweist: 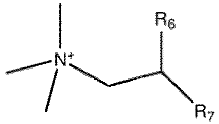 worin:
R₆ ist H oder OH;
R₇ ist ausgewählt aus H, CH₃, CI, Br, einem Carbonylsauerstoff, und 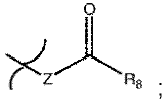
Z ist ausgewählt aus -CH₂-, O und S;
wobei R₈ ausgewählt ist aus OH, einer Alkylgruppe mit ein bis drei Kohlenstoffatomen, einer Monochloralkylgruppe mit ein bis drei Kohlenstoffatomen und einer Mono-, Di- oder Tri-Fluoralkylgruppe mit ein bis
drei Kohlenstoffatomen; und
wobei die zweite Komponente ein Zucker oder ein Zuckeralkohol mit wenigstens 3 Kohlenstoffatomen ist;
wobei optional die erste Komponente Trimethylglycin ist und die zweite Komponente Glycerin ist.

7. Verfahren nach einem der vorhergehenden Ansprüche, wobei das Fixiermittel ferner eine Säure aufweist, die ausgewählt ist aus: Ameisensäure, Essigsäure, Zitronensäure, Milchsäure, Brenztraubensäure, Weinsäure, Propionsäure, Oxalsäure, Maleinsäure, Salicylsäure, Ascorbinsäure, Benzoesäure, Butansäure, Salzsäure, Schwefelsäure, Phosphorsäure, Salpetersäure und Borsäure; wobei optional
i) die Säure Essigsäure ist und die Essigsäure im Dissoziationsmedium in einer Konzentration von wenigstens 2 M, wenigstens 8 M, wenigstens 11 M oder wenigstens 13 M vorhanden ist; oder
ii) die Säure Ameisensäure ist und die Ameisensäure im Fixiermittel in einer Konzentration im Bereich von 4 bis 9 M, 6 bis 8 M oder 7 bis 8 M; oder von 8 M vorhanden ist; oder
iii) die Säure Ameisensäure ist und die Ameisensäure im Fixiermittel in einer Konzentration im Bereich von 50 bis 500 mM, 150 bis 400 mM oder 250 bis 350 mM vorhanden ist.

8. Verfahren nach einem der vorhergehenden Ansprüche, wobei das Dissoziationsmedium ein tief eutektisches Lösungsmittel aufweist, wobei optional:
i) das Dissoziationsmedium ferner Wasser in einer Menge von bis zu 50 Volumenprozent des Dissoziationsmediums aufweist; und/oder
ii) das Dissoziationsmedium ferner eine Säure aufweist, die ausgewählt ist aus: Ameisensäure, Essigsäure, Zitronensäure, Milchsäure, Brenztraubensäure, Weinsäure, Propionsäure, Oxalsäure, Maleinsäure, Salicylsäure, Ascorbinsäure, Benzoesäure, Butansäure, Salzsäure, Schwefelsäure, Phosphorsäure, Salpetersäure und Borsäure.

9. Verfahren nach Anspruch 8, wobei das tief eutektische Lösungsmittel des Dissoziationsmediums eine erste Komponente und eine zweite Komponente aufweist, wobei die erste Komponente eine Verbindung der Formel I ist: 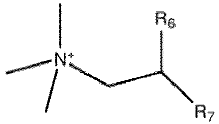 worin:
R₆ ist H oder OH;
R₇ ist ausgewählt aus H, CH₃, CI, Br, einem Carbonylsauerstoff, und 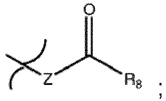
Z ist ausgewählt aus -CH₂-, O und S;
R₈ ist R₁₁ oder OH; und
wobei die zweite Komponente eine Verbindung der Formel II oder ein Salz davon aufweist: 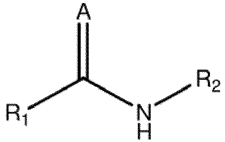
worin:
A ist ausgewählt aus O, S und NH;
R₁ ist ausgewählt aus H, einer Alkengruppe mit 1 bis 6 Kohlenstoffatomen, R₉, -NH₂, -NH-(CH₂)ₙCH₃, und -C(R₃)(R₄)(R₅);
wobei n 0 oder eine ganze Zahl von 1 bis 5 ist;
R₂ ist ausgewählt aus H und einer linearen Alkylgruppe mit 1 bis 3 Kohlenstoffatomen;
R₃ ist ein gegebenenfalls substituierter 5- oder 6-gliedriger aliphatischer oder
aromatischer Ring, wobei der Substituent R₁₀ ist;
R₄ und R₅ sind jeweils unabhängig voneinander H oder F; und
wobei R₉, R₁₀ und R₁₁ jeweils unabhängig voneinander ausgewählt sind aus Alkylgruppen mit ein bis drei Kohlenstoffatomen, Monochloralkylgruppen mit ein bis drei Kohlenstoffatomen, und Mono-, Di- oder Tri-Fluoralkylgruppen mit ein bis drei Kohlenstoffatomen;
wobei optional:
i) die erste Komponente des tief eutektische Lösungsmittels des Dissoziationsmediums Trimethylglycin ist und die zweite Komponente Trifluoracetamid ist; oder
ii) die erste Komponente des tief eutektischen Lösungsmittels des Dissoziationsmediums Trimethylglycin ist und die zweite Komponente Harnstoff ist; oder
iii) die erste Komponente des tiefen eutektischen Lösungsmittels des Dissoziationsmediums Cholinchlorid ist und die zweite Komponente Harnstoff ist.

10. Verfahren nach Anspruch 8, wobei das tief eutektische Lösungsmittel des Dissoziationsmediums eine erste Komponente und eine zweite Komponente aufweist, wobei die erste Komponente eine Verbindung der Formel III ist: 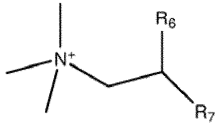 worin:
R₆ ist H oder OH;
R₇ ist ausgewählt aus H, CH₃, CI, Br, einem Carbonylsauerstoff, und 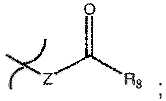
Z ist ausgewählt aus -CH₂-, O und S;
wobei R₈ ausgewählt ist aus OH, einer Alkylgruppe mit ein bis drei Kohlenstoffatomen, einer Monochloralkylgruppe mit ein bis drei Kohlenstoffatomen und einer Mono-, Di- oder Tri-Fluoralkylgruppe mit ein bis
drei Kohlenstoffatomen; und
wobei die zweite Komponente ein Zucker oder ein Zuckeralkohol mit wenigstens 3 Kohlenstoffatomen ist;
wobei optional die erste Komponente des tief eutektischen Lösungsmittels des Dissoziationsmediums Trimethylglycin ist und die zweite Komponente Glycerin ist.

11. Verfahren nach einem der vorhergehenden Ansprüche, wobei das Dissoziationsmedium ein Glykol aufweist, das aus Ethylenglykol, Glycerin, Diethylenglykol, Triethylenglykol und einem Polyethylenglykol, PEG, mit einem Molekulargewicht im Bereich von 200 bis 800 ausgewählt ist, wobei optional:
i) das Dissoziationsmedium PEG 200, PEG 300, PEG 400 oder PEG 600 aufweist; und/oder
ii) das Dissoziationsmedium ferner bis zu 60 % Wasser, bezogen auf das Gewicht des Dissoziationsmediums, aufweist.

12. Das Verfahren nach einem der vorhergehenden Ansprüche, ferner aufweisend:
Einfrieren der biologischen Probe vor dem Fixieren; und
Auftauen der biologischen Probe während des Fixierens.

13. Verfahren nach einem der vorhergehenden Ansprüche, ferner aufweisend nach dem Fixieren und vor dem Dissoziieren ein Erweichen der biologischen Probe unter Verwendung eines Glykols, wobei das Glykol optional ausgewählt ist aus: Ethylenglykol, Glycerin, Diethylenglykol, Triethylenglykol, PEG 200, PEG 300, PEG 400, PEG 600 und Mischungen davon.

14. Das Verfahren nach einem der vorangehenden Ansprüche, ferner aufweisend:
i) nach dem Dissoziieren ein Lysieren der einzelnen Zellen und/oder Zellgruppen, wobei optional das Verfahren ferner ein Isolieren von Zellkernen nach dem Lysieren aufweist; und/oder
ii) zu einem oder mehreren Zeitpunkten während der Dissoziation ein Isolieren von Zellgruppen aus dem Dissoziationsmedium; und/oder
iii) Zugeben von Dimethylsulfoxid zum Dissoziationsmedium vor, während oder nach der Dissoziation.

## Revendications

1. Procédé de traitement d'un échantillon biologique pour obtenir des cellules individuelles et/ou des groupes de cellules, le procédé consistant à :
fixer des cellules de l'échantillon biologique à l'aide d'un fixateur ;
mettre l'échantillon biologique en contact avec un milieu de dissociation ;
dissocier l'échantillon biologique en présence du milieu de dissociation pour obtenir les cellules individuelles et/ou les groupes de cellules,
dans lequel le fixateur comprend un solvant eutectique profond.

2. Procédé selon la revendication 1, dans lequel l'étape de dissociation de l'échantillon biologique comprend l'étape de sonication de l'échantillon biologique à l'aide d'un sonicateur, éventuellement dans lequel le procédé consiste en outre à contraindre au moins une partie de l'échantillon biologique à une zone de cavitation pendant l'étape de sonication, et en outre éventuellement dans lequel l'étape de contrainte consiste à introduire l'échantillon biologique dans la zone de cavitation lorsque la taille de l'échantillon biologique est réduite.

3. Procédé selon la revendication 1 ou 2, dans lequel l'étape de fixation consiste à soniquer l'échantillon biologique, éventuellement dans lequel :
i) la sonication est une sonication en régime impulsionnel, la sonication en régime impulsionnel ayant un cycle d'impulsions dans lequel le sonicateur est actif pendant une durée inférieure ou égale à 20 % du cycle d'impulsions, et l'amplitude de la sonication est inférieure ou égale à 2 µm ; et/ou
ii) la sonication est effectuée à une température comprise entre -20 et 37 °C ; et/ou
iii) la sonication est appliquée pendant une durée comprise entre 10 et 60 minutes.

4. Procédé selon la revendication 2 ou 3, dans lequel le sonicateur comporte un bain liquide, éventuellement dans lequel le bain liquide contient une huile, un hydrocolloïde, un gel échographique ou une solution saline.

5. Procédé selon l'une quelconque des revendications précédentes, dans lequel le solvant eutectique profond du fixateur comprend un premier composant et un deuxième composant,
dans lequel le premier composant est un composé de formule 1 : 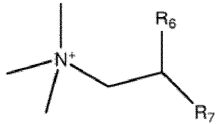 où :
R₆ représente H ou OH ;
R₇ est choisi parmi H, CH₃, Cl, Br, un oxygène carbonyle et 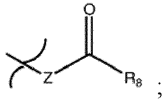
Z est choisi parmi -CH₂-, O et S ;
R₈ représente R₁₁ ou OH, et
dans lequel le deuxième composant comprend un composé de formule II ou l'un de ses sels : 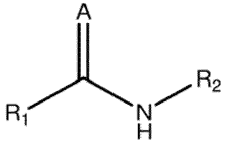 où :
A est choisi parmi O, S et NH ;
R₁ est choisi parmi H, un groupe alcène ayant 1 à 6 atomes de carbone, R₉, -NH₂, -NH-(CH₂)ₙCH₃ et -C(R₃)(R₄)(R₅), n valant 0 ou un nombre entier compris entre 1 et 5 ;
R₂ est choisi parmi H et un groupe alkyle linéaire ayant 1 à 3 atomes de carbone ;
R₃ représente un cycle aliphatique ou aromatique à 5 ou 6 chaînons éventuellement substitué, le substituant étant R₁₀ ;
R₄ et R₅ représentent chacun indépendamment H ou F, et
dans lequel R₉, R₁₀ et R₁₁ sont chacun indépendamment choisis parmi des groupes alkyle ayant un à trois atomes de carbone, des groupes monochloroalkyle ayant un à trois atomes de carbone et des groupes mono-, di- ou trifluoroalkyle ayant un à trois atomes de carbone,
éventuellement dans lequel :
i) le premier composant est la triméthylglycine et le deuxième composant est le trifluoroacétamide ; ou
ii) le premier composant est la triméthylglycine et le deuxième composant est l'urée ; ou
iii) le premier composant est le chlorure de choline et le deuxième composant est l'urée.

6. Procédé selon l'une quelconque des revendications 1 à 4, dans lequel le solvant eutectique profond du fixateur comprend un premier composant et un deuxième composant,
dans lequel le premier composant comprend un composé de formule III : 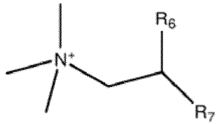 où :
R₆ représente H ou OH ;
R₇ est choisi parmi H, CH₃, Cl, Br, un oxygène carbonyle et 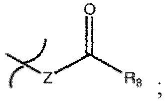
Z est choisi parmi -CH₂-, O et S ;
R₈ est choisi parmi OH, un groupe alkyle ayant un à trois atomes de carbone, un groupe monochloroalkyle ayant un à trois atomes de carbone et un groupe mono-, di- ou trifluoroalkyle ayant un à trois atomes de carbone, et
dans lequel le deuxième composant est un sucre ou un alcool de sucre ayant au moins 3 atomes de carbone,
éventuellement dans lequel le premier composant est la triméthylglycine et le deuxième composant est le glycérol.

7. Procédé selon l'une quelconque des revendications précédentes, dans lequel le fixateur comprend en outre un acide choisi parmi : l'acide formique, l'acide acétique, l'acide citrique, l'acide lactique, l'acide pyruvique, l'acide tartrique, l'acide propionique, l'acide oxalique, l'acide maléique, l'acide salicylique, l'acide ascorbique, l'acide benzoïque, l'acide butanoïque, l'acide chlorhydrique, l'acide sulfurique, l'acide phosphorique, l'acide nitrique et l'acide borique, éventuellement dans lequel :
i) l'acide est l'acide acétique, et l'acide acétique est présent dans le milieu de dissociation à une concentration d'au moins 2 M, d'au moins 8 M, d'au moins 11 M ou d'au moins 13 M ; ou
ii) l'acide est l'acide formique, et l'acide formique est présent dans le fixateur à une concentration comprise entre 4 et 9 M, 6 et 8 M ou 7 et 8 M, ou égale à 8 M ; ou
iii) l'acide est l'acide formique, et l'acide formique est présent dans le fixateur à une concentration comprise entre 50 et 500 mM, 150 et 400 mM ou 250 et 350 mM.

8. Procédé selon l'une quelconque des revendications précédentes, dans lequel le milieu de dissociation comprend un solvant eutectique profond, éventuellement dans lequel :
i) le milieu de dissociation comprend en outre de l'eau en une quantité allant jusqu'à 50 % en volume du milieu de dissociation ; et/ou
ii) le milieu de dissociation comprend en outre un acide choisi parmi : l'acide formique, l'acide acétique, l'acide citrique, l'acide lactique, l'acide pyruvique, l'acide tartrique, l'acide propionique, l'acide oxalique, l'acide maléique, l'acide salicylique, l'acide ascorbique, l'acide benzoïque, l'acide butanoïque, l'acide chlorhydrique, l'acide sulfurique, l'acide phosphorique, l'acide nitrique et l'acide borique.

9. Procédé selon la revendication 8, dans lequel le solvant eutectique profond du milieu de dissociation comprend un premier composant et un deuxième composant,
dans lequel le premier composant est un composé de formule 1 : 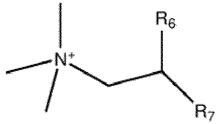 où :
R₆ représente H ou OH ;
R₇ est choisi parmi H, CH₃, Cl, Br, un oxygène carbonyle et 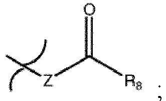
Z est choisi parmi -CH₂-, O et S ;
R₈ représente R₁₁ ou OH, et
dans lequel le deuxième composant comprend un composé de formule II ou l'un de ses sels : 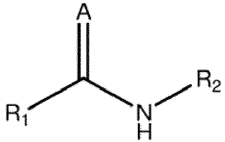 où :
A est choisi parmi O, S et NH ;
R₁ est choisi parmi H, un groupe alcène ayant 1 à 6 atomes de carbone, R₉, -NH₂, -NH-(CH₂)ₙCH₃ et -C(R₃)(R₄)(R₅), n valant 0 ou un nombre entier de 1 à 5 ;
R₂ est choisi parmi H et un groupe alkyle linéaire ayant 1 à 3 atomes de carbone ;
R₃ représente un cycle aliphatique ou aromatique à 5 ou 6 chaînons éventuellement substitué, le substituant étant R₁₀ ;
R₄ et R₅ représentent chacun indépendamment H ou F, et
dans lequel R₉, R₁₀ et R₁₁ sont chacun indépendamment choisis parmi des groupes alkyle ayant un à trois atomes de carbone, des groupes monochloroalkyle ayant un à trois atomes de carbone et des groupes mono-, di- ou trifluoroalkyle ayant un à trois atomes de carbone,
éventuellement dans lequel :
i) le premier composant du solvant eutectique profond du milieu de dissociation est la triméthylglycine et le deuxième composant est le trifluoroacétamide ; ou
ii) le premier composant du solvant eutectique profond du milieu de dissociation est la triméthylglycine et le deuxième composant est l'urée ; ou
iii) le premier composant du solvant eutectique profond du milieu de dissociation est le chlorure de choline et le deuxième composant est l'urée.

10. Procédé selon la revendication 8, dans lequel le solvant eutectique profond du milieu de dissociation comprend un premier composant et un deuxième composant, dans lequel le premier composant comprend un composé de formule III : 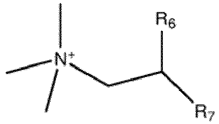 où :
R₆ représente H ou OH ;
R₇ est choisi parmi H, CH₃, Cl, Br, un oxygène carbonyle et 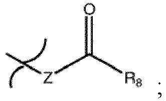
Z étant choisi parmi -CH₂-, O et S ;
R₈ étant choisi parmi OH, un groupe alkyle ayant un à trois atomes de carbone, un groupe monochloroalkyle ayant un à trois atomes de carbone et un groupe mono-, di- ou trifluoroalkyle ayant un à trois atomes de carbone, et
dans lequel le deuxième composant est un sucre ou un alcool de sucre ayant au moins 3 atomes de carbone,
éventuellement dans lequel le premier composant du solvant eutectique profond du milieu de dissociation est la triméthylglycine et le deuxième composant est le glycérol.

11. Procédé selon l'une quelconque des revendications précédentes, dans lequel le milieu de dissociation comprend un glycol choisi parmi l'éthylène glycol, le glycérol, le diéthylène glycol, le triéthylène glycol et un polyéthylène glycol, le PEG, ayant un poids moléculaire compris entre 200 et 800, éventuellement dans lequel :
i) le milieu de dissociation comprend du PEG 200, du PEG 300, du PEG 400 ou du PEG 600 ; et/ou
ii) le milieu de dissociation comprend en outre jusqu'à 60 % d'eau en poids du milieu de dissociation.

12. Procédé selon l'une quelconque des revendications précédentes, consistant en outre à :
congeler l'échantillon biologique avant l'étape de fixation ; et
décongeler l'échantillon biologique pendant l'étape de fixation.

13. Procédé selon l'une quelconque des revendications précédentes, consistant en outre, après l'étape de fixation et avant l'étape de dissociation, à ramollir l'échantillon biologique à l'aide d'un glycol, éventuellement dans lequel le glycol est choisi parmi : l'éthylène glycol, le glycérol, le diéthylène glycol, le triéthylène glycol, le PEG 200, le PEG 300, le PEG 400, le PEG 600 et leurs mélanges.

14. Procédé selon l'une quelconque des revendications précédentes, dans lequel le procédé consiste en outre à :
i) lyser, après l'étape de dissociation, les cellules individuelles et/ou les groupes de cellules, éventuellement dans lequel le procédé consiste en outre à isoler des noyaux cellulaires après l'étape de lyse ; et/ou
ii) isoler, à un ou plusieurs moments pendant l'étape de dissociation, des groupes de cellules du milieu de dissociation ; et/ou
iii) ajouter du diméthylsulfoxyde au milieu de dissociation avant, pendant ou après l'étape de dissociation.
